# EUROPEAN PATENT SPECIFICATION

(11) **EP 3 390 365 B1**
(45) Date of publication and mention of the grant of the patent: **14.04.2021**
(21) Application number: 16805287.6
(22) Date of filing: 28.11.2016
(51) Int. Cl.: C07D 219/02, C07D 221/16, C07D 333/76, C07C 211/61, C07D 265/38, C07D 279/22, C07D 209/86, C07D 307/91, H01L 51/00

(54) **MATERIALS FOR ORGANIC ELECTROLUMINESCENT DEVICES**
MATERIALIEN FÜR ORGANISCHE ELEKTROLUMINESZENTE VORRICHTUNGEN
MATÉRIAUX POUR DISPOSITIFS ÉLECTROLUMINESCENTS ORGANIQUES

(30) Priority: 16.12.2015 EP 15200294
(43) Date of publication of application: 24.10.2018
(73) Proprietor: Merck Patent GmbH, 64293 Darmstadt (DE)
(72) Inventor: PFISTER, Jochen, 64342 Seeheim-Jugenheim (DE); MUJICA-FERNAUD, Teresa, 64283 Darmstadt (DE); MONTENEGRO, Elvira, 69469 Weinheim (DE); ANÉMIAN, Rémi Manouk, Seoul 657-169 (KR)
(86) International application number: PCT/EP2016/002009
(87) International publication number: WO 2017/102063

(56) References cited:
- EP-A1- 2 978 040
- EP-A1- 3 010 066
- EP-A1- 3 059 773
- WO-A1-2013/017192
- WO-A1-2013/120577
- WO-A1-2014/044344
- WO-A1-2014/166572
- WO-A1-2015/000549
- WO-A1-2015/022051
- WO-A1-2015/041428
- WO-A1-2015/071473
- WO-A1-2016/062368
- WO-A1-2016/078747
- WO-A1-2016/137243
- WO-A1-2016/199784
- CN-A- 104 557 440
- DE-A1-102009 032 922
- US-A1- 2016 225 993

## Description

The present invention relates to materials for use in electronic devices, in particular in organic electroluminescent devices, and to electronic devices comprising these materials.

The structure of organic electroluminescent devices (OLEDs) in which organic semiconductors are employed as functional materials is described, for example, in US 4,539,507, US 5,151,629, EP 0676461 and WO 98/27136. The emitting materials employed here are increasingly organometallic complexes which exhibit phosphorescence instead of fluorescence (M. A. Baldo et al., Appl. Phys. Lett. 1999, 75, 4-6).

In accordance with the prior art, the hole-transport materials used in the hole-transport layer or in the hole-injection layer are, in particular, triarylamine derivatives which frequently contain at least two triarylamino groups or at least one triarylamino group and at least one carbazole group. These compounds are frequently derived from diarylamino-substituted triphenyl-amines (TPA type), from diarylamino-substituted biphenyl derivatives (TAD type) or combinations of these base compounds. Furthermore, for example, use is made of spirobifluorene derivatives which are substituted by one to four diarylamino groups (for example in accordance with EP 676461, US 7,714,145, EP2814906). Further spirobifluorene amines are disclosed in WO 2013/120577 A1 and DE 10 2009 032922 A1. In the case of these compounds, there is still a need for improvement both in the case of fluorescent and in the case of phosphorescent OLEDs, in particular with respect to efficiency, lifetime and operating voltage on use in an organic electroluminescent device.
At the same time, it is important that the compounds processed by vacuum evaporation exhibit a high temperature stability, in order to obtain OLEDs with reproducible properties. Finally, the compounds used in OLEDs should exhibit a low crystallinity and a high glass transition temperature, in order to obtain OLEDs with a satisfying lifetime.
The object of the present invention is to provide compounds which are suitable for use in a fluorescent or phosphorescent OLED, in particular a phosphorescent OLED, for example as hole-transport material in a hole-transport or exciton-blocking layer or as matrix material in an emitting layer.

It has now been found that certain compounds described below in greater detail achieve this object and result in significant improvements in the organic electroluminescent device, in particular with respect to the lifetime, the efficiency and the operating voltage. This applies to phosphorescent and fluorescent electroluminescent devices, especially on use of the compounds according to the invention as hole-transport material or as matrix material. Furthermore, the compounds described below contain a rigid planar Spiro unit and flexible structure elements in the outer periphery, whereby the flexibility of the molecule center is reduced and the solubility is increased by the substituents, which leads to an easier cleaning and an easier handling of these compounds. Finally, the compounds of the present invention generally have high thermal stability and can therefore be sublimed without decomposition and without a residue. The present invention therefore relates to these compounds and to electronic devices which comprise compounds of this type.

The present invention therefore relates to a compound of the following formula (1): 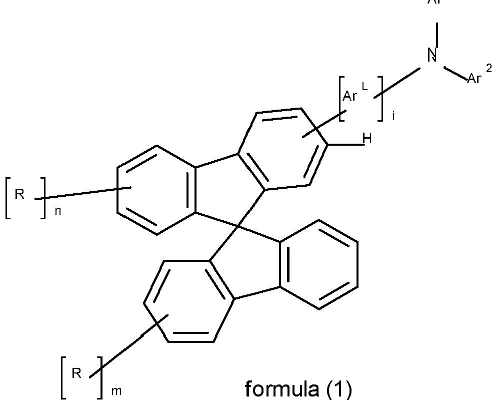 where the symbols and indices used are as defined in claim 1.

In particular, in the compounds of formula (1):
R is an aromatic ring system selected from phenyl, biphenyl, terphenyl and naphthyl;
Ar¹ , Ar² are on each occurrence, identically or differently, an aromatic or heteroaromatic ring system having 5 to 60 aromatic ring atoms, which may in each case also be substituted by one or more radicals R² ; Ar¹ and Ar² here may also be connected via a single bond or a divalent bridge selected from -N(R²)-, -O-, -S-, -C(R2)2-, -C(R²)₂-C(R²)₂-, -Si(R²)2- or -B(R²)-;
Ar^{L} is an aromatic or heteroaromatic ring system having 5 to 40 aromatic ring atoms, which may in each case be substituted by one or more radicals R² ;
R² are selected on each occurrence, identically or differently, from the group consisting of H, D, F, CI, Br, I, CHO, CN, C(=O)Ar³ , P(=O)(Ar³)₂, S(=O)Ar³ , S(=O)2Ar³, NO₂, Si(R³)₃, B(OR³)₂, OSO₂R³, straight-chain alkyl, alkoxy or thioalkyl groups having 1 to 40 C atoms or branched or cyclic alkyl, alkoxy or thioalkyl groups having 3 to 40 C atoms, each of which may be substituted by one or more radicals R³, where in each case one or more non-adjacent CH₂ groups may be replaced by R³C=CR³, C=C, Si(R³)₂, Ge(R³)₂, Sn(R³)₂, C=O, C=S, C=Se, P(=O)(R³ ), SO, SO₂ , O, S or CONR³ and where one or more H atoms may be replaced by D, F, CI, Br, I, CN or NO₂ , aromatic or heteroaromatic ring systems having 5 to 60 aromatic ring atoms, which may in each case be substituted by one or more radicals R³, and aryloxy groups having 5 to 60 aromatic ring atoms, which may be substituted by one or more radicals R³, where two or more adjacent substituents R² may optionally form a mono- or polycyclic, aliphatic ring system or aromatic ring system, which may be substituted by one or more radicals R³;
R³ is selected on each occurrence, identically or differently, from the group consisting of H, D, F, CI, Br, I, CHO, CN, C(=O)Ar³ , P(=O)(Ar³)₂ , S(=O)Ar³ , S(=O)₂Ar³ , NO₂ , Si(R⁴)₃ , B(OR⁴)₂ , OSO₂R⁴ , straight-chain alkyl, alkoxy or thioalkyl groups having 1 to 40 C atoms or a branched or cyclic alkyl, alkoxy or thioalkyl groups having 3 to 40 C atoms, each of which may be substituted by one or more radicals R⁴ , where in each case one or more non-adjacent CH₂ groups may be replaced by R⁴C=CR⁴ , C=C, Si(R⁴)₂ , Ge(R⁴)₂ , Sn(R⁴)₂ , C=O, C=S, C=Se, P(=O)(R⁴), SO, SO₂ , O, S or CONR⁴ and where one or more H atoms may be replaced by D, F, CI, Br, I, CN or NO₂, aromatic or heteroaromatic ring systems having 5 to 60 aromatic ring atoms, which may in each case be substituted by one or more radicals R⁴ , and aryloxy groups having 5 to 60 aromatic ring atoms, which may be substituted by one or more radicals R⁴ , where two or more adjacent substituents R³ may optionally form a mono- or polycyclic, aliphatic ring system or aromatic ring system, which may be substituted by one or more radicals R⁴;
R⁴ is selected on each occurrence, identically or differently, from the group consisting of H, D, F, CI, Br, I, CN, straight-chain alkyl, alkoxy or thioalkyl groups having 1 to 20 C atoms or branched or cyclic alkyl, alkoxy or thioalkyl groups having 3 to 20 C atoms, where in each case one or more non-adjacent CH₂ groups may be replaced by SO, SO₂, O or S and where one or more H atoms may be replaced by D, F, CI, Br or I, and aromatic or heteroaromatic ring systems having 5 to 24 C atoms;
Ar³ is an aromatic or heteroaromatic ring system having 5 to 24 aromatic ring atoms, which may in each case be substituted by one or more radicals R⁴;
i is 0 or 1;
m, n are, identically or differently, 0, or 1;
and m + n = 1.

For the purposes of the present application, the following definitions of chemical groups apply:
An aryl group in the sense of this invention contains 6 to 60 aromatic ring atoms; a heteroaryl group in the sense of this invention contains 5 to 60 aromatic ring atoms, at least one of which is a heteroatom. The hetero atoms are preferably selected from N, O and S. This represents the basic definition. If other preferences are indicated in the description of the present invention, for example with respect to the number of aromatic ring atoms or the heteroatoms present, these apply.

An aryl group or heteroaryl group here is taken to mean either a simple aromatic ring, i.e. benzene, or a simple heteroaromatic ring, for example pyridine, pyrimidine or thiophene, or a condensed (annellated) aromatic or heteroaromatic polycycle, for example naphthalene, phenanthrene, quino line or carbazole. A condensed (annellated) aromatic or heteroaromatic polycycle in the sense of the present application consists of two or more simple aromatic or heteroaromatic rings condensed with one another.

An aryl or heteroaryl group, which may in each case be substituted by the above-mentioned radicals and which may be linked to the aromatic or heteroaromatic ring system via any desired positions, is taken to mean, in particular, groups derived from benzene, naphthalene, anthracene, phenanthrene, pyrene, dihydropyrene, chrysene, perylene, fluoranthene, benzanthracene, benzophenanthrene, tetracene, pentacene, benzopyrene, furan, benzofuran, isobenzofuran, dibenzofuran, thiophene, benzothiophene, isobenzothiophene, dibenzothiophene, pyrrole, indole, isoindole, carbazole, pyridine, quinoline, isoquinoline, acridine, phenanthridine, benzo-5,6-quinoline, benzo-6,7-quinoline, benzo-7,8-quinoline, phenothiazine, phenoxazine, pyrazole, indazole, imidazole, benzimidazole, naphthimidazole, phenanthrimidazole, pyridimidazole, pyrazinimidazole, quinoxalinimidazole, oxazole, benzoxazole, naphthoxazole, anthroxazole, phenanthroxazole, isoxazole, 1,2-thiazole, 1,3-thiazole, benzothiazole, pyridazine, benzopyridazine, pyrimidine, benzopyrimidine, quinoxaline, pyrazine, phenazine, naphthyridine, azacarbazole, benzocarboline, phenanthroline, 1,2,3-triazole, 1,2,4-triazole, benzotriazole, 1,2,3-oxadiazole, 1,2,4-oxadiazole, 1,2,5-oxadiazole, 1,3,4-oxadiazole, 1,2,3-thiadiazole, 1,2,4-thiadiazole, 1,2,5-thiadiazole, 1,3,4-thiadiazole, 1,3,5-triazine, 1,2,4-triazine, 1,2,3-triazine, tetrazole, 1,2,4,5-tetrazine, 1,2,3,4-tetrazine, 1,2,3,5-tetrazine, purine, pteridine, indolizine and benzothiadiazole.

An aryloxy group in accordance with the definition of the present invention is taken to mean an aryl group, as defined above, which is bonded via an oxygen atom. An analogous definition applies to heteroaryloxy groups. An aromatic ring system in the sense of this invention contains 6 to 60 C atoms in the ring system. A heteroaromatic ring system in the sense of this invention contains 5 to 60 aromatic ring atoms, at least one of which is a heteroatom. The heteroatoms are preferably selected from N, O and/or S. An aromatic or heteroaromatic ring system in the sense of this invention is intended to be taken to mean a system which does not necessarily contain only aryl or heteroaryl groups, but instead in which, in addition, a plurality of aryl or heteroaryl groups may be connected by a non-aromatic unit (preferably less than 10% of the atoms other than H), such as, for example, an sp³-hybridised C, Si, N or O atom, an sp²-hybridised C or N atom or an sp-hybridised C atom. Thus, for example, systems such as 9,9'-spirobifluorene, 9,9'-diarylfluorene, triarylamine, diaryl ether, stilbene, etc., are also intended to be taken to be aromatic ring systems in the sense of this invention, as are systems in which two or more aryl groups are connected, for example, by a linear or cyclic alkyl, alkenyl or alkynyl group or by a silyl group. Furthermore, systems in which two or more aryl or heteroaryl groups are linked to one another via single bonds are also taken to be aromatic or heteroaromatic ring systems in the sense of this invention, such as, for example, systems such as biphenyl, terphenyl or diphenyltriazine.

An aromatic or heteroaromatic ring system having 5 - 60 aromatic ring atoms, which may in each case also be substituted by radicals as defined above and which may be linked to the aromatic or heteroaromatic group via any desired positions, is taken to mean, in particular, groups derived from benzene, naphthalene, anthracene, benzanthracene, phenanthrene, benzophenanthrene, pyrene, chrysene, perylene, fluoranthene, naphthacene, pentacene, benzopyrene, biphenyl, biphenylene, terphenyl, terphenylene, quaterphenyl, fluorene, spirobifluorene, dihydrophenanthrene, dihydropyrene, tetrahydropyrene, cis- or trans-indenofluorene, truxene, isotruxene, spirotruxene, spiroisotruxene, furan, benzofuran, isobenzofuran, dibenzofuran, thiophene, benzothiophene, isobenzothiophene, dibenzothiophene, pyrrole, indole, isoindole, carbazole, indolocarbazole, indenocarbazole, pyridine, quinoline, isoquinoline, acridine, phenanthridine, benzo-5,6-quinoline, benzo-6,7-quinoline, benzo-7,8-quinoline, phenothiazine, phenoxazine, pyrazole, indazole, imidazole, benzimidazole, naphthimidazole, phenanthrimidazole, pyridimidazole, pyrazinimidazole, quinoxalinimidazole, oxazole, benzoxazole, naphthoxazole, anthroxazole, phenanthroxazole, isoxazole, 1,2-thiazole, 1,3-thiazole, benzothiazole, pyridazine, benzopyridazine, pyrimidine, benzopyrimidine, quinoxaline, 1,5-diazaanthracene, 2,7-diazapyrene, 2,3-diazapyrene, 1,6-diazapyrene, 1,8-diazapyrene, 4,5-diazapyrene, 4,5,9,10-tetraazaperylene, pyrazine, phenazine, phenoxazine, phenothiazine, fluorubin, naphthyridine, azacarbazole, benzocarboline, phenanthroline, 1,2,3-triazole, 1,2,4-triazole, benzotriazole, 1,2,3-oxadiazole, 1,2,4-oxadiazole, 1,2,5-oxadiazole, 1,3,4-oxadiazole, 1,2,3-thiadiazole, 1,2,4-thiadiazole, 1,2,5-thiadiazole, 1,3,4-thiadiazole, 1,3,5-triazine, 1,2,4-triazine, 1,2,3-triazine, tetrazole, 1,2,4,5-tetrazine, 1,2,3,4-tetrazine, 1,2,3,5-tetrazine, purine, pteridine, indolizine and benzothiadiazole, or combinations of these groups.

For the purposes of the present invention, a straight-chain alkyl group having 1 to 40 C atoms or a branched or cyclic alkyl group having 3 to 40 C atoms or an alkenyl or alkynyl group having 2 to 40 C atoms, in which, in addition, individual H atoms or CH₂ groups may be substituted by the groups mentioned above under the definition of the radicals, is preferably taken to mean the radicals methyl, ethyl, n-propyl, i-propyl, n-butyl, i-butyl, s-butyl, t-butyl, 2-methylbutyl, n-pentyl, s-pentyl, cyclopentyl, neopentyl, n-hexyl, cyclohexyl, neohexyl, n-heptyl, cycloheptyl, n-octyl, cyclooctyl, 2-ethylhexyl, trifluoromethyl, pentafluoroethyl, 2,2,2-trifluoroethyl, ethenyl, propenyl, butenyl, pentenyl, cyclopentenyl, hexenyl, cyclohexenyl, heptenyl, cycloheptenyl, octenyl, cyclooctenyl, ethynyl, propynyl, butynyl, pentynyl, hexynyl or octynyl. An alkoxy or thioalkyl group having 1 to 40 C atoms is preferably taken to mean methoxy, trifluoromethoxy, ethoxy, n-propoxy, i-propoxy, n-butoxy, i-butoxy, s-butoxy, t-butoxy, n-pentoxy, s-pentoxy, 2-methylbutoxy, n-hexoxy, cyclohexyloxy, n-heptoxy, cycloheptyloxy, n-octyloxy, cyclooctyloxy, 2-ethylhexyloxy, pentafluoroethoxy, 2,2,2-trifluoroethoxy, methylthio, ethylthio, n-propylthio, i-propylthio, n-butylthio, i-butylthio, s-butylthio, t-butylthio, n-pentylthio, s-pentylthio, n-hexylthio, cyclohexylthio, n-heptylthio, cycloheptylthio, n-octylthio, cyclooctylthio, 2-ethylhexylthio, trifluoromethylthio, pentafluoroethylthio, 2,2,2-trifluoroethylthio, ethenylthio, propenylthio, butenylthio, pentenylthio, cyclopentenylthio, hexenylthio, cyclohexenylthio, heptenylthio, cycloheptenylthio, octenylthio, cyclooctenylthio, ethynylthio, propynylthio, butynylthio, pentynylthio, hexynylthio, heptynylthio or octynylthio.

The formulation that two or more radicals may form a ring with one another is, for the purposes of the present application, intended to be taken to mean, inter alia, that the two radicals are linked to one another by a chemical bond. This is illustrated by the following schemes:

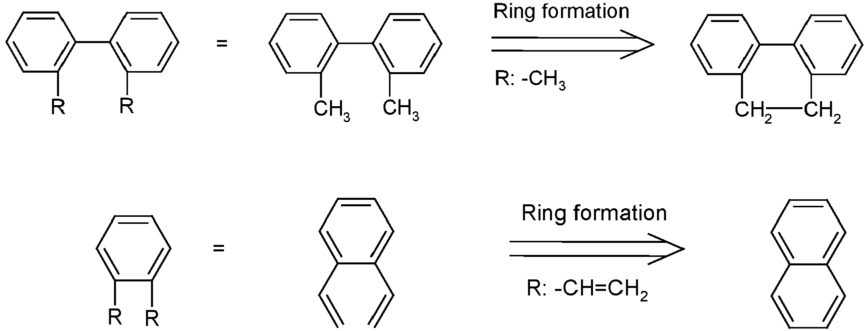

Furthermore, however, the above-mentioned formulation is also intended to be taken to mean that, in the case where one of the two radicals represents hydrogen, the second radical is bonded at the position to which the hydrogen atom was bonded, with formation of a ring. This is illustrated by the following scheme:

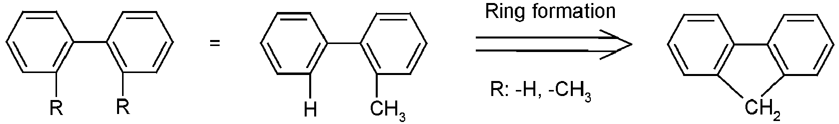

It is preferred that m = 1 and n = 0.

The group Ar^{L} is preferably, identically or differently on each occurrence, selected from aromatic or heteroaromatic ring systems having 5 to 40, more preferably 5 to 30, even more preferably 5 to 18 aromatic ring atoms, which may in each case also be substituted by one or more radicals R².

Particularly preferable groups Ar^{L} are selected from the groups of formulae (Ar^{L}-1) to (Ar^{L}-37) below: 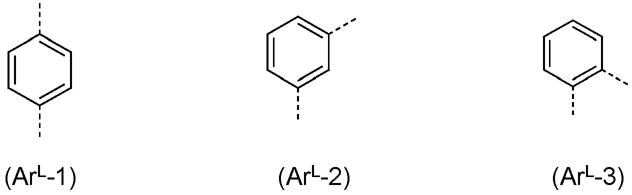 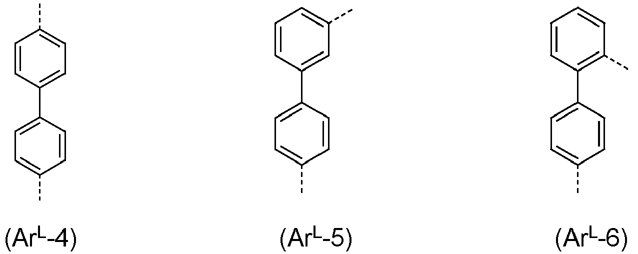 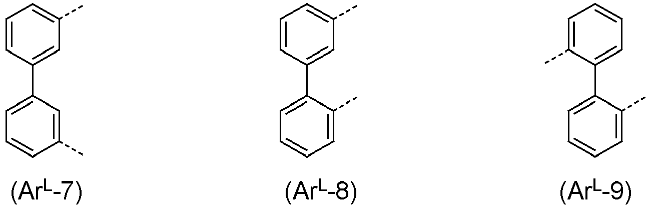 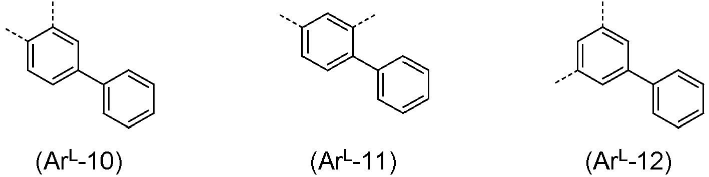 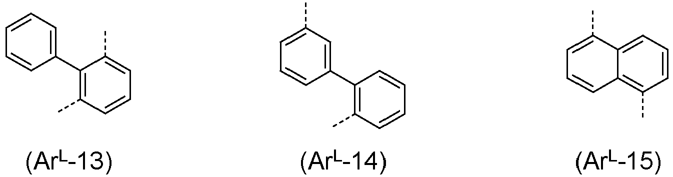 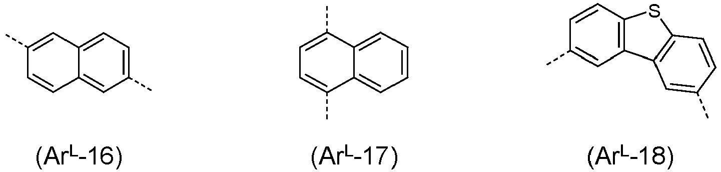 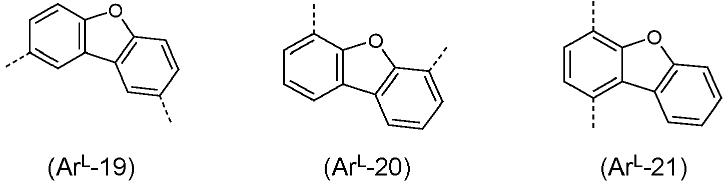 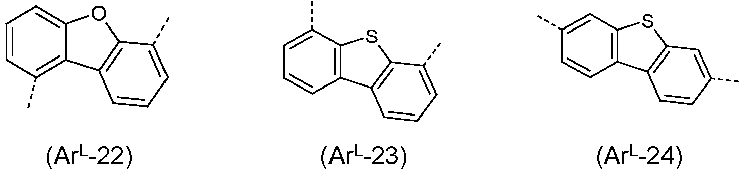 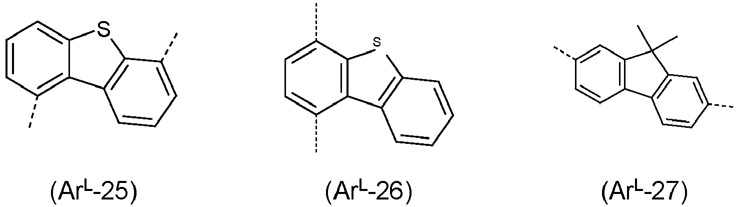 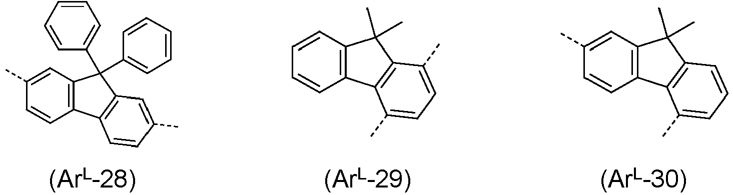 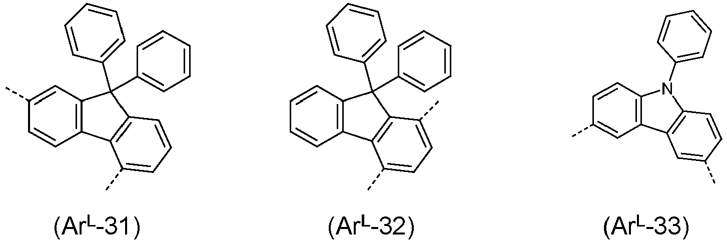 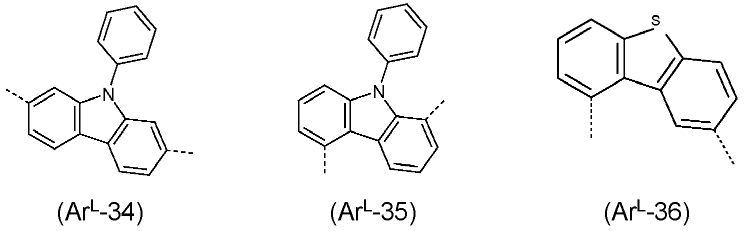 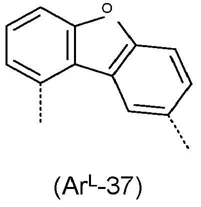 where the dashed bonds indicate the bonds to the spirobifluorene and to the amine, and where the groups (Ar^{L}-1) to (Ar^{L}-37) may be substituted at each free position by a group R² but are preferably unsubstituted.

Ar^{L} is particularly preferably a phenyl group, which may be substituted at each free position by a group R². In a very particularly preferred form, Ar^{L} is chosen from the groups (Ar^{L}-1) (Ar^{L}-2) and (Ar^{L}-3), which are unsubstituted.

In a preferred embodiment of the invention, the groups Ar¹ and Ar² are connected via a single bond or a divalent bridge and the group -NAr¹Ar² as depicted in formula (1) is selected from the groups of formulae (E-1) to (E-24), 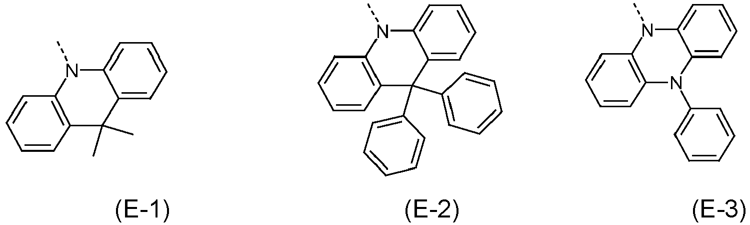 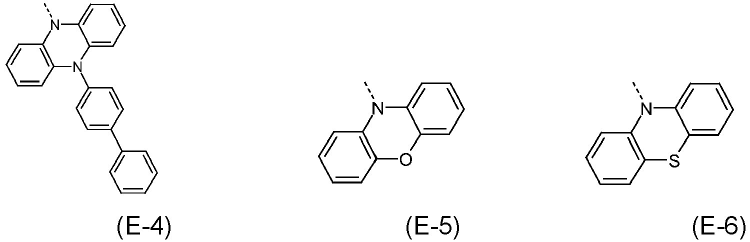 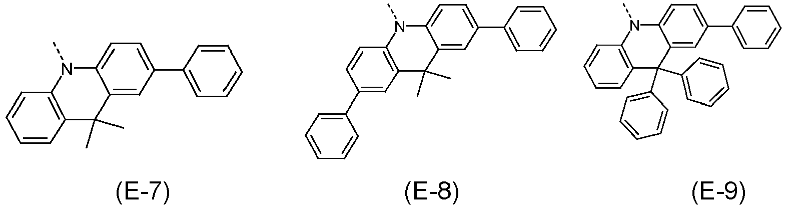 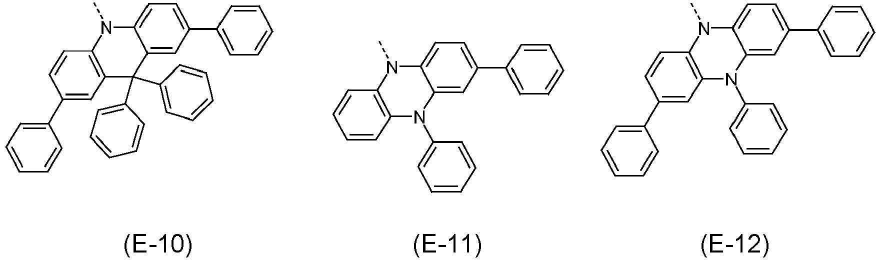 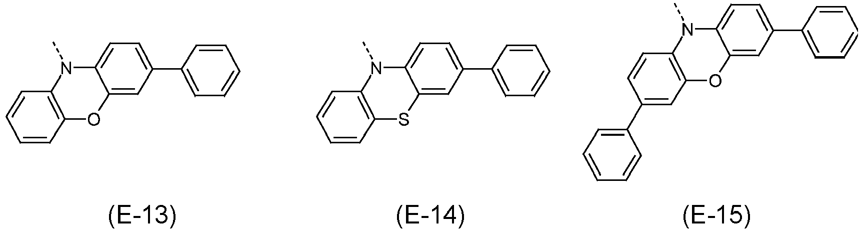 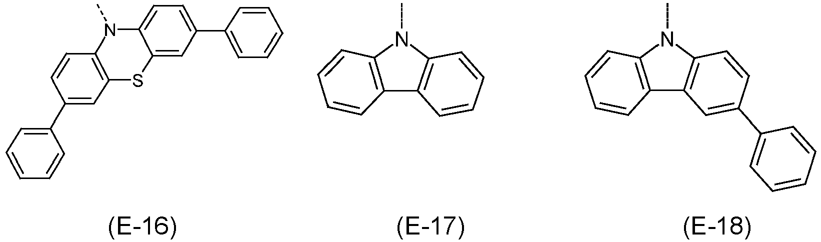 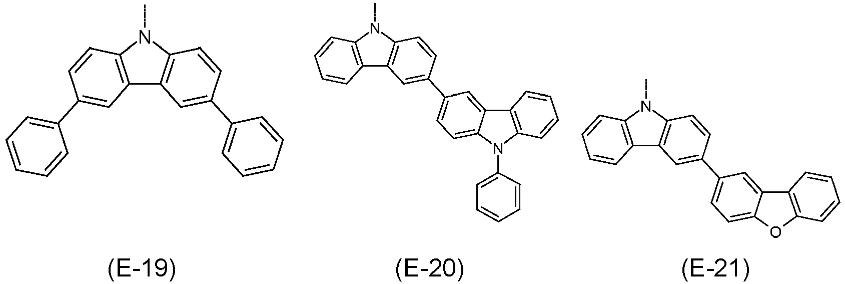 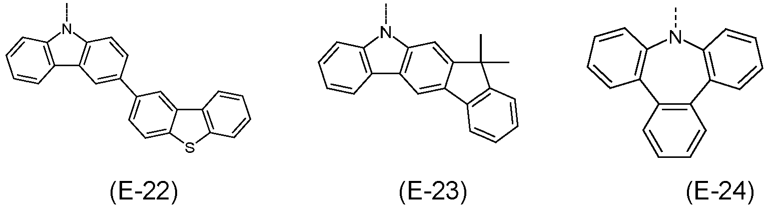 where the dashed bonds indicate the bonds to the spirobifluorene or to the group Ar^{L} when present, and where the groups (E-1) to (E-24) may be substituted at each free position by a group R² as defined above, but are preferably unsubstituted. From the groups (E-1) to (E-24), the following unsubstituted groups are preferred: (E-8), (E-10), (E-12), (E-15), (E-16), (E-18), (E-19), (E-20) and (E-23).

In a another preferred embodiment of the invention, the groups Ar¹ and Ar² are selected from the groups of the following formulae (A-1) to (A-48), 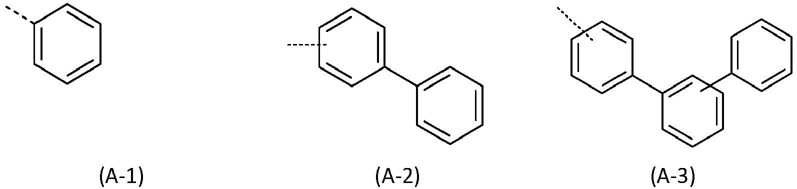 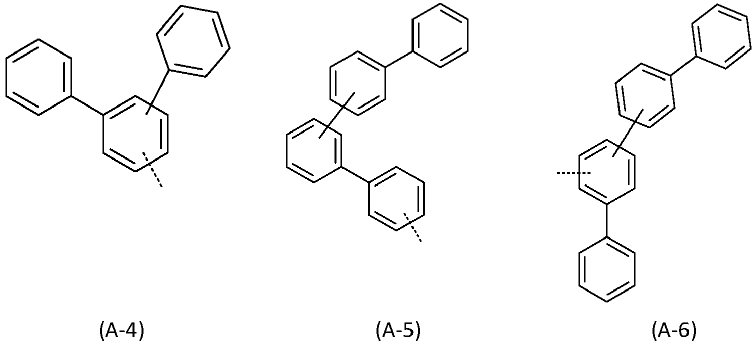 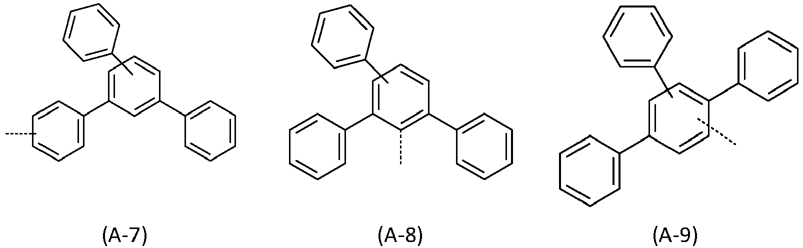 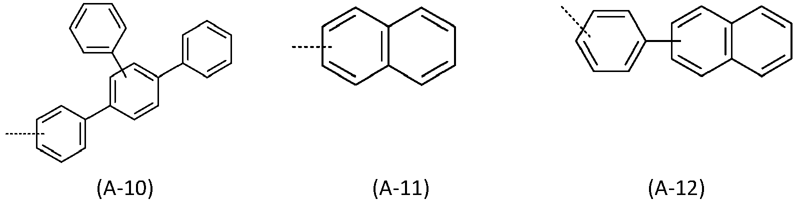 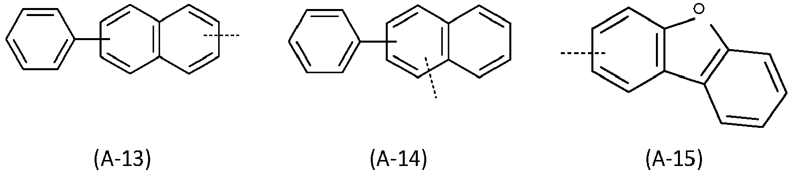 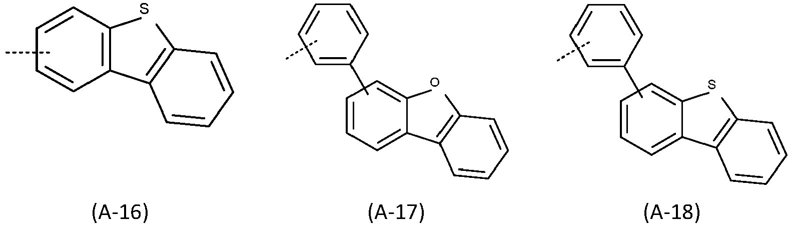 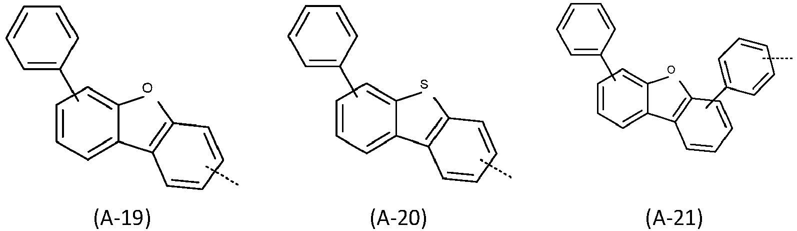 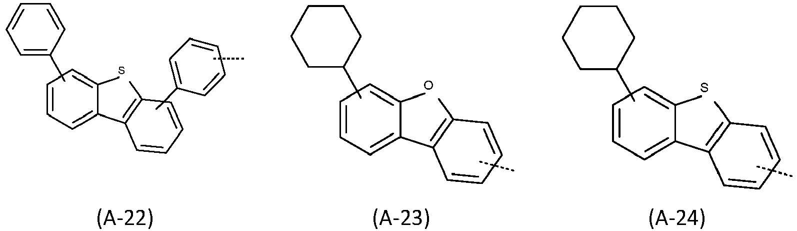 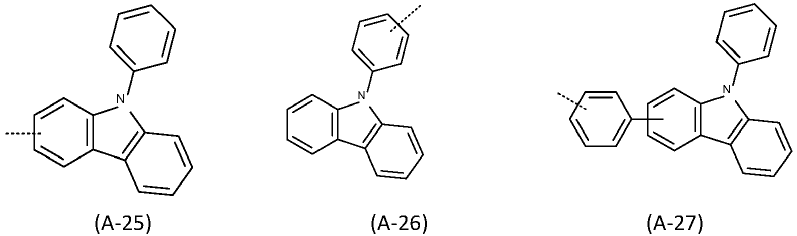 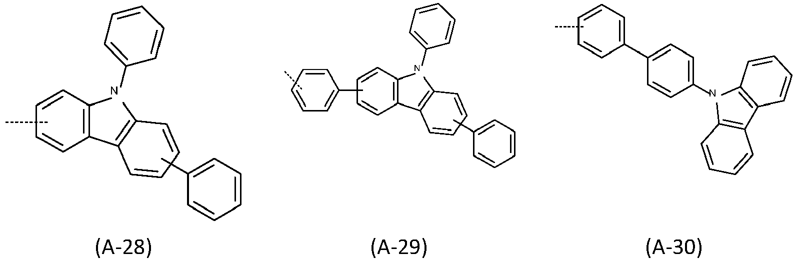 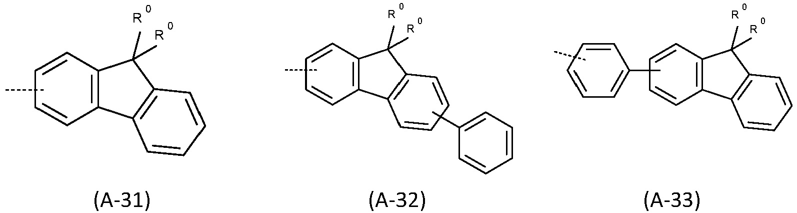 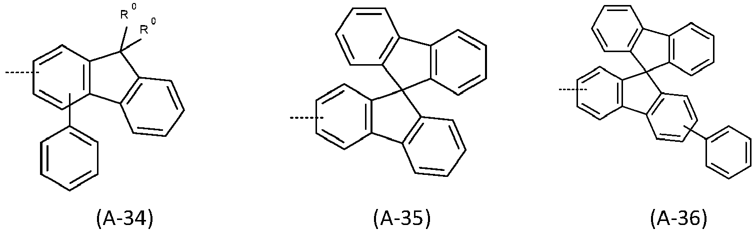 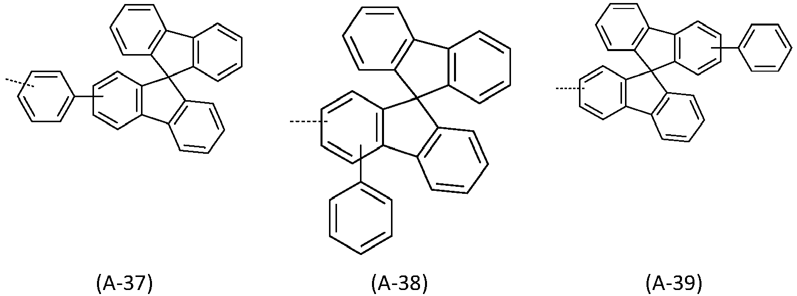 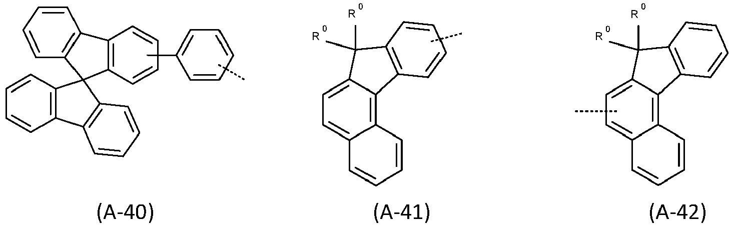 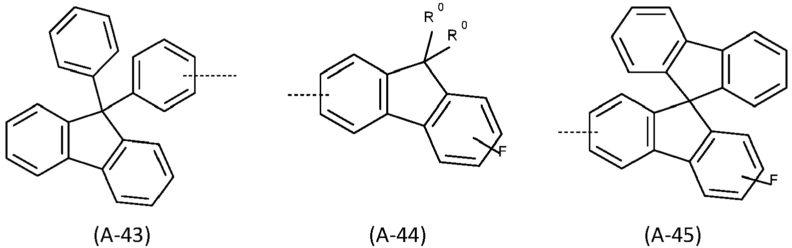 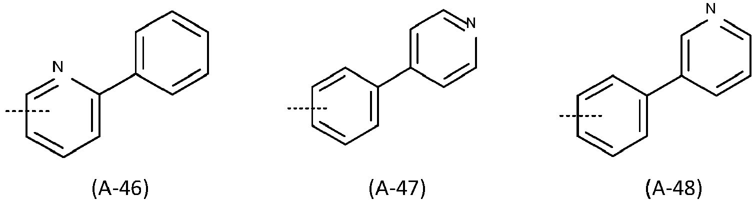 where the dashed bonds indicate the bonds to the nitrogen atom, where the groups of formulae (A-1) to (A-48) may further be substituted at each free position by a group R² as defined above but are preferably unsubstituted and
where the groups R⁰, in formulae (A-31) to (A-34), (A-41), (A-42) and (A-44), are selected on each occurrence, identically or differently, from the group consisting of H, D, F, CN, Si(R³)₃, a straight-chain alkyl group having 1 to 10 C atoms or a branched or cyclic alkyl group having 3 to 10 C atoms, each of which may be substituted by one or more radicals R³, an aryl or heteroaryl group having 5 to 18 aromatic ring atoms, which may in each case be substituted by one or more radicals R³, where two adjacent substituents R⁰ may optionally form a mono- or polycyclic, aliphatic ring system or aromatic ring system, which may be substituted by one or more radicals R³;

More preferably, the group Ar¹ and Ar² are selected from the groups (A-1), (A-2), (A-3), (A-15), (A-16), (A-17), (A-18), (A-25), (A-26), (A-31) or (A-35), which may be substituted at each free position by a group R² as defined above.

Suitable groups of formulae (A-1) to (A-48) are for example the groups (Ar-1) to (Ar-194), 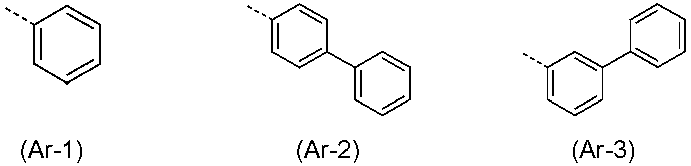 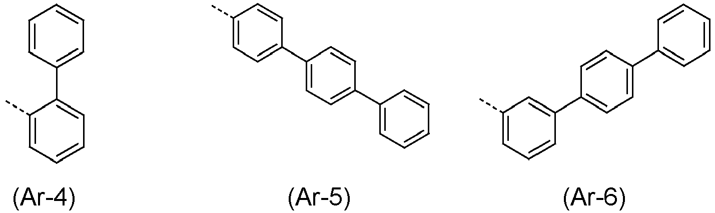 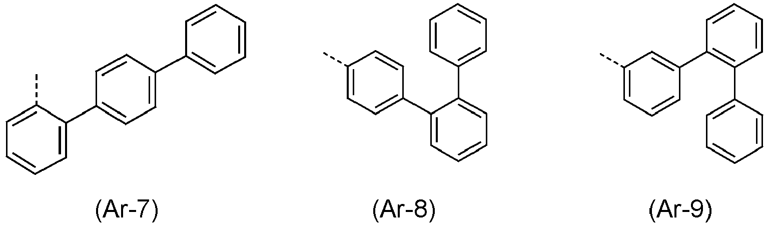 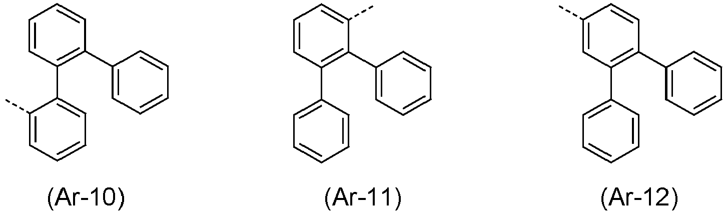 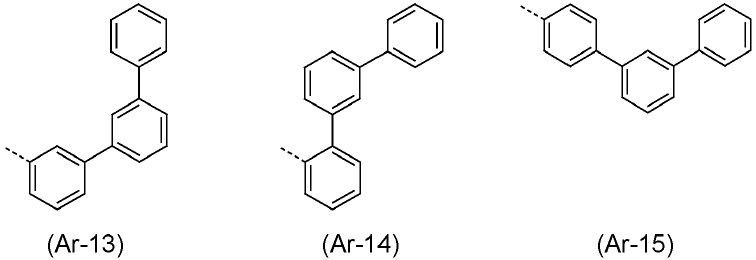 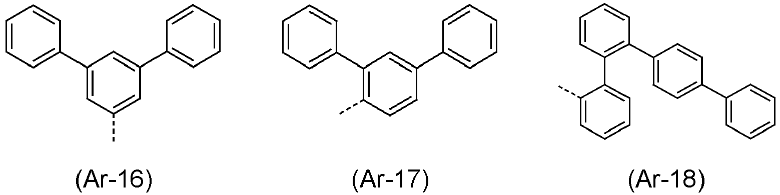 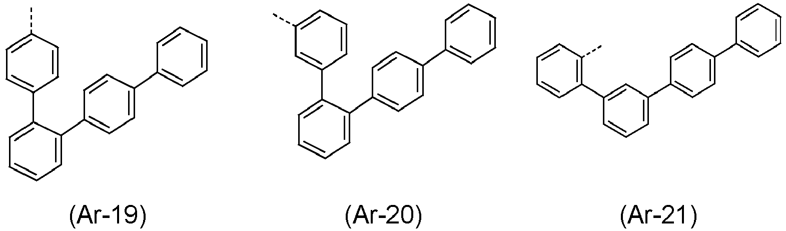 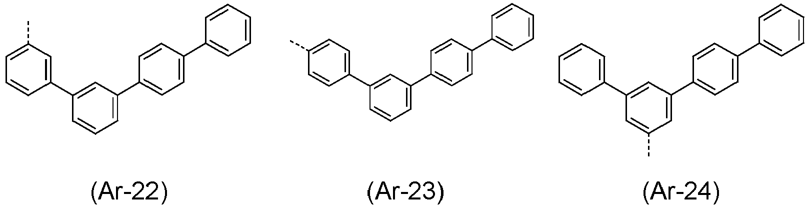 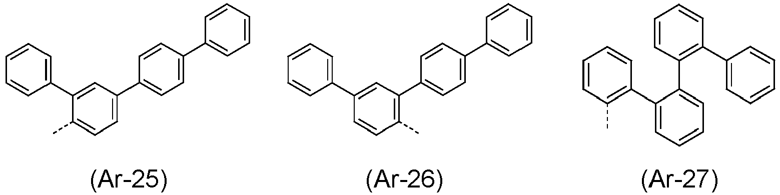 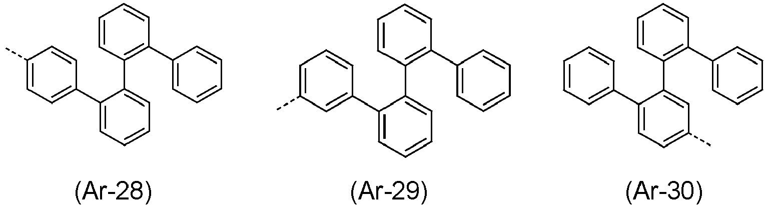 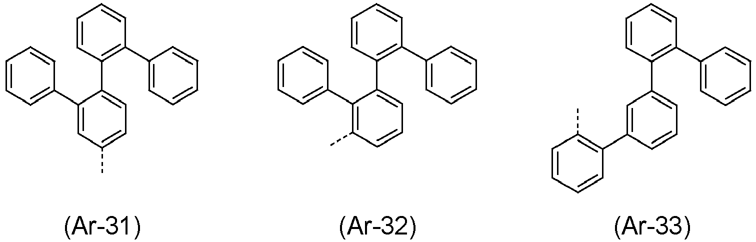 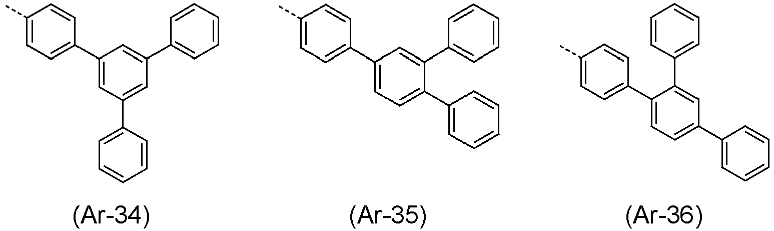 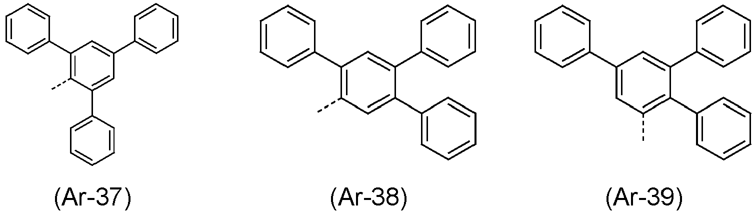 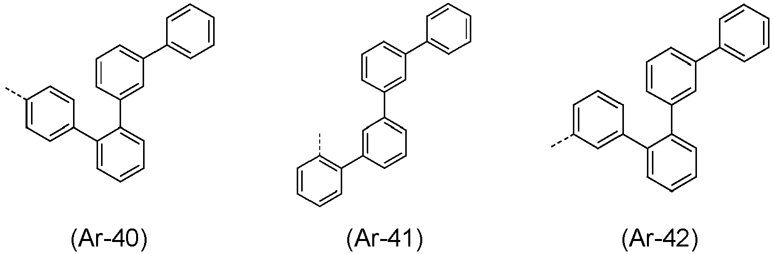 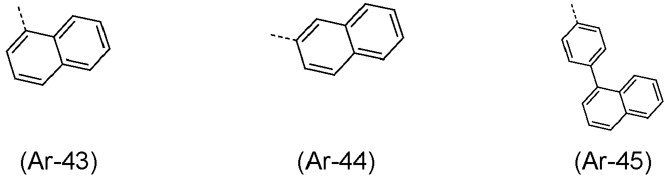 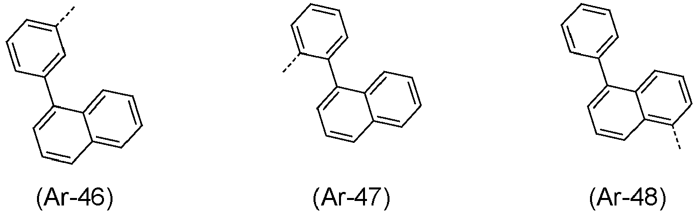 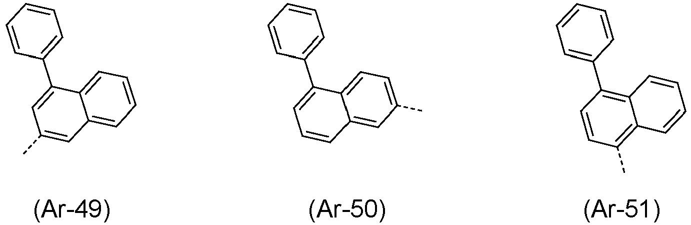 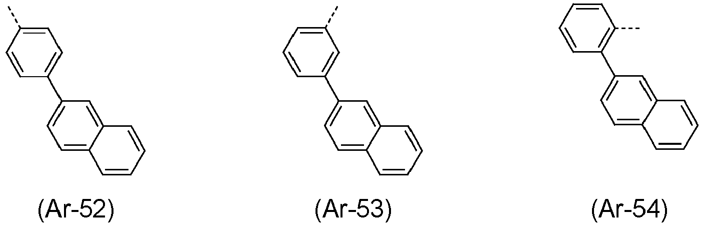 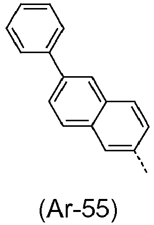 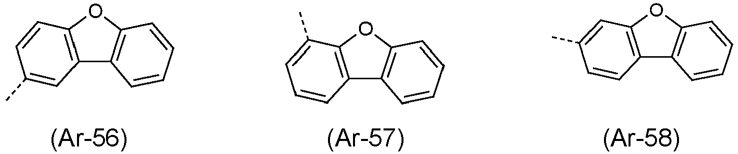 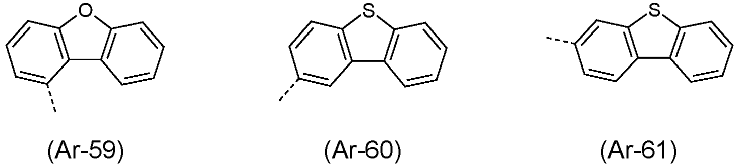 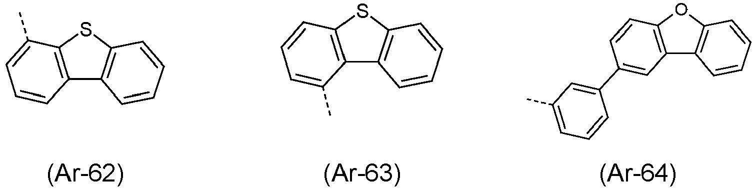 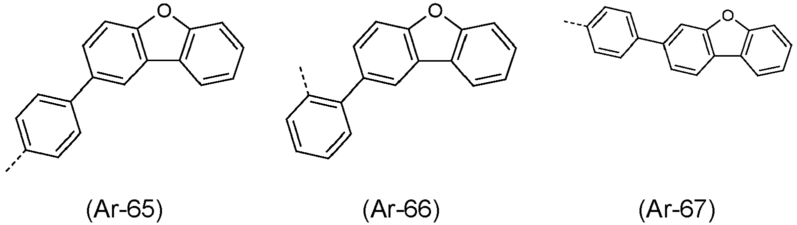 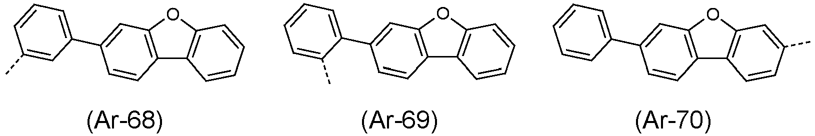 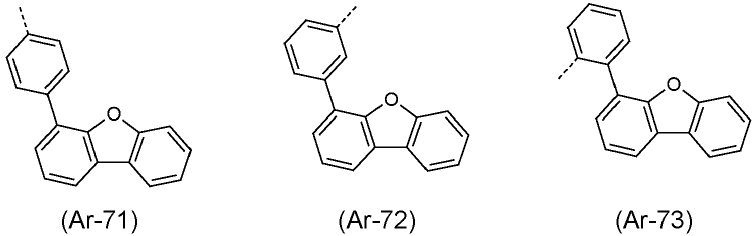 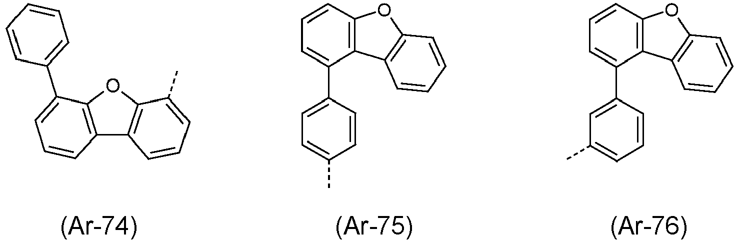 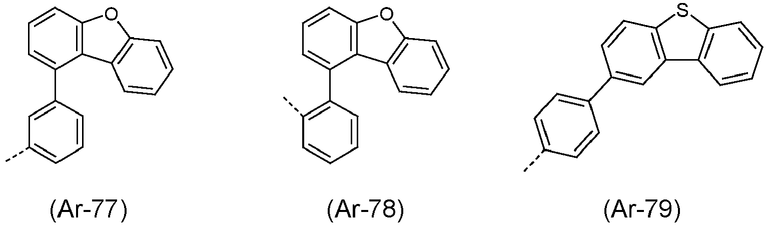 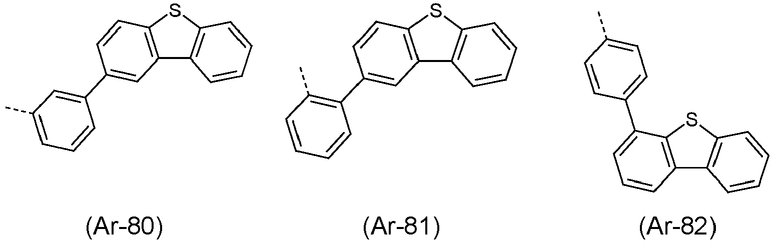 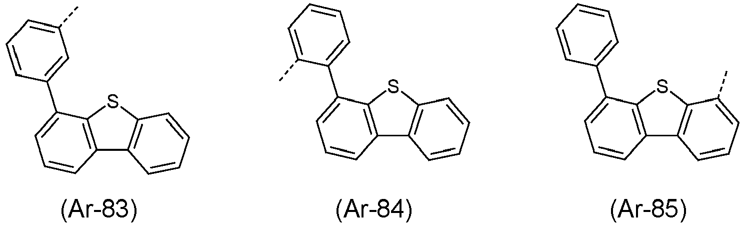 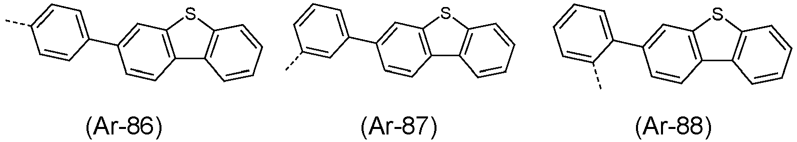 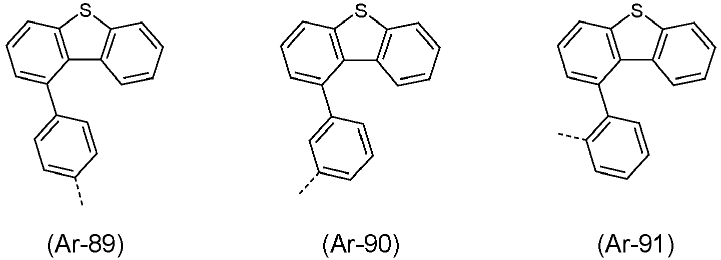 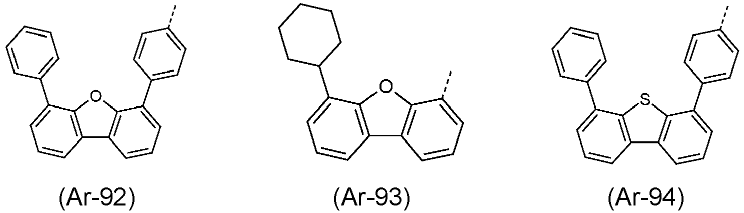 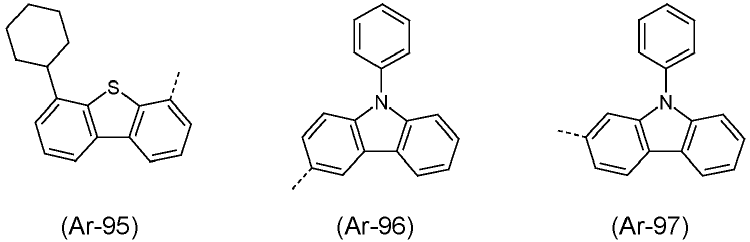 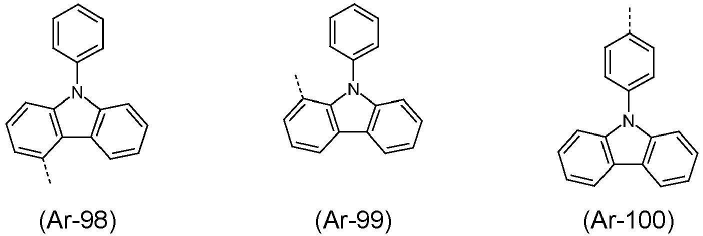 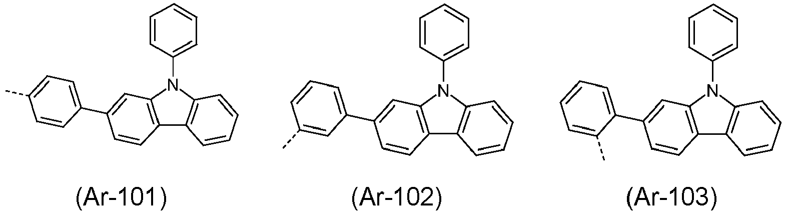 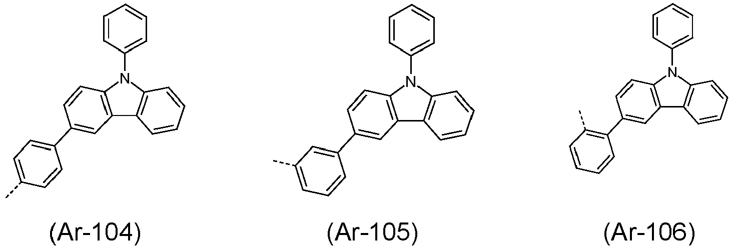 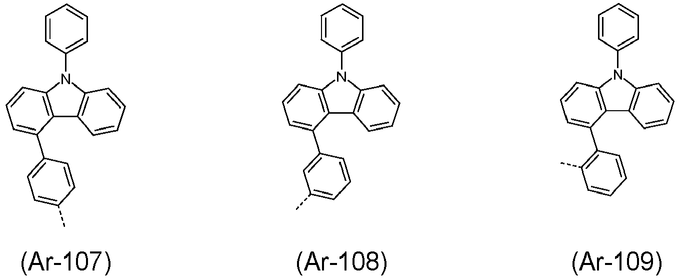 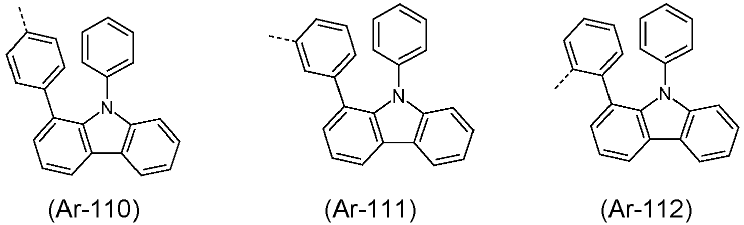 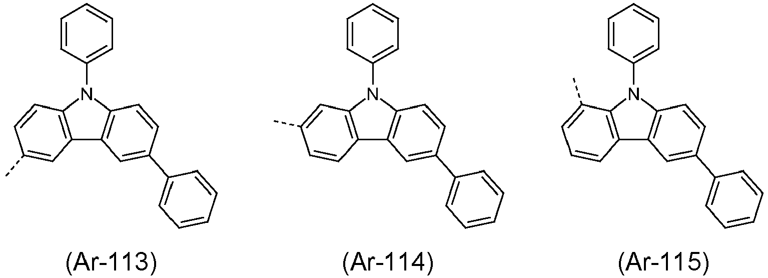 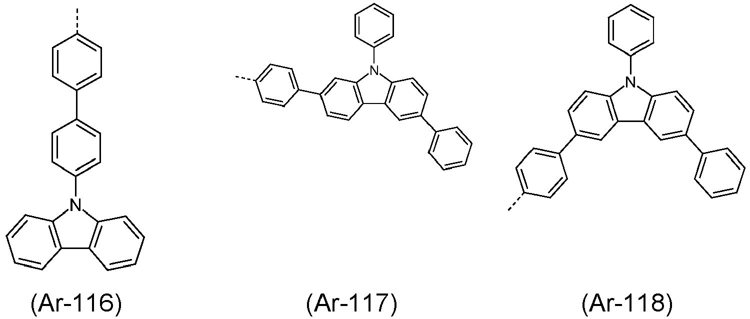 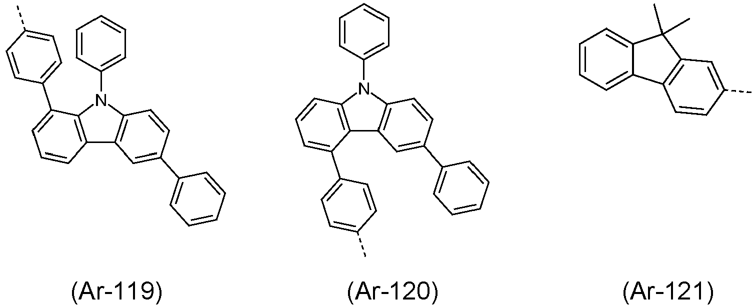 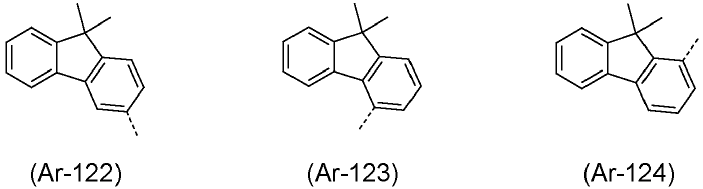 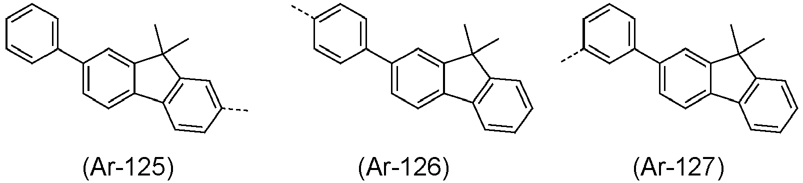 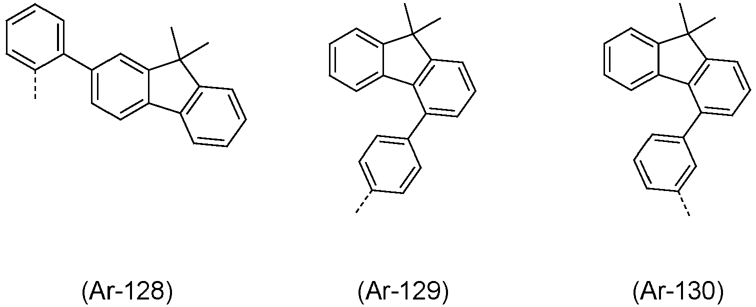 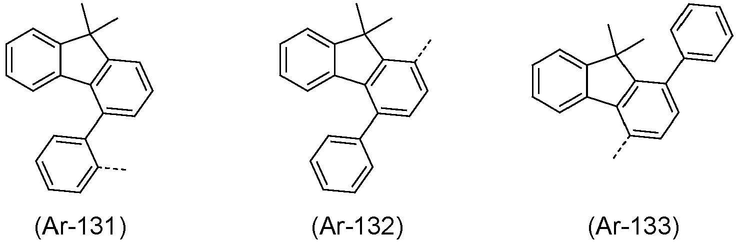 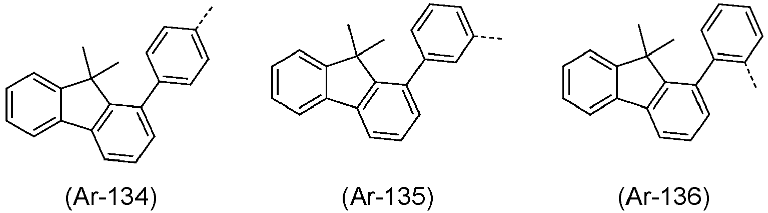 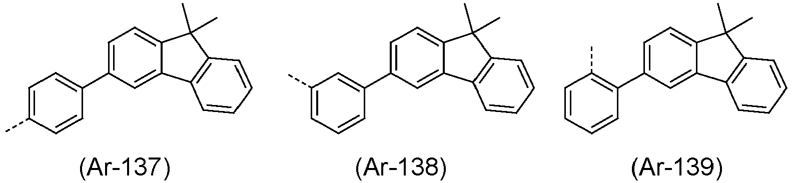 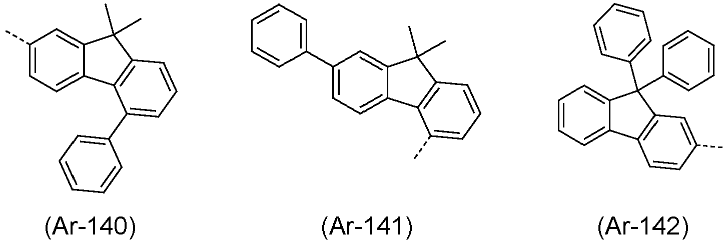 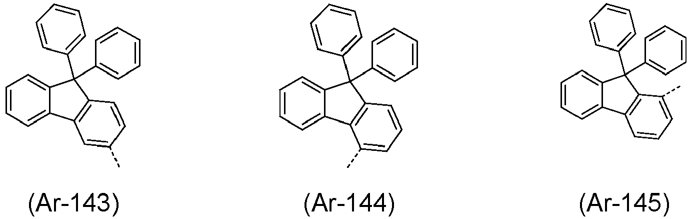 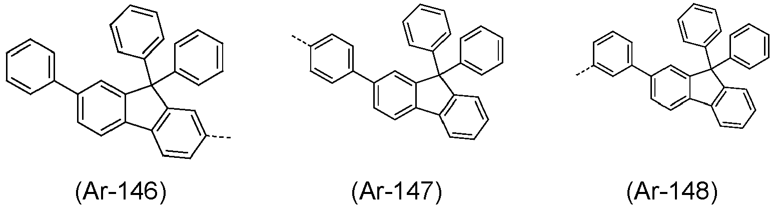 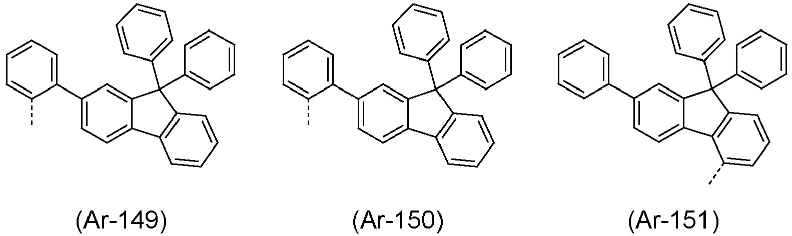 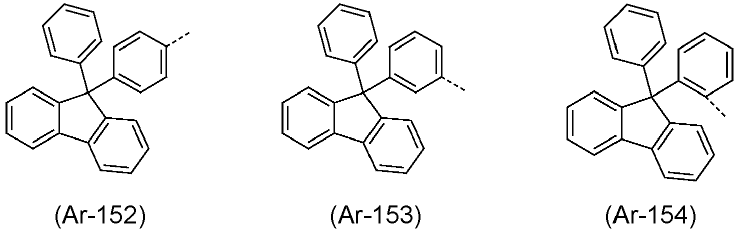 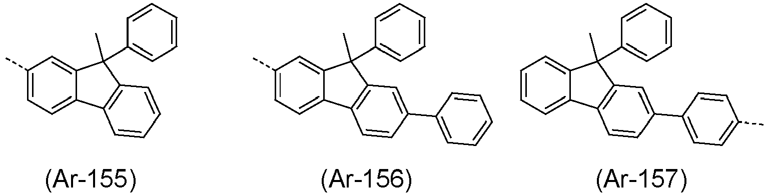 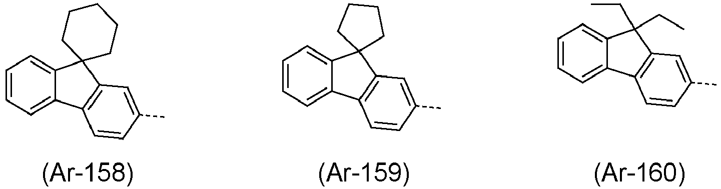 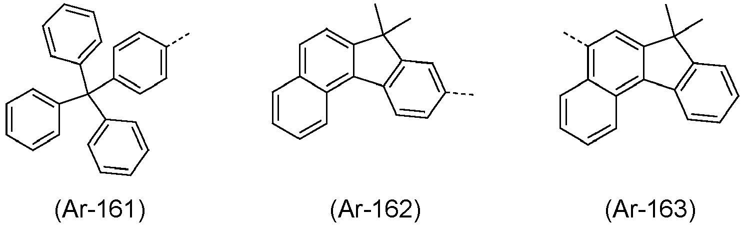 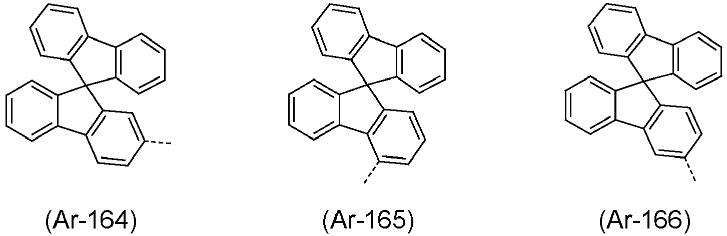 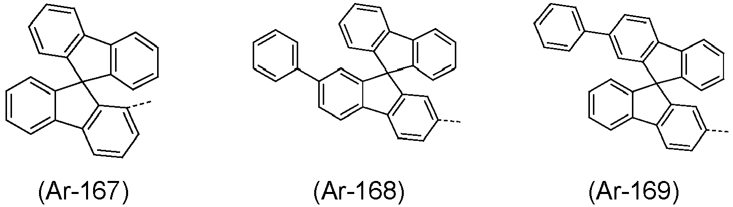 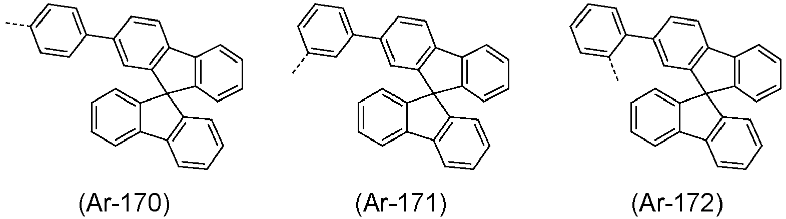 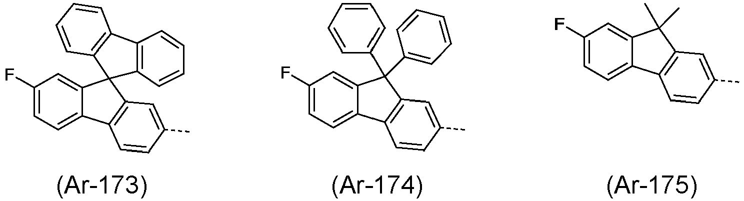 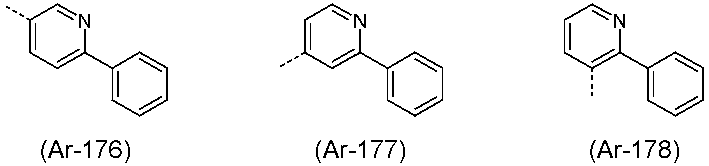 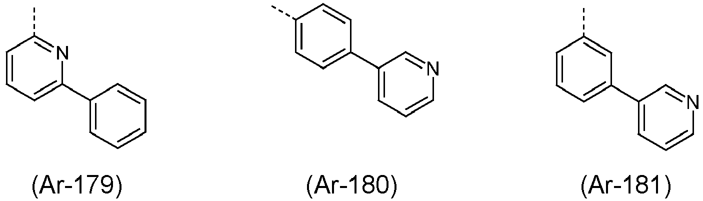 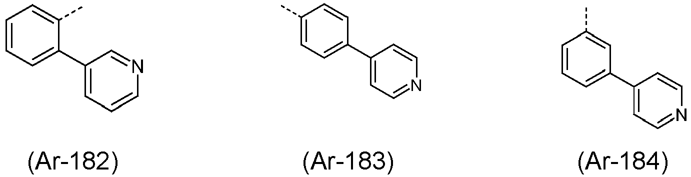 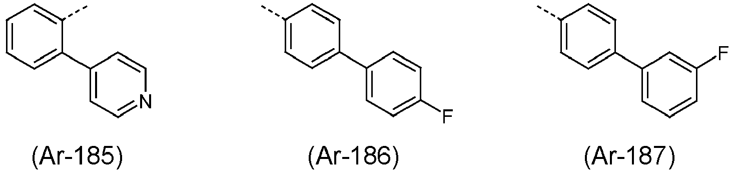 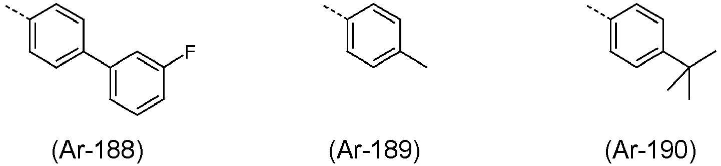 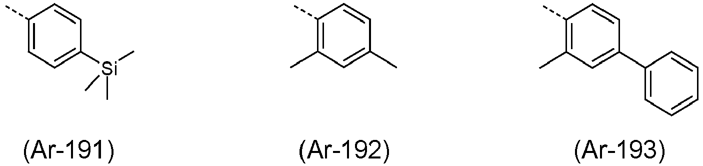 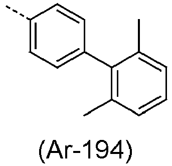 where the dashed bonds indicate the bonds to the nitrogen atom.

More preferably, the group Ar¹ is selected from the groups (Ar-2), (Ar-3), (Ar-4), (Ar-121), (Ar-123) or (Ar-142).

More preferably, the group Ar² is selected from the groups (Ar-1), (Ar-2), (Ar-3), (Ar-4), (Ar-56), (Ar-57), (Ar-60), (Ar-62), (Ar-71), (Ar-82), (Ar-96), (Ar-104), (Ar-121), (Ar-123), (Ar-142), (Ar-144), (Ar-164), (Ar-165).

Example of suitable groups R are following groups (R-1) to (R-19), 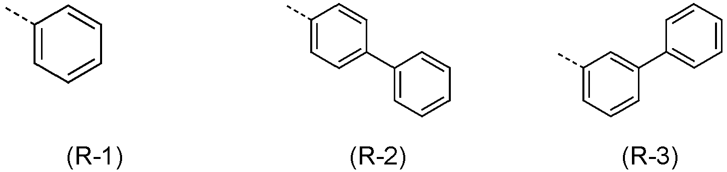 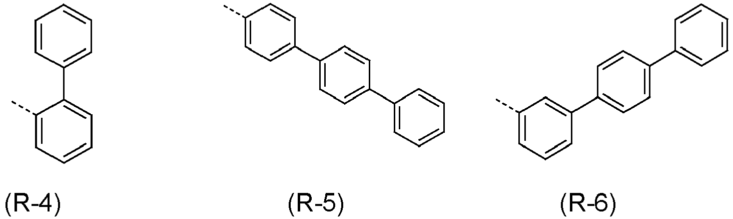 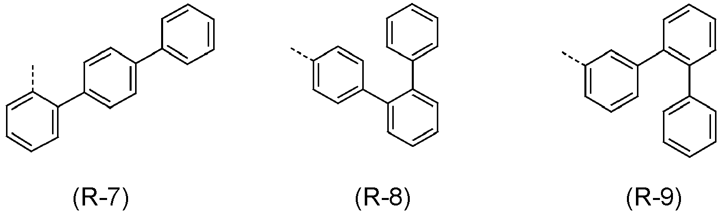 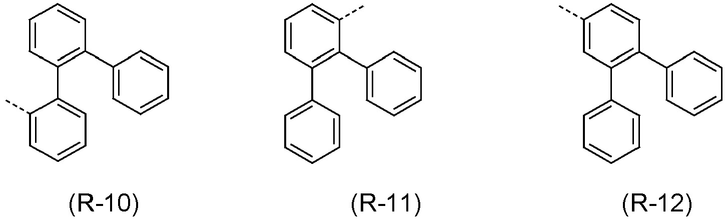 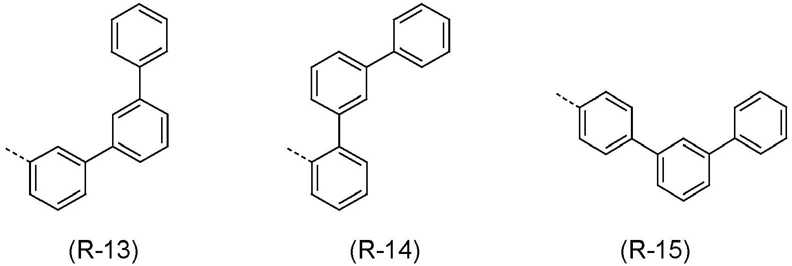 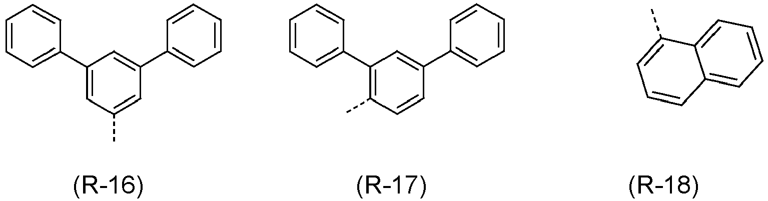 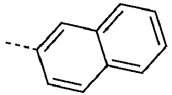 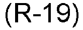 where the dashed bonds indicate the bonds to the nitrogen atom.

More preferably, the group R is an aromatic ring system selected from phenyl, biphenyl or naphtyl, even more preferably phenyl. It is particularly preferably that the group R is selected from the groups (R-1), (R-2), (R-3), (R-4), (R-18) or (R-19), more particularly from the groups (R-1), (R-2), (R-3) or (R-4), very particularly from the group (R-1).

In a preferred embodiment of the invention, R² is selected, identically or differently on each occurrence, from the group consisting of H, D, F, CN, a straight-chain alkyl or alkoxy group having 1 to 10 C atoms or a branched or cyclic alkyl or alkoxy group having 3 to 10 C atoms, each of which may be substituted by one or more radicals R³, where one or more non-adjacent CH₂ groups may be replaced by O and where one or more H atoms may be replaced by F, an aromatic or heteroaromatic ring system having 5 to 24 aromatic ring atoms, which may in each case be substituted by one or more radicals R³.

In a very preferred embodiment of the invention, R² is selected, identically or differently on each occurrence, from the group consisting of H, D, F, CN, a straight-chain alkyl having 1 to 5 C atoms or a branched or cyclic alkyl group having 3 to 6 C atoms, or an aryl or heteroaryl group having 5 to 18 aromatic ring atoms, which may in each case be substituted by one or more radicals R³_{.}

In a preferred embodiment of the invention, R³ is selected, identically or differently on each occurrence, from the group consisting of H, D, F, CN, a straight-chain alkyl or alkoxy group having 1 to 10 C atoms or a branched or cyclic alkyl or alkoxy group having 3 to 10 C atoms, each of which may be substituted by one or more radicals R⁴, where one or more non-adjacent CH₂ groups may be replaced by O and where one or more H atoms may be replaced by F, an aromatic or heteroaromatic ring system having 5 to 24 aromatic ring atoms, which may in each case be substituted by one or more radicals R⁴.

In a very preferred embodiment of the invention, R³ is selected, identically or differently on each occurrence, from the group consisting of H, D, F, CN, a straight-chain alkyl having 1 to 5 C atoms or a branched or cyclic alkyl group having 3 to 6 C atoms, or an aryl or heteroaryl group having 5 to 18 aromatic ring atoms, which may in each case be substituted by one or more radicals R⁴.

In accordance with a preferred embodiment of the invention, the compounds of formula (1) are selected from the compounds of the following formulae (1-1) to (1-24), 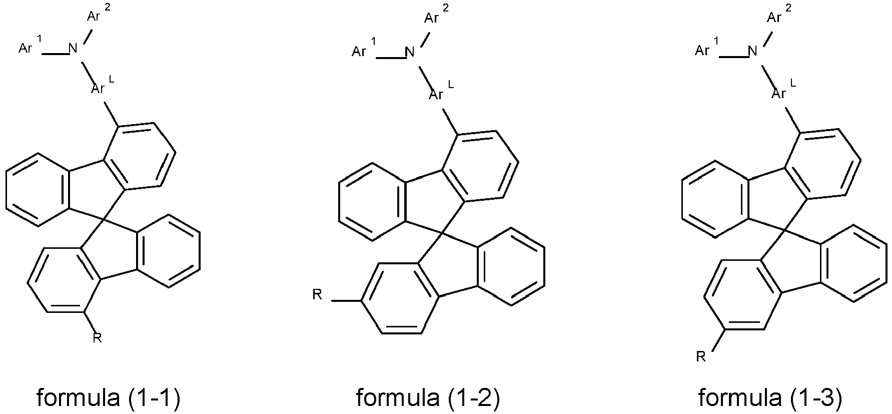 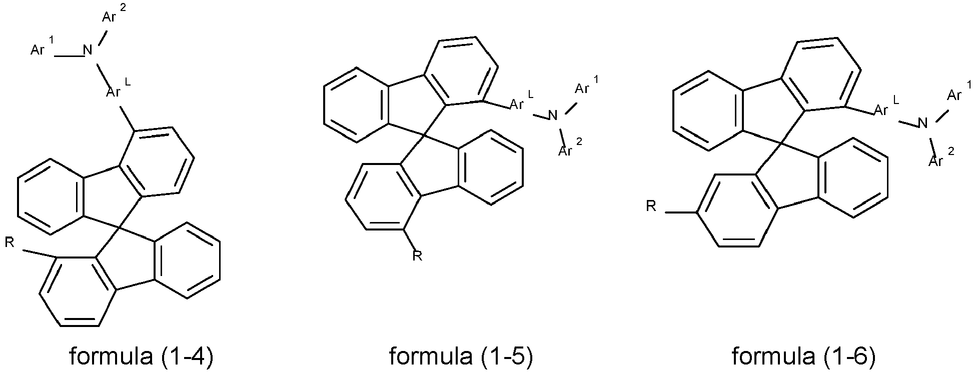 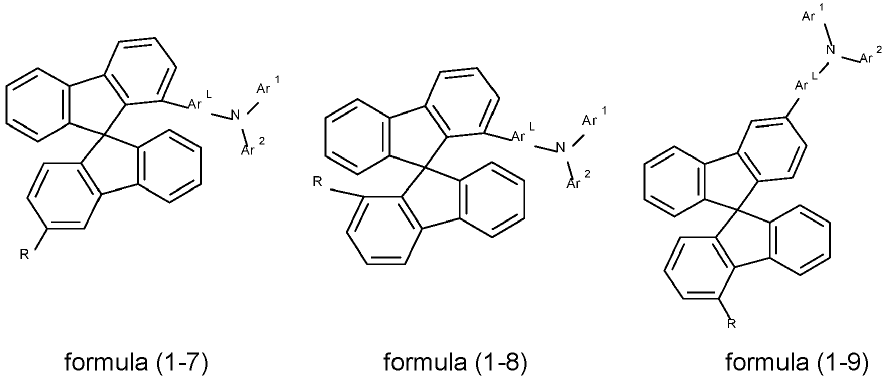 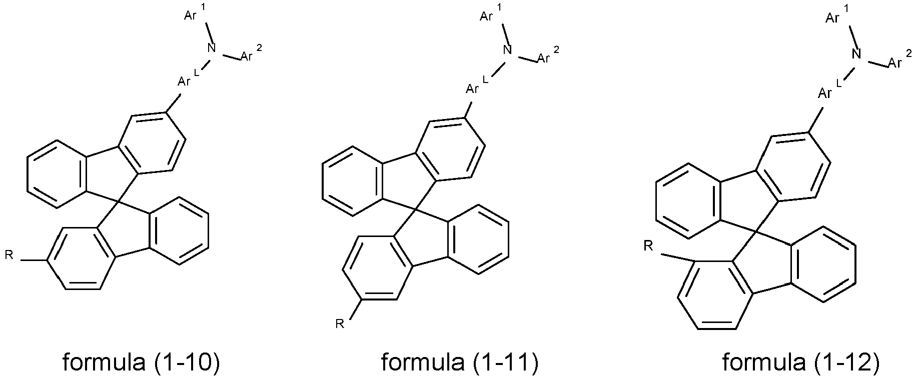 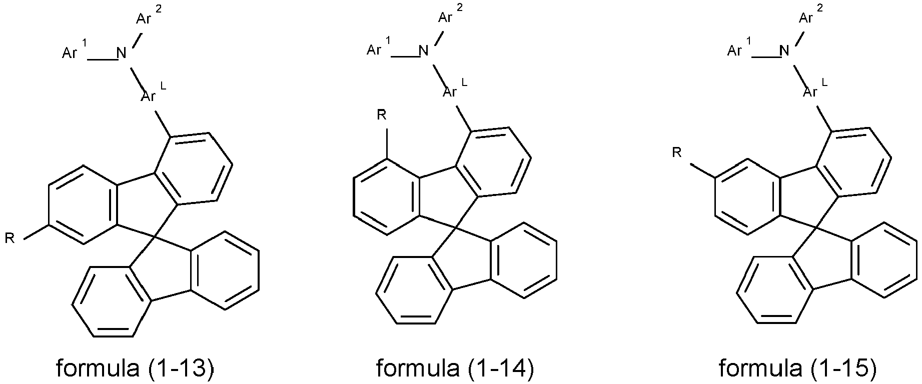 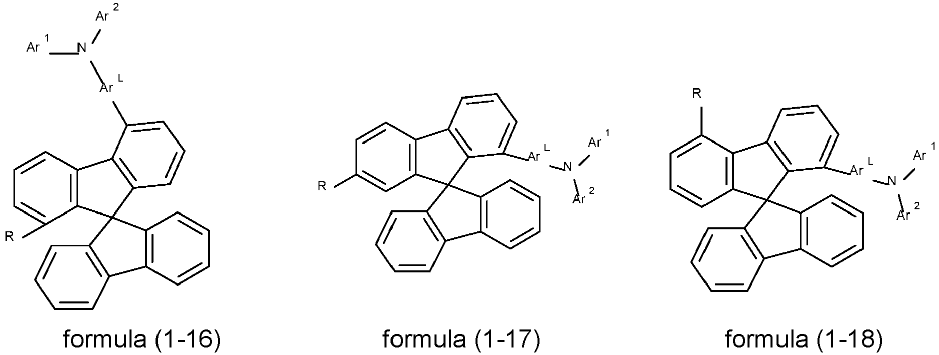 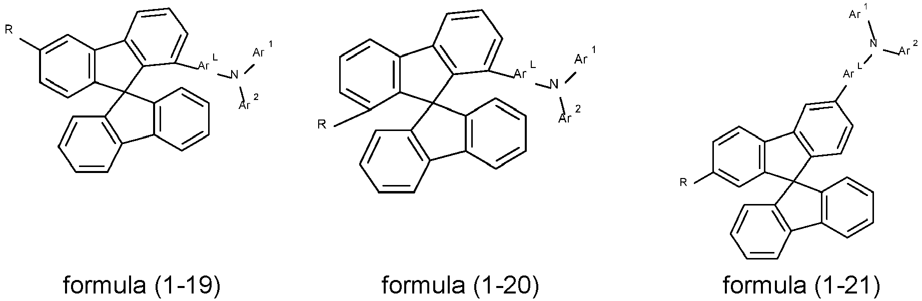 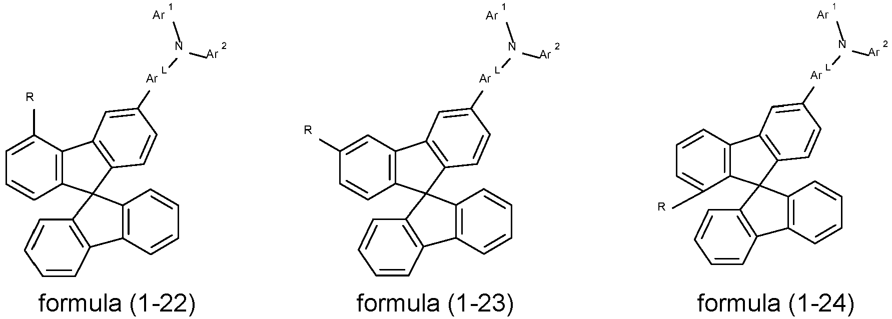 where the symbols and indices used have the meaning as given above.

In accordance with a further preferred embodiment of the invention, the compounds of formula (1) are selected from the compounds of the following formulae (1-31) to (1-54),

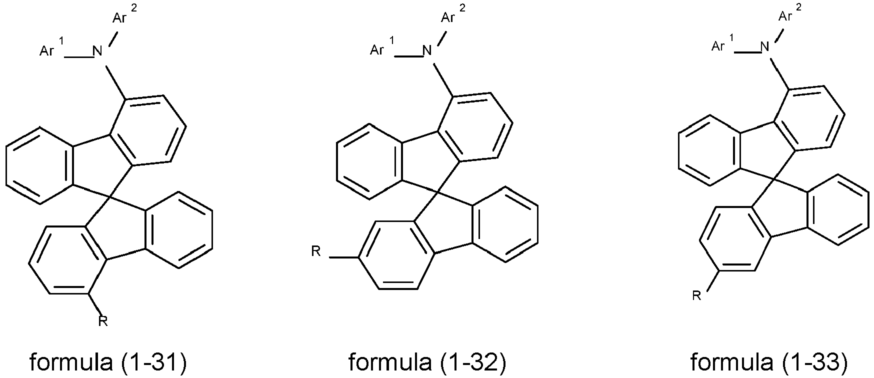

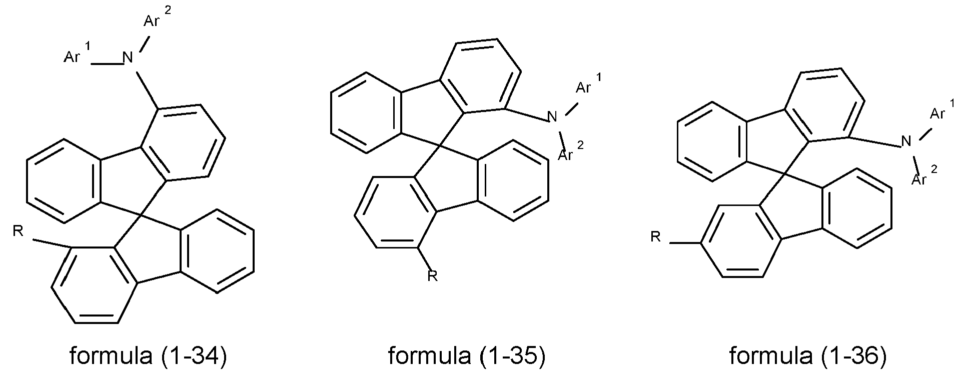

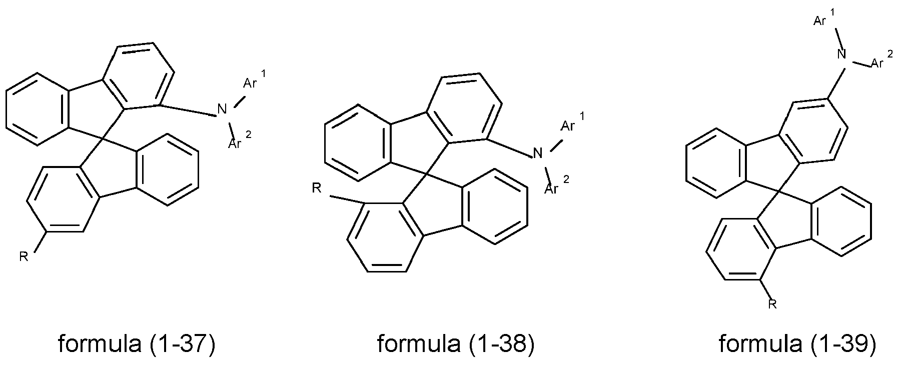

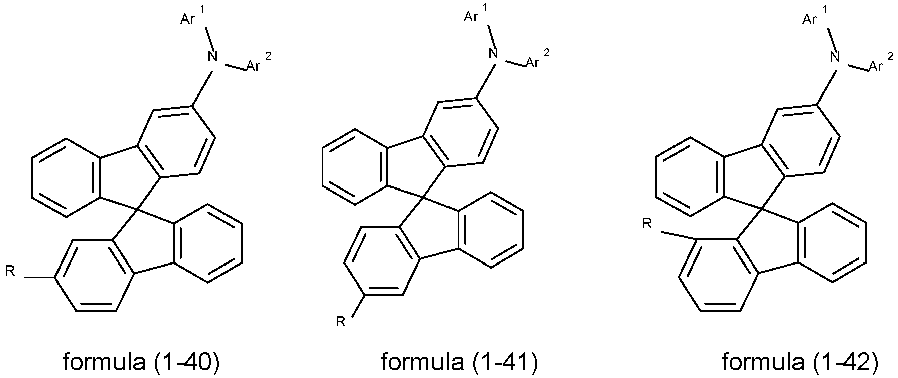

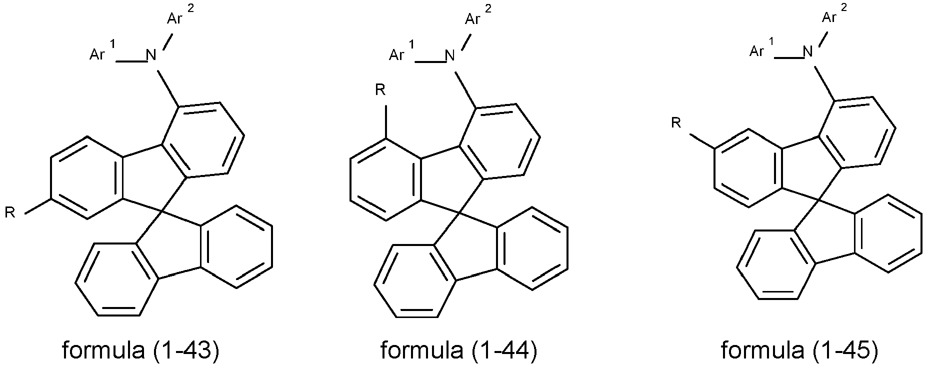

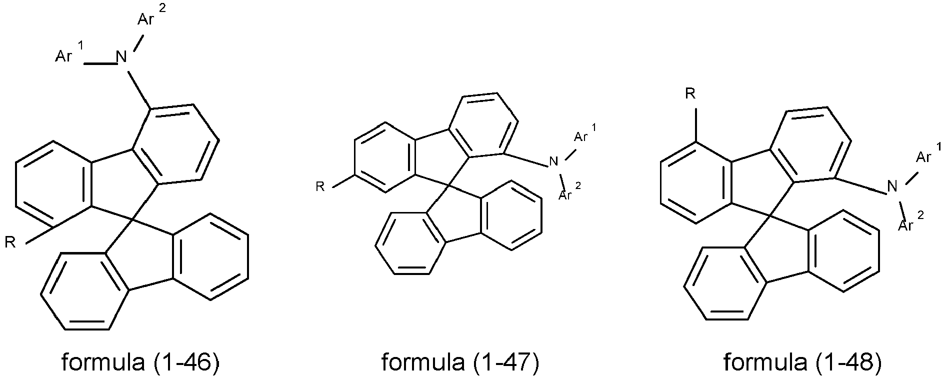

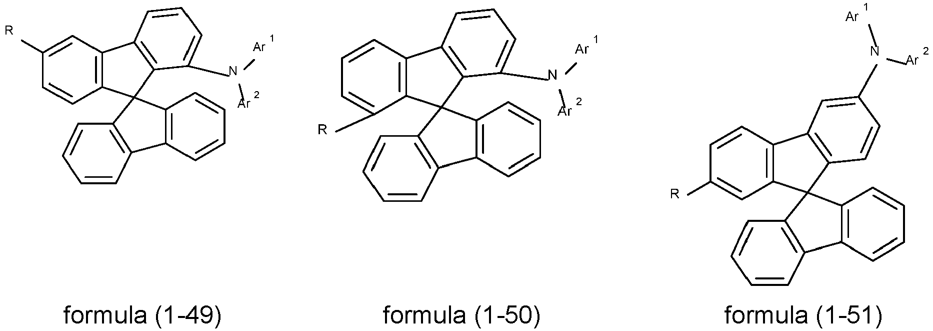

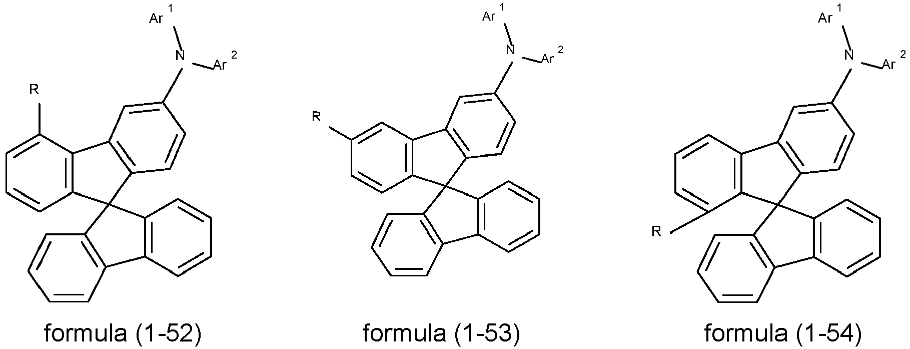

Among formulae (1-1) to (1-60), formulae (1-1) to (1-12) and (1-31) to (1-42) are preferred. Formulae (1-1) to (1-4) und (1-31) to (1-34) are particularly preferred. Very particularly preferred formulae are formulae (1-1), (1-2), (1-31) and (1-32).

Particular preference is given to compounds of the formulae (1) and (1-1) to (1-24) and (1-31) to (I-54), in which the preferred embodiments mentioned above occur simultaneously. Particular preference is therefore given to compounds for which:
R is selected from the groups (R-1), (R-2), (R-3), (R-4), (R-18) or (R-19) as as depicted above,
Ar¹, Ar² are on each occurrence, identically or differently, selected from the groups of formulae (Ar-1) to (Ar-194) as depicted above, which may be substituted by one or more radicals R², or Ar¹ and Ar² are connected via a single bond or a divalent bridge and the group -NAr¹Ar² as depicted in formula (1) is selected from the groups of formulae (E-1) to (E-24) as depicted above;
Ar^{L} is a phenyl group, which may be substituted at each free position by a group R²;
R² is selected, identically or differently on each occurrence, from the group consisting of H, D, F, CN, a straight-chain alkyl or alkoxy group having 1 to 10 C atoms or a branched or cyclic alkyl or alkoxy group having 3 to 10 C atoms, each of which may be substituted by one or more radicals R³, where one or more non-adjacent CH₂ groups may be replaced by O and where one or more H atoms may be replaced by F, an aromatic or heteroaromatic ring system having 5 to 24 aromatic ring atoms, which may in each case be substituted by one or more radicals R³;
R³ is selected, identically or differently on each occurrence, from the group consisting of H, D, F, CN, a straight-chain alkyl or alkoxy group having 1 to 10 C atoms or a branched or cyclic alkyl or alkoxy group having 3 to 10 C atoms, each of which may be substituted by one or more radicals R⁴, where one or more non-adjacent CH₂ groups may be replaced by O and where one or more H atoms may be replaced by F, an aromatic or heteroaromatic ring system having 5 to 24 aromatic ring atoms, which may in each case be substituted by one or more radicals R⁴,
R⁴ is selected on each occurrence, identically or differently, from the group consisting of H, D, F, Cl, Br, I, CN, a straight-chain alkyl, alkoxy or thioalkyl group having 1 to 20 C atoms or a branched or cyclic alkyl, alkoxy or thioalkyl group having 3 to 20 C atoms, where in each case one or more non-adjacent CH₂ groups may be replaced by SO, SO₂, O, S and where one or more H atoms may be replaced by D, F, Cl, Br or I, an aromatic or heteroaromatic ring system having 5 to 24 C atoms;
Ar³ is an aromatic or heteroaromatic ring system having 5 to 24 aromatic ring atoms, more preferably having 5 to 18 aromatic ring atoms, which may in each case also be substituted by one or more radicals R⁴;
i is 0 or 1.

For compounds which are processed by vacuum evaporation, the alkyl groups preferably have not more than four C atoms, particularly preferably not more than 1 C atom. For compounds which are processed from solution, suitable compounds are also those which are substituted by linear, branched or cyclic alkyl groups having up to 10 C atoms or which are substituted by oligoarylene groups, for example ortho-, meta-, para- or branched terphenyl or quaterphenyl groups.

Examples of preferred structures for compounds according to fomula (1) are compounds of formula (1-1), (1-2), (1-31), (1-32),
where Ar^{L} in formulae (1-1) and (1-2) is an unsubstituted phenyl group of formula (Ar^{L}-1), (Ar^{L}-2) or (Ar^{L}-3); and
where the groups R, Ar¹ and Ar² in formulae (1-1), (1-2), (1-31) and (1-32) are combined as listed in the table below:

| R | Ar¹ | Ar² |
|---|---|---|
| (R-1) | (Ar-2) | (Ar-1) |
| (R-1) | (Ar-2) | (Ar-2) |
| (R-1) | (Ar-2) | (Ar-3) |
| (R-1) | (Ar-2) | (Ar-4) |
| (R-1) | (Ar-2) | (Ar-56) |
| (R-1) | (Ar-2) | (Ar-57) |
| (R-1) | (Ar-2) | (Ar-60) |
| (R-1) | (Ar-2) | (Ar-62) |
| (R-1) | (Ar-2) | (Ar-71) |
| (R-1) | (Ar-2) | (Ar-82) |
| (R-1) | (Ar-2) | (Ar-96) |
| (R-1) | (Ar-2) | (Ar-104) |
| (R-1) | (Ar-2) | (Ar-121) |
| (R-1) | (Ar-2) | (Ar-123) |
| (R-1) | (Ar-2) | (Ar-142) |
| (R-1) | (Ar-2) | (Ar-144) |
| (R-1) | (Ar-2) | (Ar-164) |
| (R-1) | (Ar-2) | (Ar-165) |
| (R-1) | (Ar-3) | (Ar-1) |
| (R-1) | (Ar-3) | (Ar-2) |
| (R-1) | (Ar-3) | (Ar-3) |
| (R-1) | (Ar-3) | (Ar-4) |
| (R-1) | (Ar-3) | (Ar-56) |
| (R-1) | (Ar-3) | (Ar-57) |
| (R-1) | (Ar-3) | (Ar-60) |
| (R-1) | (Ar-3) | (Ar-62) |
| (R-1) | (Ar-3) | (Ar-71) |
| (R-1) | (Ar-3) | (Ar-82) |
| (R-1) | (Ar-3) | (Ar-96) |
| (R-1) | (Ar-3) | (Ar-104) |
| (R-1) | (Ar-3) | (Ar-121) |
| (R-1) | (Ar-3) | (Ar-123) |
| (R-1) | (Ar-3) | (Ar-142) |
| (R-1) | (Ar-3) | (Ar-144) |
| (R-1) | (Ar-3) | (Ar-164) |
| (R-1) | (Ar-3) | (Ar-165) |
| (R-1) | (Ar-4) | (Ar-1) |
| (R-1) | (Ar-4) | (Ar-2) |
| (R-1) | (Ar-4) | (Ar-3) |
| (R-1) | (Ar-4) | (Ar-4) |
| (R-1) | (Ar-4) | (Ar-56) |
| (R-1) | (Ar-4) | (Ar-57) |
| (R-1) | (Ar-4) | (Ar-60) |
| (R-1) | (Ar-4) | (Ar-62) |
| (R-1) | (Ar-4) | (Ar-71) |
| (R-1) | (Ar-4) | (Ar-82) |
| (R-1) | (Ar-4) | (Ar-96) |
| (R-1) | (Ar-4) | (Ar-104) |
| (R-1) | (Ar-4) | (Ar-121) |
| (R-1) | (Ar-4) | (Ar-123) |
| (R-1) | (Ar-4) | (Ar-142) |
| (R-1) | (Ar-4) | (Ar-144) |
| (R-1) | (Ar-4) | (Ar-164) |
| (R-1) | (Ar-4) | (Ar-165) |
| (R-1) | (Ar-121) | (Ar-1) |
| (R-1) | (Ar-121) | (Ar-2) |
| (R-1) | (Ar-121) | (Ar-3) |
| (R-1) | (Ar-121) | (Ar-4) |
| (R-1) | (Ar-121) | (Ar-56) |
| (R-1) | (Ar-121) | (Ar-57) |
| (R-1) | (Ar-121) | (Ar-60) |
| (R-1) | (Ar-121) | (Ar-62) |
| (R-1) | (Ar-121) | (Ar-71) |
| (R-1) | (Ar-121) | (Ar-82) |
| (R-1) | (Ar-121) | (Ar-96) |
| (R-1) | (Ar-121) | (Ar-104) |
| (R-1) | (Ar-121) | (Ar-121) |
| (R-1) | (Ar-121) | (Ar-123) |
| (R-1) | (Ar-121) | (Ar-142) |
| (R-1) | (Ar-121) | (Ar-144) |
| (R-1) | (Ar-121) | (Ar-164) |
| (R-1) | (Ar-121) | (Ar-165) |
| (R-1) | (Ar-123) | (Ar-1) |
| (R-1) | (Ar-123) | (Ar-2) |
| (R-1) | (Ar-123) | (Ar-3) |
| (R-1) | (Ar-123) | (Ar-4) |
| (R-1) | (Ar-123) | (Ar-56) |
| (R-1) | (Ar-123) | (Ar-57) |
| (R-1) | (Ar-123) | (Ar-60) |
| (R-1) | (Ar-123) | (Ar-62) |
| (R-1) | (Ar-123) | (Ar-71) |
| (R-1) | (Ar-123) | (Ar-82) |
| (R-1) | (Ar-123) | (Ar-96) |
| (R-1) | (Ar-123) | (Ar-104) |
| (R-1) | (Ar-123) | (Ar-121) |
| (R-1) | (Ar-123) | (Ar-123) |
| (R-1) | (Ar-123) | (Ar-142) |
| (R-1) | (Ar-123) | (Ar-144) |
| (R-1) | (Ar-123) | (Ar-164) |
| (R-1) | (Ar-123) | (Ar-165) |
| (R-1) | (Ar-142) | (Ar-1) |
| (R-1) | (Ar-142) | (Ar-2) |
| (R-1) | (Ar-142) | (Ar-3) |
| (R-1) | (Ar-142) | (Ar-4) |
| (R-1) | (Ar-142) | (Ar-56) |
| (R-1) | (Ar-142) | (Ar-57) |
| (R-1) | (Ar-142) | (Ar-60) |
| (R-1) | (Ar-142) | (Ar-62) |
| (R-1) | (Ar-142) | (Ar-71) |
| (R-1) | (Ar-142) | (Ar-82) |
| (R-1) | (Ar-142) | (Ar-96) |
| (R-1) | (Ar-142) | (Ar-104) |
| (R-1) | (Ar-142) | (Ar-121) |
| (R-1) | (Ar-142) | (Ar-123) |
| (R-1) | (Ar-142) | (Ar-142) |
| (R-1) | (Ar-142) | (Ar-144) |
| (R-1) | (Ar-142) | (Ar-164) |
| (R-1) | (Ar-142) | (Ar-165) |
| (R-2) | (Ar-2) | (Ar-1) |
| (R-2) | (Ar-2) | (Ar-2) |
| (R-2) | (Ar-2) | (Ar-3) |
| (R-2) | (Ar-2) | (Ar-4) |
| (R-2) | (Ar-2) | (Ar-56) |
| (R-2) | (Ar-2) | (Ar-57) |
| (R-2) | (Ar-2) | (Ar-60) |
| (R-2) | (Ar-2) | (Ar-62) |
| (R-2) | (Ar-2) | (Ar-71) |
| (R-2) | (Ar-2) | (Ar-82) |
| (R-2) | (Ar-2) | (Ar-96) |
| (R-2) | (Ar-2) | (Ar-104) |
| (R-2) | (Ar-2) | (Ar-121) |
| (R-2) | (Ar-2) | (Ar-123) |
| (R-2) | (Ar-2) | (Ar-142) |
| (R-2) | (Ar-2) | (Ar-144) |
| (R-2) | (Ar-2) | (Ar-164) |
| (R-2) | (Ar-2) | (Ar-165) |
| (R-2) | (Ar-3) | (Ar-1) |
| (R-2) | (Ar-3) | (Ar-2) |
| (R-2) | (Ar-3) | (Ar-3) |
| (R-2) | (Ar-3) | (Ar-4) |
| (R-2) | (Ar-3) | (Ar-56) |
| (R-2) | (Ar-3) | (Ar-57) |
| (R-2) | (Ar-3) | (Ar-60) |
| (R-2) | (Ar-3) | (Ar-62) |
| (R-2) | (Ar-3) | (Ar-71) |
| (R-2) | (Ar-3) | (Ar-82) |
| (R-2) | (Ar-3) | (Ar-96) |
| (R-2) | (Ar-3) | (Ar-104) |
| (R-2) | (Ar-3) | (Ar-121) |
| (R-2) | (Ar-3) | (Ar-123) |
| (R-2) | (Ar-3) | (Ar-142) |
| (R-2) | (Ar-3) | (Ar-144) |
| (R-2) | (Ar-3) | (Ar-164) |
| (R-2) | (Ar-3) | (Ar-165) |
| (R-2) | (Ar-4) | (Ar-1) |
| (R-2) | (Ar-4) | (Ar-2) |
| (R-2) | (Ar-4) | (Ar-3) |
| (R-2) | (Ar-4) | (Ar-4) |
| (R-2) | (Ar-4) | (Ar-56) |
| (R-2) | (Ar-4) | (Ar-57) |
| (R-2) | (Ar-4) | (Ar-60) |
| (R-2) | (Ar-4) | (Ar-62) |
| (R-2) | (Ar-4) | (Ar-71) |
| (R-2) | (Ar-4) | (Ar-82) |
| (R-2) | (Ar-4) | (Ar-96) |
| (R-2) | (Ar-4) | (Ar-104) |
| (R-2) | (Ar-4) | (Ar-121) |
| (R-2) | (Ar-4) | (Ar-123) |
| (R-2) | (Ar-4) | (Ar-142) |
| (R-2) | (Ar-4) | (Ar-144) |
| (R-2) | (Ar-4) | (Ar-164) |
| (R-2) | (Ar-4) | (Ar-165) |
| (R-2) | (Ar-121) | (Ar-1) |
| (R-2) | (Ar-121) | (Ar-2) |
| (R-2) | (Ar-121) | (Ar-3) |
| (R-2) | (Ar-121) | (Ar-4) |
| (R-2) | (Ar-121) | (Ar-56) |
| (R-2) | (Ar-121) | (Ar-57) |
| (R-2) | (Ar-121) | (Ar-60) |
| (R-2) | (Ar-121) | (Ar-62) |
| (R-2) | (Ar-121) | (Ar-71) |
| (R-2) | (Ar-121) | (Ar-82) |
| (R-2) | (Ar-121) | (Ar-96) |
| (R-2) | (Ar-121) | (Ar-104) |
| (R-2) | (Ar-121) | (Ar-121) |
| (R-2) | (Ar-121) | (Ar-123) |
| (R-2) | (Ar-121) | (Ar-142) |
| (R-2) | (Ar-121) | (Ar-144) |
| (R-2) | (Ar-121) | (Ar-164) |
| (R-2) | (Ar-121) | (Ar-165) |
| (R-2) | (Ar-123) | (Ar-1) |
| (R-2) | (Ar-123) | (Ar-2) |
| (R-2) | (Ar-123) | (Ar-3) |
| (R-2) | (Ar-123) | (Ar-4) |
| (R-2) | (Ar-123) | (Ar-56) |
| (R-2) | (Ar-123) | (Ar-57) |
| (R-2) | (Ar-123) | (Ar-60) |
| (R-2) | (Ar-123) | (Ar-62) |
| (R-2) | (Ar-123) | (Ar-71) |
| (R-2) | (Ar-123) | (Ar-82) |
| (R-2) | (Ar-123) | (Ar-96) |
| (R-2) | (Ar-123) | (Ar-104) |
| (R-2) | (Ar-123) | (Ar-121) |
| (R-2) | (Ar-123) | (Ar-123) |
| (R-2) | (Ar-123) | (Ar-142) |
| (R-2) | (Ar-123) | (Ar-144) |
| (R-2) | (Ar-123) | (Ar-164) |
| (R-2) | (Ar-123) | (Ar-165) |
| (R-2) | (Ar-142) | (Ar-1) |
| (R-2) | (Ar-142) | (Ar-2) |
| (R-2) | (Ar-142) | (Ar-3) |
| (R-2) | (Ar-142) | (Ar-4) |
| (R-2) | (Ar-142) | (Ar-56) |
| (R-2) | (Ar-142) | (Ar-57) |
| (R-2) | (Ar-142) | (Ar-60) |
| (R-2) | (Ar-142) | (Ar-62) |
| (R-2) | (Ar-142) | (Ar-71) |
| (R-2) | (Ar-142) | (Ar-82) |
| (R-2) | (Ar-142) | (Ar-96) |
| (R-2) | (Ar-142) | (Ar-104) |
| (R-2) | (Ar-142) | (Ar-121) |
| (R-2) | (Ar-142) | (Ar-123) |
| (R-2) | (Ar-142) | (Ar-142) |
| (R-2) | (Ar-142) | (Ar-144) |
| (R-2) | (Ar-142) | (Ar-164) |
| (R-2) | (Ar-142) | (Ar-165) |
| (R-3) | (Ar-2) | (Ar-1) |
| (R-3) | (Ar-2) | (Ar-2) |
| (R-3) | (Ar-2) | (Ar-3) |
| (R-3) | (Ar-2) | (Ar-4) |
| (R-3) | (Ar-2) | (Ar-56) |
| (R-3) | (Ar-2) | (Ar-57) |
| (R-3) | (Ar-2) | (Ar-60) |
| (R-3) | (Ar-2) | (Ar-62) |
| (R-3) | (Ar-2) | (Ar-71) |
| (R-3) | (Ar-2) | (Ar-82) |
| (R-3) | (Ar-2) | (Ar-96) |
| (R-3) | (Ar-2) | (Ar-104) |
| (R-3) | (Ar-2) | (Ar-121) |
| (R-3) | (Ar-2) | (Ar-123) |
| (R-3) | (Ar-2) | (Ar-142) |
| (R-3) | (Ar-2) | (Ar-144) |
| (R-3) | (Ar-2) | (Ar-164) |
| (R-3) | (Ar-2) | (Ar-165) |
| (R-3) | (Ar-3) | (Ar-1) |
| (R-3) | (Ar-3) | (Ar-2) |
| (R-3) | (Ar-3) | (Ar-3) |
| (R-3) | (Ar-3) | (Ar-4) |
| (R-3) | (Ar-3) | (Ar-56) |
| (R-3) | (Ar-3) | (Ar-57) |
| (R-3) | (Ar-3) | (Ar-60) |
| (R-3) | (Ar-3) | (Ar-62) |
| (R-3) | (Ar-3) | (Ar-71) |
| (R-3) | (Ar-3) | (Ar-82) |
| (R-3) | (Ar-3) | (Ar-96) |
| (R-3) | (Ar-3) | (Ar-104) |
| (R-3) | (Ar-3) | (Ar-121) |
| (R-3) | (Ar-3) | (Ar-123) |
| (R-3) | (Ar-3) | (Ar-142) |
| (R-3) | (Ar-3) | (Ar-144) |
| (R-3) | (Ar-3) | (Ar-164) |
| (R-3) | (Ar-3) | (Ar-165) |
| (R-3) | (Ar-4) | (Ar-1) |
| (R-3) | (Ar-4) | (Ar-2) |
| (R-3) | (Ar-4) | (Ar-3) |
| (R-3) | (Ar-4) | (Ar-4) |
| (R-3) | (Ar-4) | (Ar-56) |
| (R-3) | (Ar-4) | (Ar-57) |
| (R-3) | (Ar-4) | (Ar-60) |
| (R-3) | (Ar-4) | (Ar-62) |
| (R-3) | (Ar-4) | (Ar-71) |
| (R-3) | (Ar-4) | (Ar-82) |
| (R-3) | (Ar-4) | (Ar-96) |
| (R-3) | (Ar-4) | (Ar-104) |
| (R-3) | (Ar-4) | (Ar-121) |
| (R-3) | (Ar-4) | (Ar-123) |
| (R-3) | (Ar-4) | (Ar-142) |
| (R-3) | (Ar-4) | (Ar-144) |
| (R-3) | (Ar-4) | (Ar-164) |
| (R-3) | (Ar-4) | (Ar-165) |
| (R-3) | (Ar-121) | (Ar-1) |
| (R-3) | (Ar-121) | (Ar-2) |
| (R-3) | (Ar-121) | (Ar-3) |
| (R-3) | (Ar-121) | (Ar-4) |
| (R-3) | (Ar-121) | (Ar-56) |
| (R-3) | (Ar-121) | (Ar-57) |
| (R-3) | (Ar-121) | (Ar-60) |
| (R-3) | (Ar-121) | (Ar-62) |
| (R-3) | (Ar-121) | (Ar-71) |
| (R-3) | (Ar-121) | (Ar-82) |
| (R-3) | (Ar-121) | (Ar-96) |
| (R-3) | (Ar-121) | (Ar-104) |
| (R-3) | (Ar-121) | (Ar-121) |
| (R-3) | (Ar-121) | (Ar-123) |
| (R-3) | (Ar-121) | (Ar-142) |
| (R-3) | (Ar-121) | (Ar-144) |
| (R-3) | (Ar-121) | (Ar-164) |
| (R-3) | (Ar-121) | (Ar-165) |
| (R-3) | (Ar-123) | (Ar-1) |
| (R-3) | (Ar-123) | (Ar-2) |
| (R-3) | (Ar-123) | (Ar-3) |
| (R-3) | (Ar-123) | (Ar-4) |
| (R-3) | (Ar-123) | (Ar-56) |
| (R-3) | (Ar-123) | (Ar-57) |
| (R-3) | (Ar-123) | (Ar-60) |
| (R-3) | (Ar-123) | (Ar-62) |
| (R-3) | (Ar-123) | (Ar-71) |
| (R-3) | (Ar-123) | (Ar-82) |
| (R-3) | (Ar-123) | (Ar-96) |
| (R-3) | (Ar-123) | (Ar-104) |
| (R-3) | (Ar-123) | (Ar-121) |
| (R-3) | (Ar-123) | (Ar-123) |
| (R-3) | (Ar-123) | (Ar-142) |
| (R-3) | (Ar-123) | (Ar-144) |
| (R-3) | (Ar-123) | (Ar-164) |
| (R-3) | (Ar-123) | (Ar-165) |
| (R-3) | (Ar-142) | (Ar-1) |
| (R-3) | (Ar-142) | (Ar-2) |
| (R-3) | (Ar-142) | (Ar-3) |
| (R-3) | (Ar-142) | (Ar-4) |
| (R-3) | (Ar-142) | (Ar-56) |
| (R-3) | (Ar-142) | (Ar-57) |
| (R-3) | (Ar-142) | (Ar-60) |
| (R-3) | (Ar-142) | (Ar-62) |
| (R-3) | (Ar-142) | (Ar-71) |
| (R-3) | (Ar-142) | (Ar-82) |
| (R-3) | (Ar-142) | (Ar-96) |
| (R-3) | (Ar-142) | (Ar-104) |
| (R-3) | (Ar-142) | (Ar-121) |
| (R-3) | (Ar-142) | (Ar-123) |
| (R-3) | (Ar-142) | (Ar-142) |
| (R-3) | (Ar-142) | (Ar-144) |
| (R-3) | (Ar-142) | (Ar-164) |
| (R-3) | (Ar-142) | (Ar-165) |
| (R-4) | (Ar-2) | (Ar-1) |
| (R-4) | (Ar-2) | (Ar-2) |
| (R-4) | (Ar-2) | (Ar-3) |
| (R-4) | (Ar-2) | (Ar-4) |
| (R-4) | (Ar-2) | (Ar-56) |
| (R-4) | (Ar-2) | (Ar-57) |
| (R-4) | (Ar-2) | (Ar-60) |
| (R-4) | (Ar-2) | (Ar-62) |
| (R-4) | (Ar-2) | (Ar-71) |
| (R-4) | (Ar-2) | (Ar-82) |
| (R-4) | (Ar-2) | (Ar-96) |
| (R-4) | (Ar-2) | (Ar-104) |
| (R-4) | (Ar-2) | (Ar-121) |
| (R-4) | (Ar-2) | (Ar-123) |
| (R-4) | (Ar-2) | (Ar-142) |
| (R-4) | (Ar-2) | (Ar-144) |
| (R-4) | (Ar-2) | (Ar-164) |
| (R-4) | (Ar-2) | (Ar-165) |
| (R-4) | (Ar-3) | (Ar-1) |
| (R-4) | (Ar-3) | (Ar-2) |
| (R-4) | (Ar-3) | (Ar-3) |
| (R-4) | (Ar-3) | (Ar-4) |
| (R-4) | (Ar-3) | (Ar-56) |
| (R-4) | (Ar-3) | (Ar-57) |
| (R-4) | (Ar-3) | (Ar-60) |
| (R-4) | (Ar-3) | (Ar-62) |
| (R-4) | (Ar-3) | (Ar-71) |
| (R-4) | (Ar-3) | (Ar-82) |
| (R-4) | (Ar-3) | (Ar-96) |
| (R-4) | (Ar-3) | (Ar-104) |
| (R-4) | (Ar-3) | (Ar-121) |
| (R-4) | (Ar-3) | (Ar-123) |
| (R-4) | (Ar-3) | (Ar-142) |
| (R-4) | (Ar-3) | (Ar-144) |
| (R-4) | (Ar-3) | (Ar-164) |
| (R-4) | (Ar-3) | (Ar-165) |
| (R-4) | (Ar-4) | (Ar-1) |
| (R-4) | (Ar-4) | (Ar-2) |
| (R-4) | (Ar-4) | (Ar-3) |
| (R-4) | (Ar-4) | (Ar-4) |
| (R-4) | (Ar-4) | (Ar-56) |
| (R-4) | (Ar-4) | (Ar-57) |
| (R-4) | (Ar-4) | (Ar-60) |
| (R-4) | (Ar-4) | (Ar-62) |
| (R-4) | (Ar-4) | (Ar-71) |
| (R-4) | (Ar-4) | (Ar-82) |
| (R-4) | (Ar-4) | (Ar-96) |
| (R-4) | (Ar-4) | (Ar-104) |
| (R-4) | (Ar-4) | (Ar-121) |
| (R-4) | (Ar-4) | (Ar-123) |
| (R-4) | (Ar-4) | (Ar-142) |
| (R-4) | (Ar-4) | (Ar-144) |
| (R-4) | (Ar-4) | (Ar-164) |
| (R-4) | (Ar-4) | (Ar-165) |
| (R-4) | (Ar-121) | (Ar-1) |
| (R-4) | (Ar-121) | (Ar-2) |
| (R-4) | (Ar-121) | (Ar-3) |
| (R-4) | (Ar-121) | (Ar-4) |
| (R-4) | (Ar-121) | (Ar-56) |
| (R-4) | (Ar-121) | (Ar-57) |
| (R-4) | (Ar-121) | (Ar-60) |
| (R-4) | (Ar-121) | (Ar-62) |
| (R-4) | (Ar-121) | (Ar-71) |
| (R-4) | (Ar-121) | (Ar-82) |
| (R-4) | (Ar-121) | (Ar-96) |
| (R-4) | (Ar-121) | (Ar-104) |
| (R-4) | (Ar-121) | (Ar-121) |
| (R-4) | (Ar-121) | (Ar-123) |
| (R-4) | (Ar-121) | (Ar-142) |
| (R-4) | (Ar-121) | (Ar-144) |
| (R-4) | (Ar-121) | (Ar-164) |
| (R-4) | (Ar-121) | (Ar-165) |
| (R-4) | (Ar-123) | (Ar-1) |
| (R-4) | (Ar-123) | (Ar-2) |
| (R-4) | (Ar-123) | (Ar-3) |
| (R-4) | (Ar-123) | (Ar-4) |
| (R-4) | (Ar-123) | (Ar-56) |
| (R-4) | (Ar-123) | (Ar-57) |
| (R-4) | (Ar-123) | (Ar-60) |
| (R-4) | (Ar-123) | (Ar-62) |
| (R-4) | (Ar-123) | (Ar-71) |
| (R-4) | (Ar-123) | (Ar-82) |
| (R-4) | (Ar-123) | (Ar-96) |
| (R-4) | (Ar-123) | (Ar-104) |
| (R-4) | (Ar-123) | (Ar-121) |
| (R-4) | (Ar-123) | (Ar-123) |
| (R-4) | (Ar-123) | (Ar-142) |
| (R-4) | (Ar-123) | (Ar-144) |
| (R-4) | (Ar-123) | (Ar-164) |
| (R-4) | (Ar-123) | (Ar-165) |
| (R-4) | (Ar-142) | (Ar-1) |
| (R-4) | (Ar-142) | (Ar-2) |
| (R-4) | (Ar-142) | (Ar-3) |
| (R-4) | (Ar-142) | (Ar-4) |
| (R-4) | (Ar-142) | (Ar-56) |
| (R-4) | (Ar-142) | (Ar-57) |
| (R-4) | (Ar-142) | (Ar-60) |
| (R-4) | (Ar-142) | (Ar-62) |
| (R-4) | (Ar-142) | (Ar-71) |
| (R-4) | (Ar-142) | (Ar-82) |
| (R-4) | (Ar-142) | (Ar-96) |
| (R-4) | (Ar-142) | (Ar-104) |
| (R-4) | (Ar-142) | (Ar-121) |
| (R-4) | (Ar-142) | (Ar-123) |
| (R-4) | (Ar-142) | (Ar-142) |
| (R-4) | (Ar-142) | (Ar-144) |
| (R-4) | (Ar-142) | (Ar-164) |
| (R-4) | (Ar-142) | (Ar-165) |

Examples of suitable compounds according to the invention are the compounds shown in the following table, where compounds which are not according to the claims are marked with #:

| | | |
|---|---|---|
| 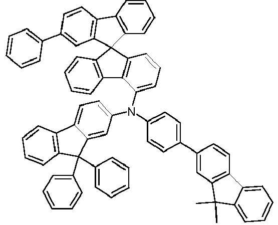 | 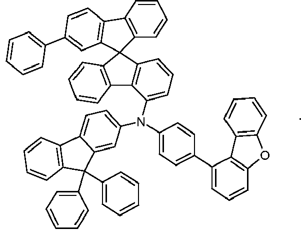 | 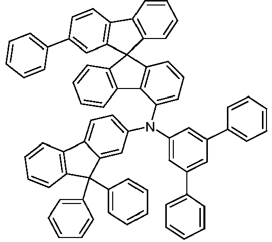 |
| 1 | 2 | 3 |
| 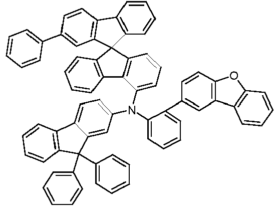 | 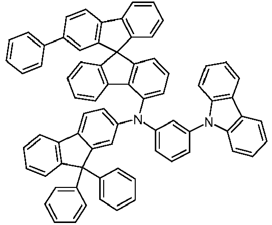 | 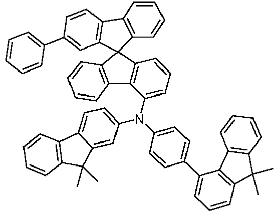 |
| 4 | 5 | 6 |
| 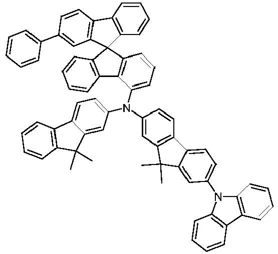 | 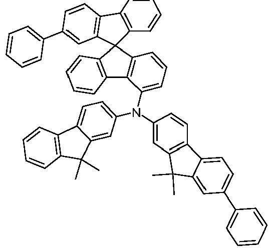 | 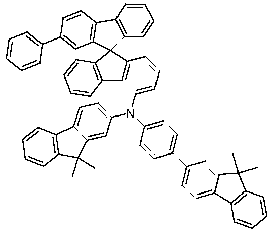 |
| 7 | 8 | 9 |
| 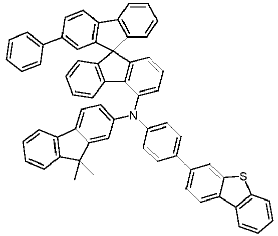 | 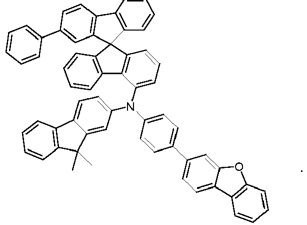 | 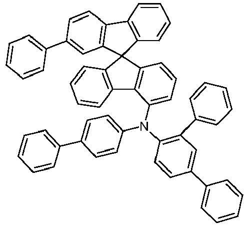 |
| 10 | 11 | 12 |
| 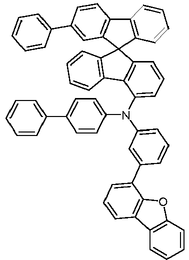 | 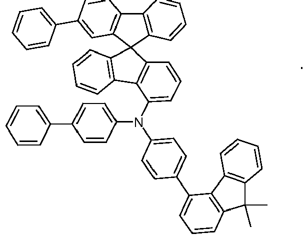 | 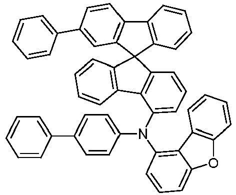 |
| 13 | 14 | 15 |
| 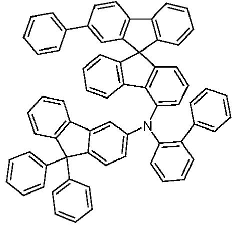 | 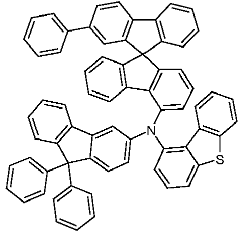 | 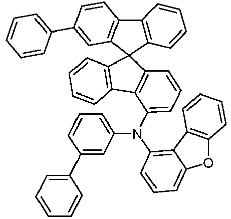 |
| 16 | 17 | 18 |
| 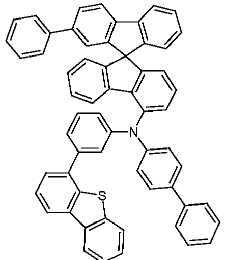 | 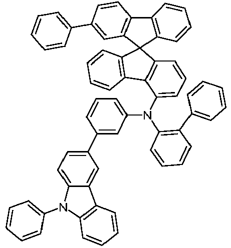 | 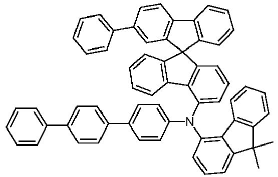 |
| 19 | 20 | 21 |
| 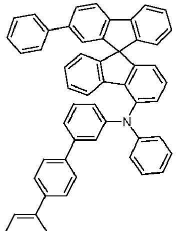 | 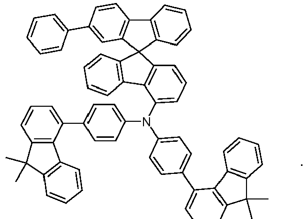 | 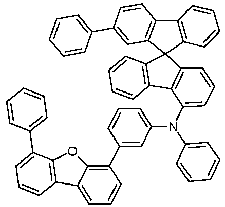 |
| 22 | 23 | 24 |
| 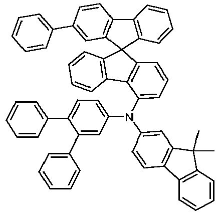 | 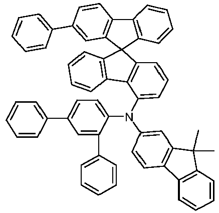 | 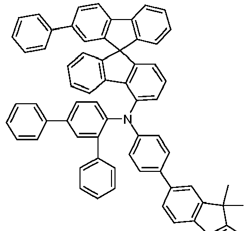 |
| 25 | 26 | 27 |
| 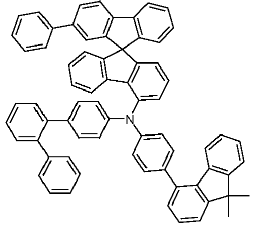 | 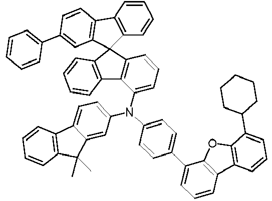 | 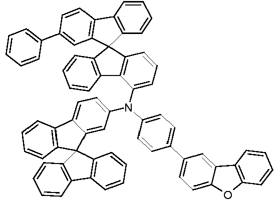 |
| 28 | 29 | 30 |
| 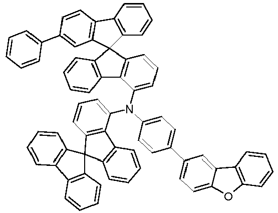 | 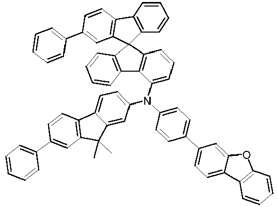 | 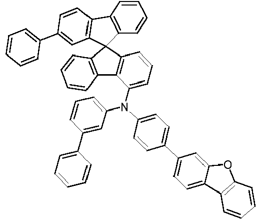 |
| 31 | 32 | 33 |
| 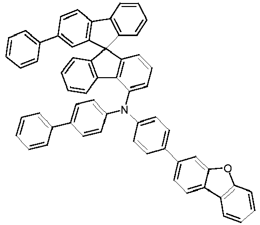 | 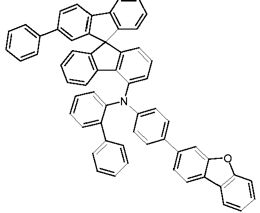 | 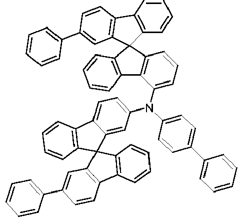 |
| 34 | 35 | 36 |
| 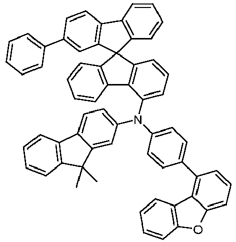 | 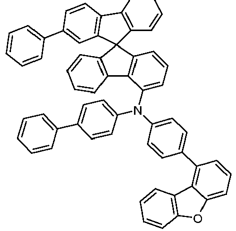 | 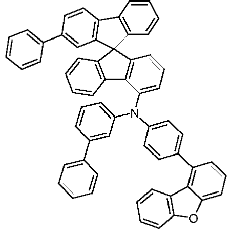 |
| 37 | 38 | 39 |
| 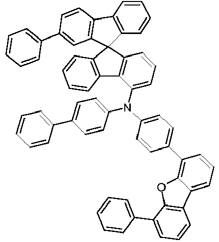 | 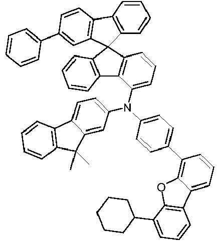 | 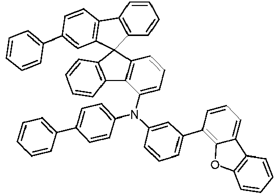 |
| 40 | 41 | 42 |
| 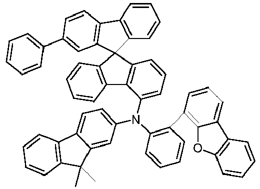 | 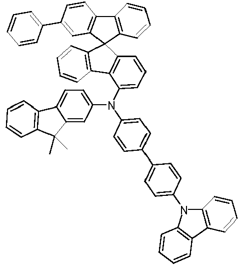 | 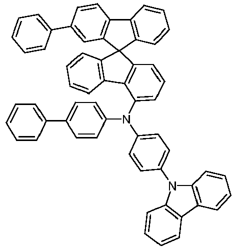 |
| 43 | 44 | 45 |
| 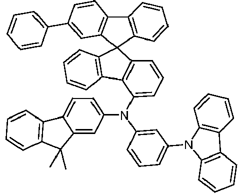 | 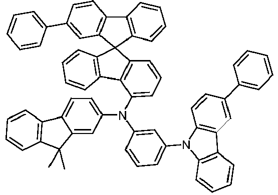 | 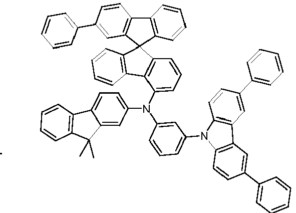 |
| 46 | 47 | 48 |
| 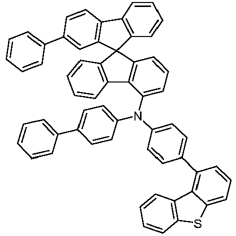 | 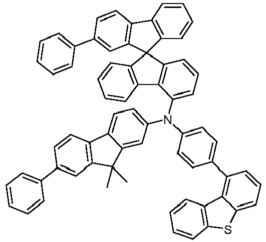 | 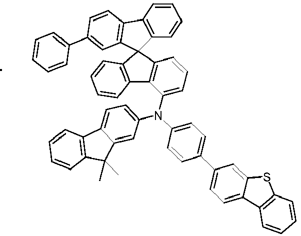 |
| 49 | 50 | 51 |
| 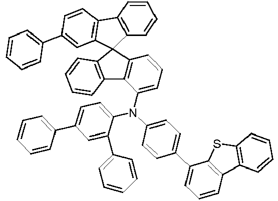 | 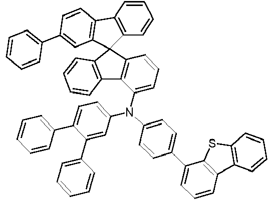 | 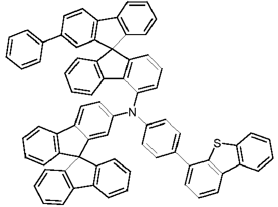 |
| 52 | 53 | 54 |
| 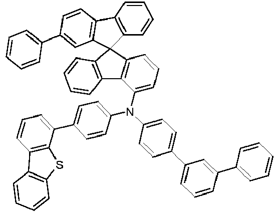 | 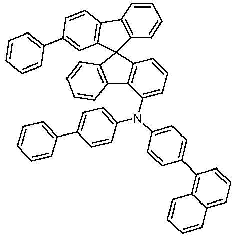 | 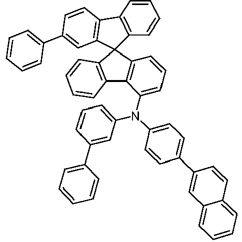 |
| 55 | 56 | 57 |
| 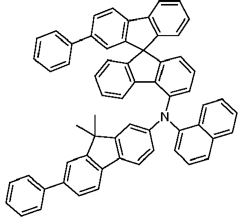 | 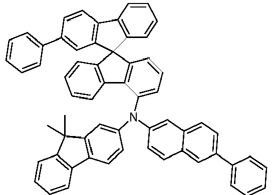 | 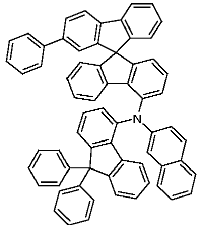 |
| 58 | 59 | 60 |
| 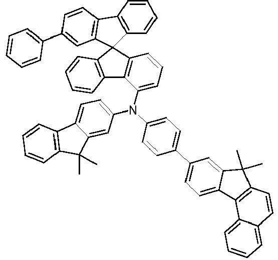 | 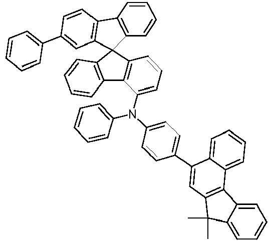 | 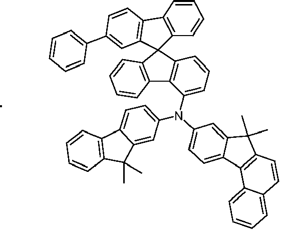 |
| 61 | 62 | 63 |
| 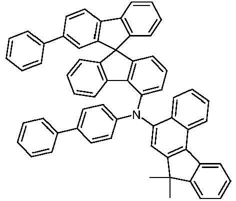 | 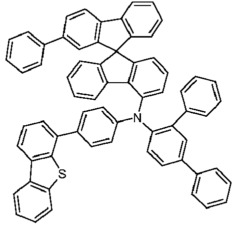 | 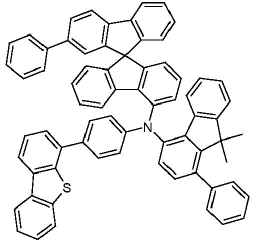 |
| 64 | 65 | 66 |
| 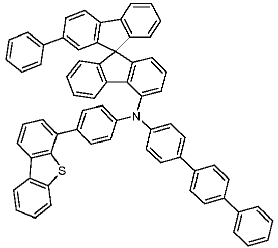 | 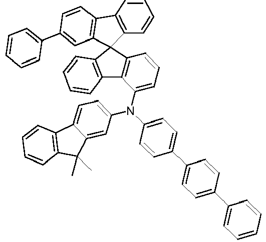 | 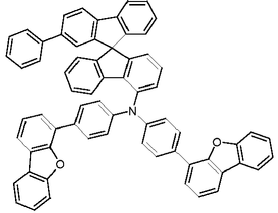 |
| 67 | 68 | 69 |
| 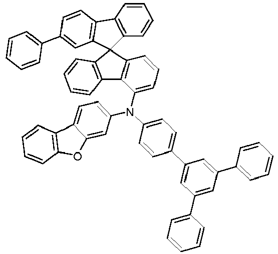 | 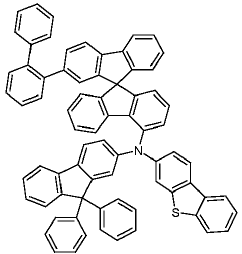 | 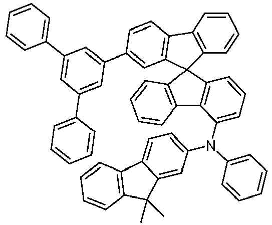 |
| 70 | 71 | 72 |
| 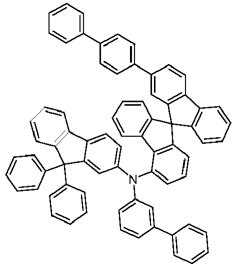 | 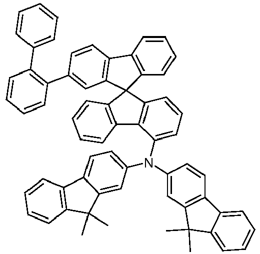 | 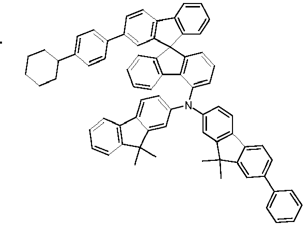 |
| 73 | 74 | 75# |
| 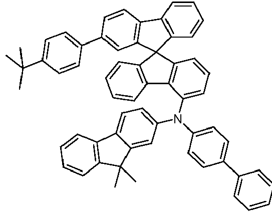 | 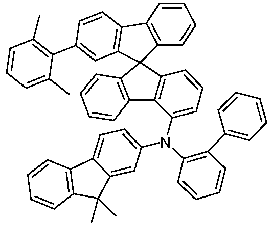 | 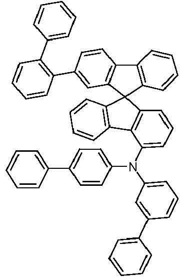 |
| 76# | 77# | 78 |
| 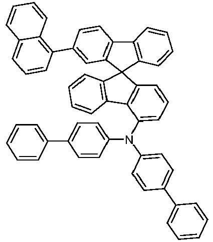 | 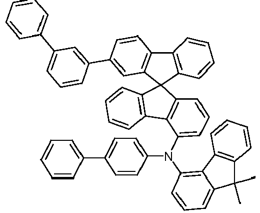 | 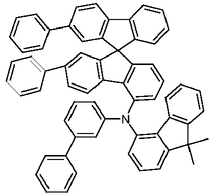 |
| 79 | 80 | 81# |
| 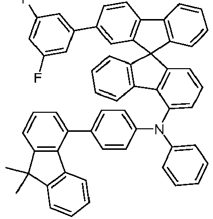 | 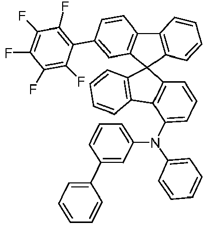 | 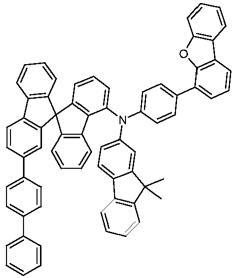 |
| 82# | 83# | 84 |
| 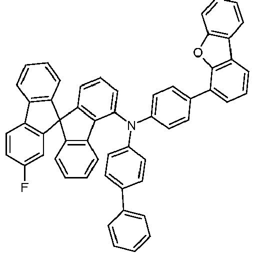 | 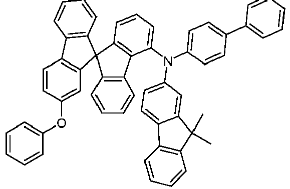 | 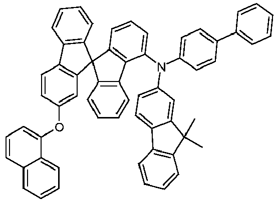 |
| 85# | 86# | 87# |
| 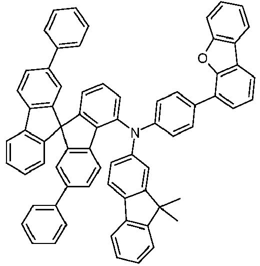 | 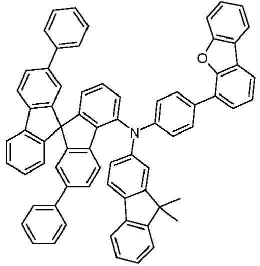 | 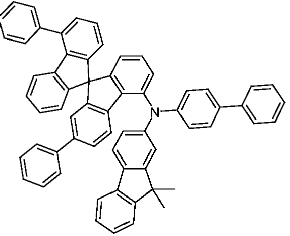 |
| 88# | 89# | 90# |
| 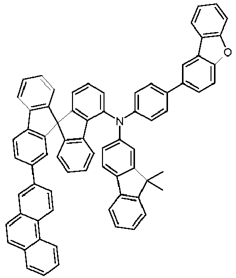 | 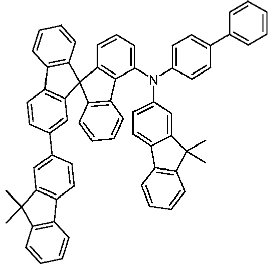 | 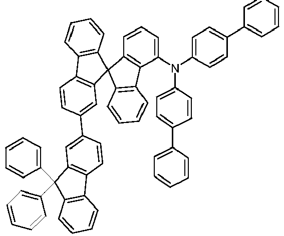 |
| 91# | 92# | 93# |
| 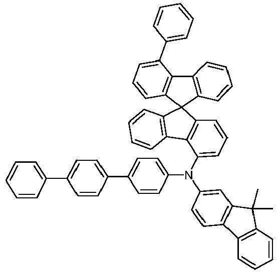 | 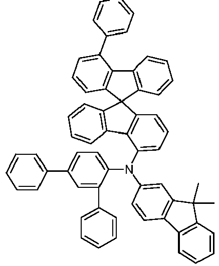 | 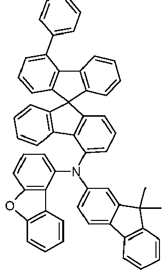 |
| 94 | 95 | 96 |
| 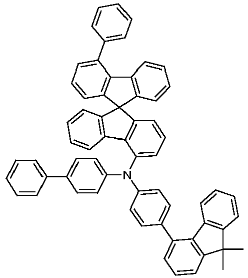 | 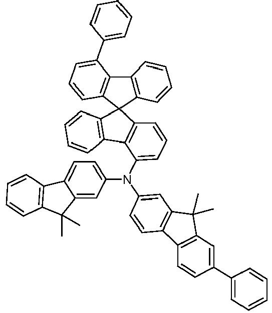 | 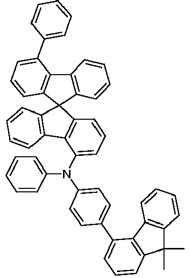 |
| 97 | 98 | 99 |
| 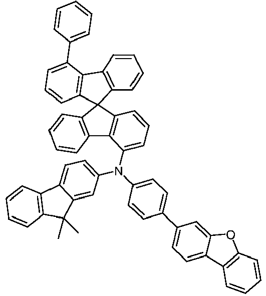 | 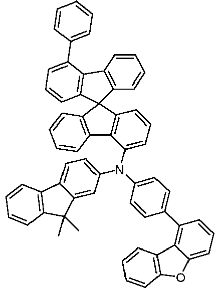 | 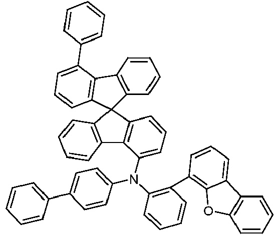 |
| 100 | 101 | 102 |
| 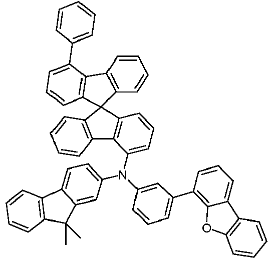 | 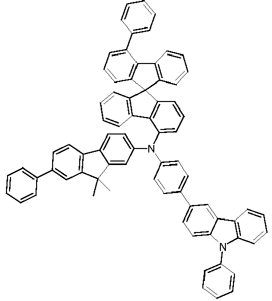 | 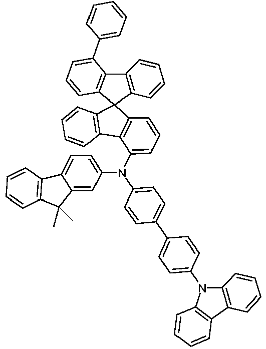 |
| 103 | 104 | 105 |
| 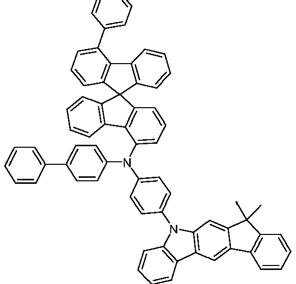 | 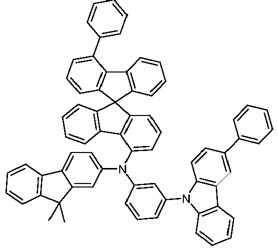 | 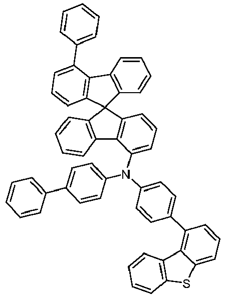 |
| 106 | 107 | 108 |
| 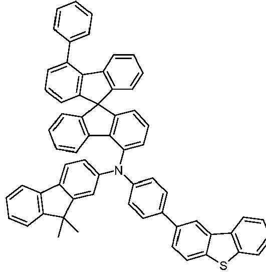 | 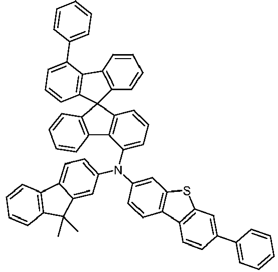 | 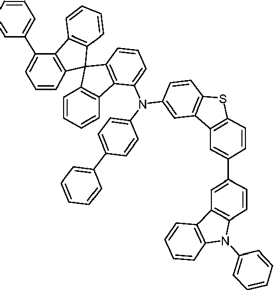 |
| 109 | 110 | 111 |
| 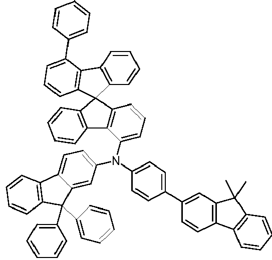 | 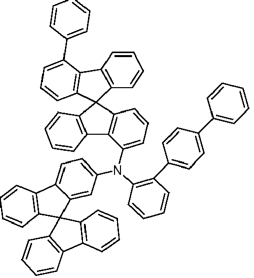 | 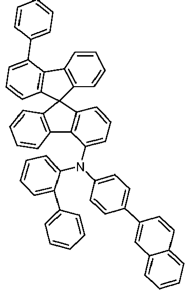 |
| 112 | 113 | 114 |
| 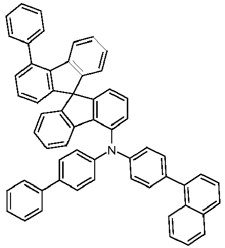 | 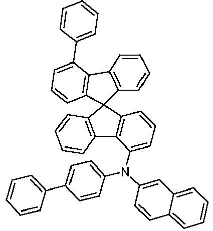 | 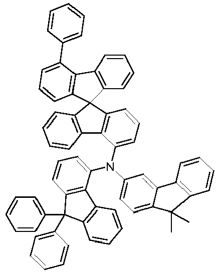 |
| 115 | 116 | 117 |
| 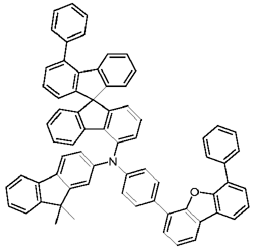 | 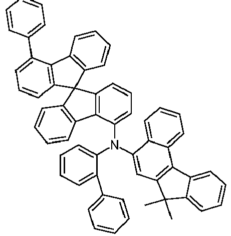 | 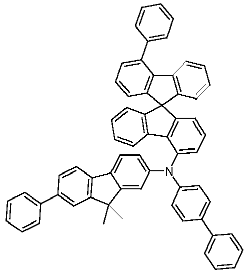 |
| 118 | 119 | 120 |
| 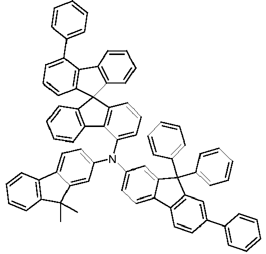 | 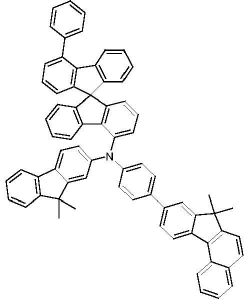 | 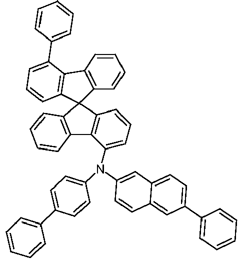 |
| 121 | 122 | 123 |
| 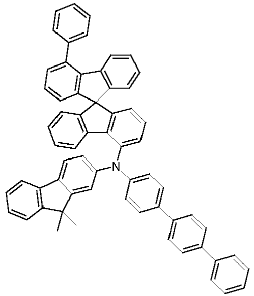 | 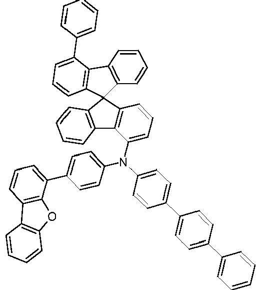 | 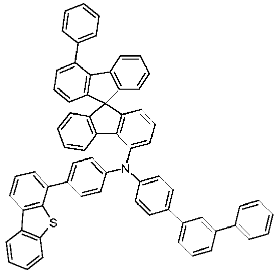 |
| 124 | 125 | 126 |
| 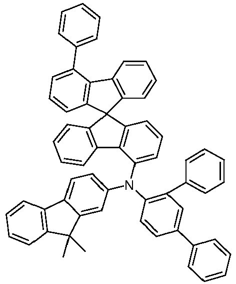 | 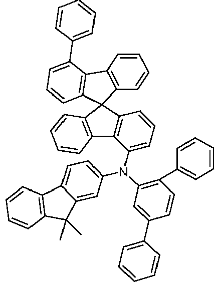 | 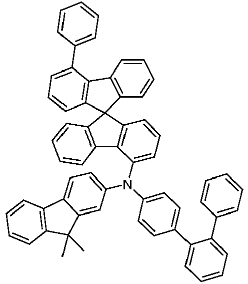 |
| 127 | 128 | 129 |
| 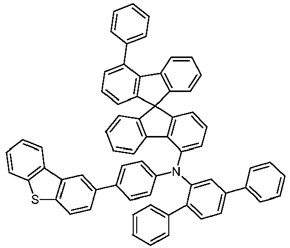 | 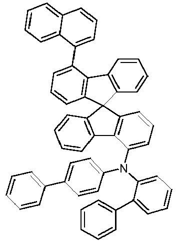 | 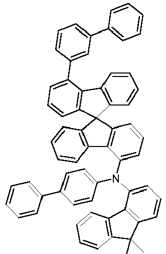 |
| 130 | 131 | 132 |
| 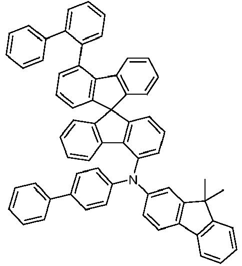 | 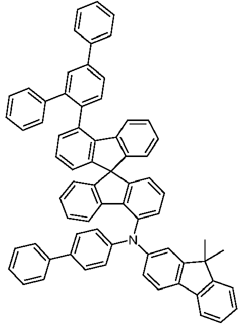 | 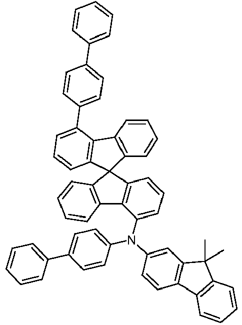 |
| 133 | 134 | 135 |
| 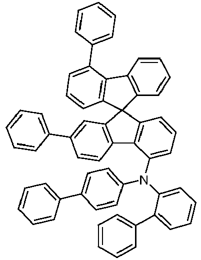 | 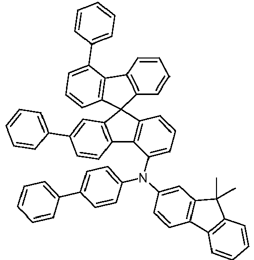 | 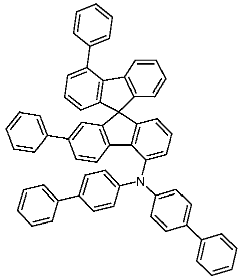 |
| 136# | 137# | 138# |
| 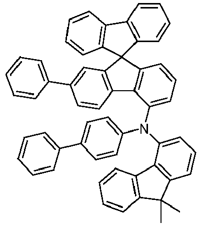 | 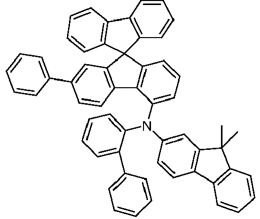 | 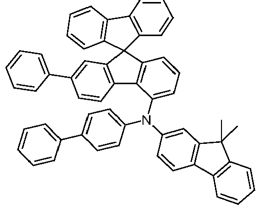 |
| 139 | 140 | 141 |
| 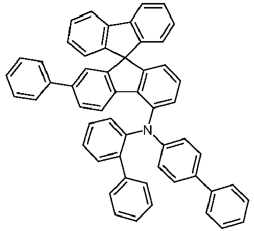 | 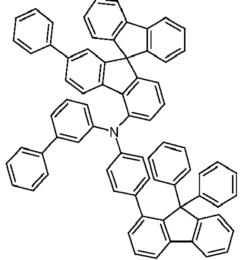 | 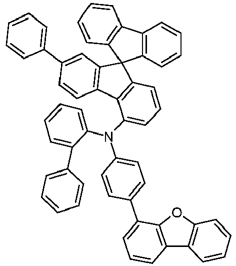 |
| 142 | 143 | 144 |
| 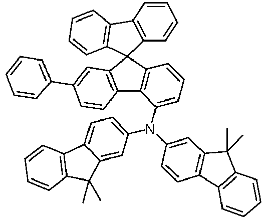 | 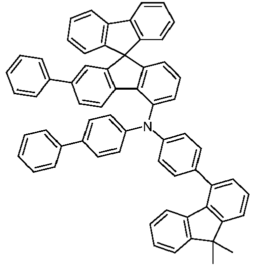 | 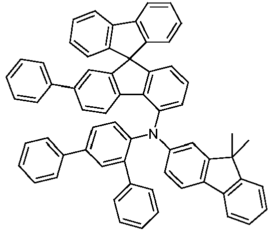 |
| 145 | 146 | 147 |
| 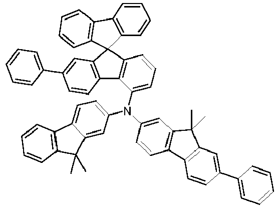 | 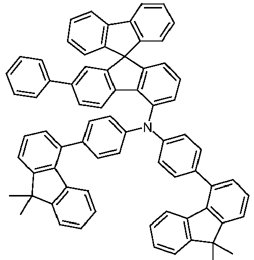 | 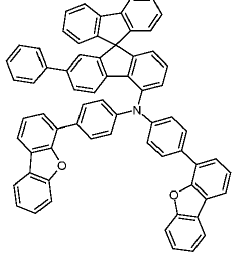 |
| 148 | 149 | 150 |
| 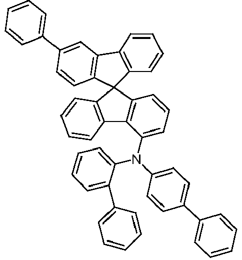 | 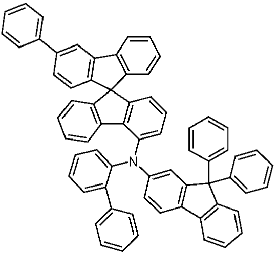 | 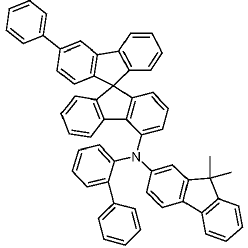 |
| 151 | 152 | 153 |
| 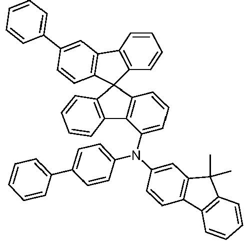 | 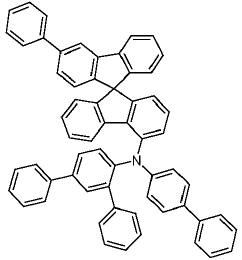 | 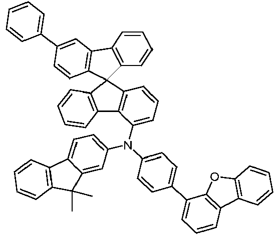 |
| 154 | 155 | 156 |
| 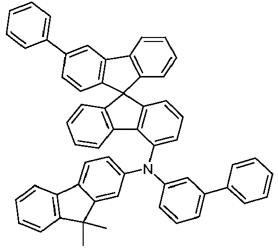 | 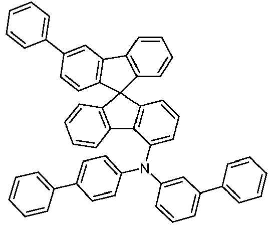 | 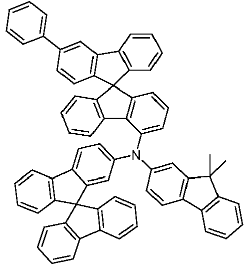 |
| 157 | 158 | 159 |
| 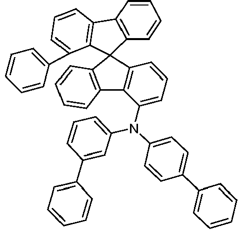 | 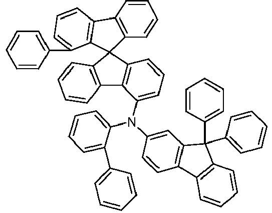 | 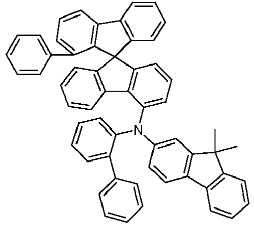 |
| 160 | 161 | 162 |
| 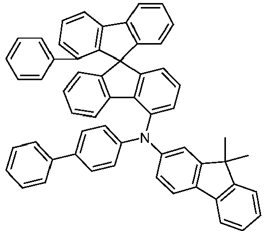 | 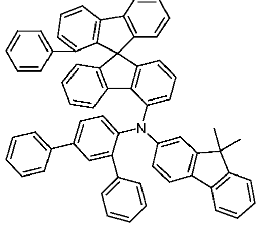 | 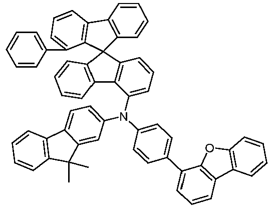 |
| 163 | 164 | 165 |
| 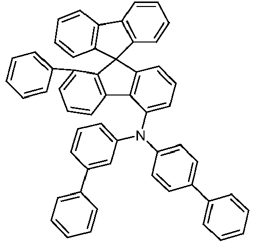 | 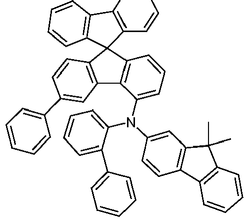 | 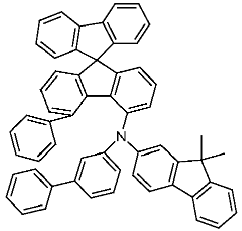 |
| 166 | 167 | 168 |
| 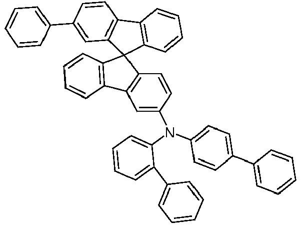 | 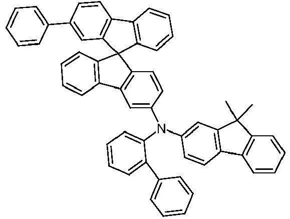 | 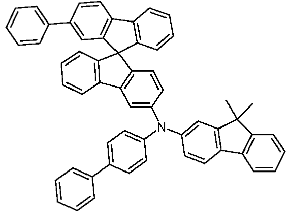 |
| 169 | 170 | 171 |
| 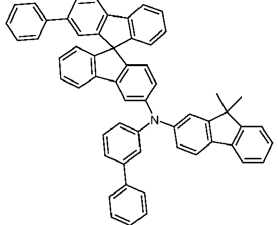 | 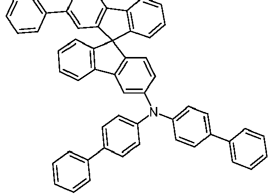 | 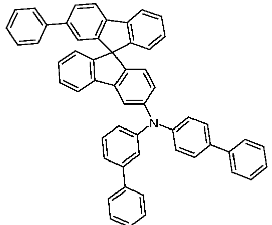 |
| 172 | 173 | 174 |
| 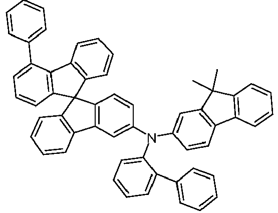 | 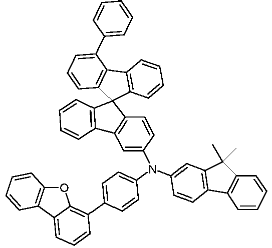 | 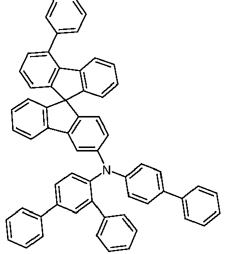 |
| 175 | 176 | 177 |
| 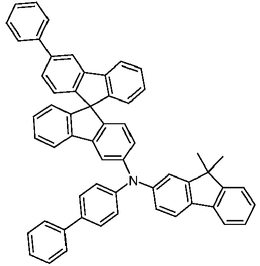 | 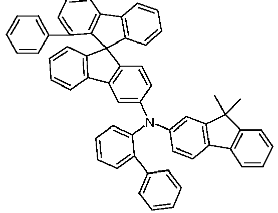 | 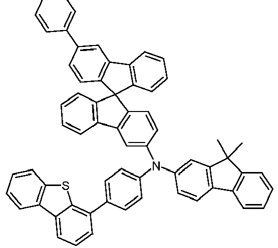 |
| 178 | 179 | 180 |
| 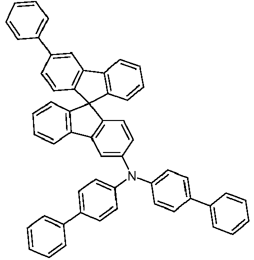 | 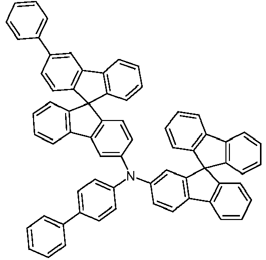 | 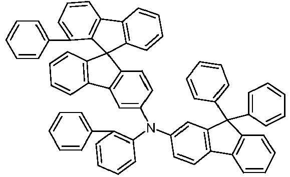 |
| 181 | 182 | 183 |
| 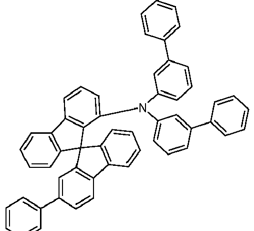 | 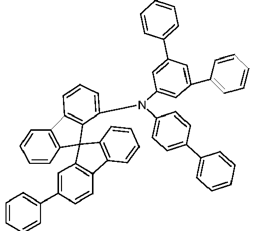 | 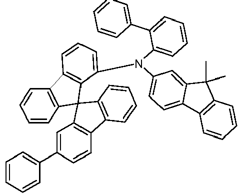 |
| 184 | 185 | 186 |
| 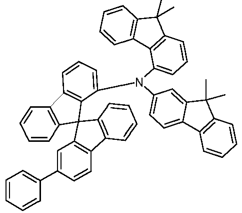 | 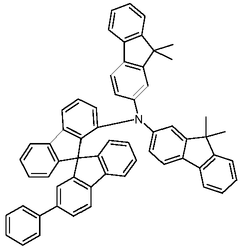 | 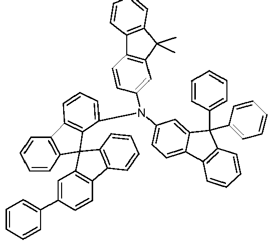 |
| 187 | 188 | 189 |
| 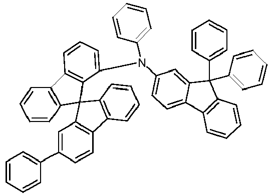 | 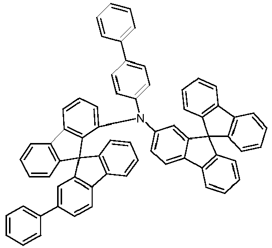 | 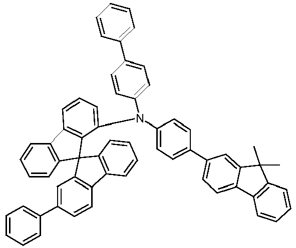 |
| 190 | 191 | 192 |
| 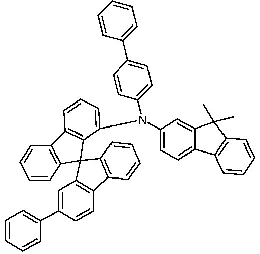 | 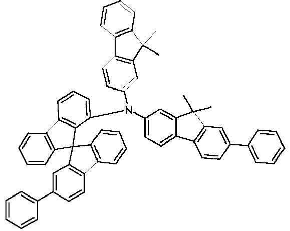 | 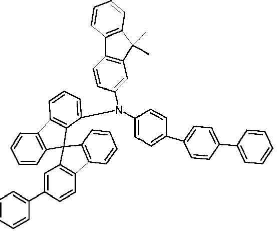 |
| 193 | 194 | 195 |
| 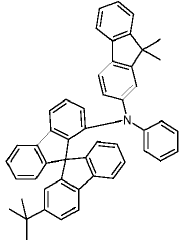 | 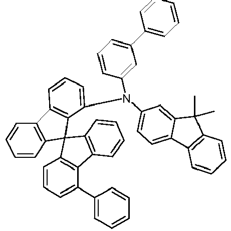 | 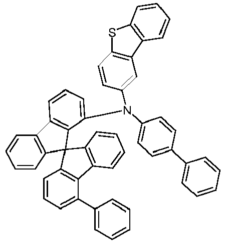 |
| 196# | 197 | 198 |
| 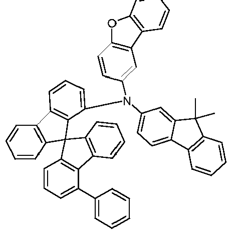 | 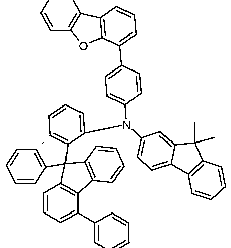 | 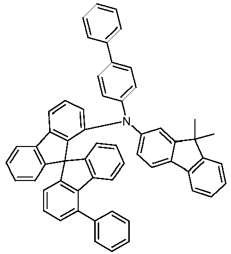 |
| 199 | 200 | 201 |
| 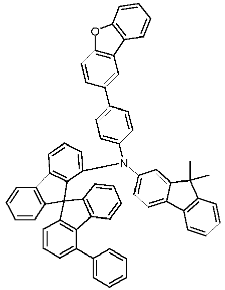 | 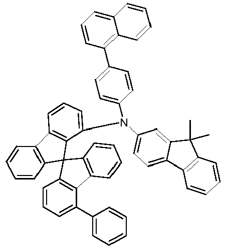 | 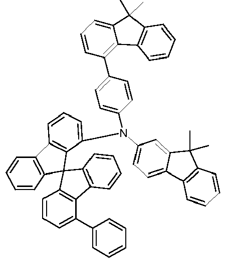 |
| 202 | 203 | 204 |
| 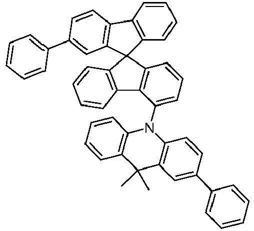 | 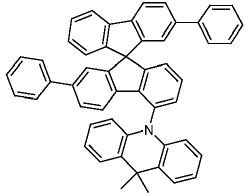 | 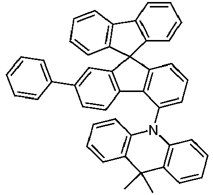 |
| 205 | 206 | 207 |
| 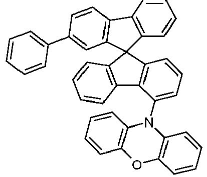 | 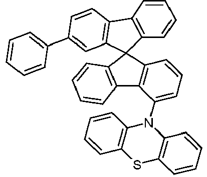 | 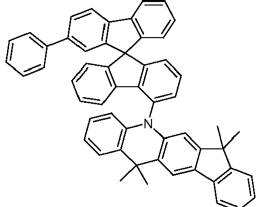 |
| 208 | 209 | 210 |
| 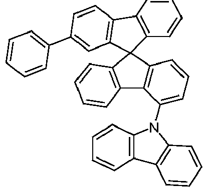 | 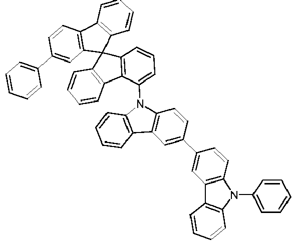 | 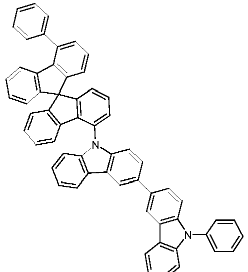 |
| 211 | 212 | 213 |
| 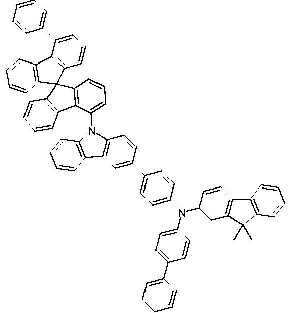 | 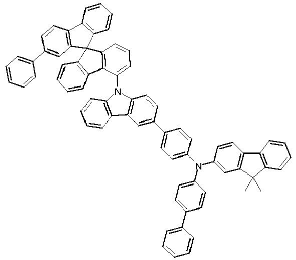 | 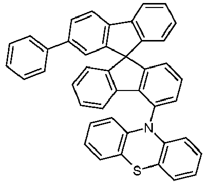 |
| 214 | 215 | 216 |
| 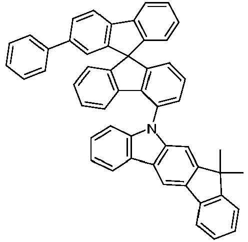 | 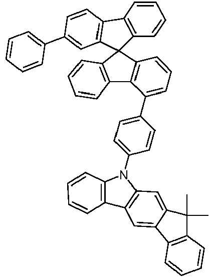 | 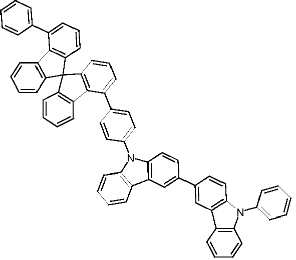 |
| 217 | 218 | 219 |
| 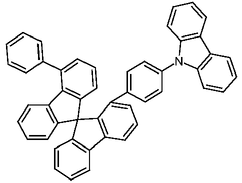 | 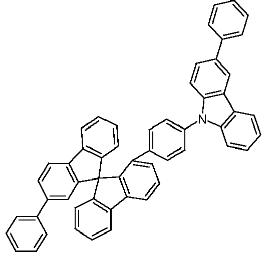 | 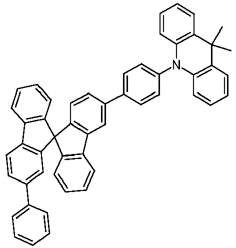 |
| 220 | 221 | 222 |
| 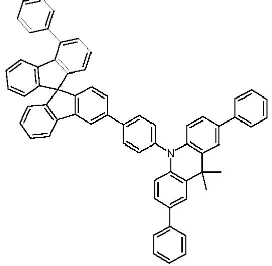 | 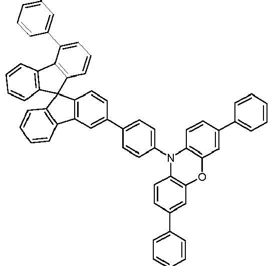 | 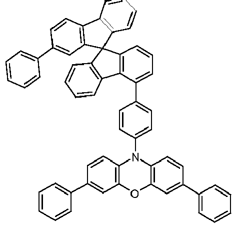 |
| 223 | 224 | 225 |
| 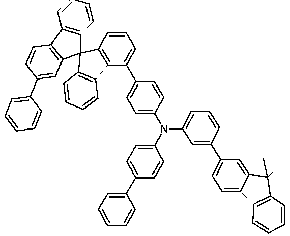 | 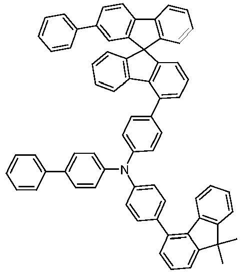 | 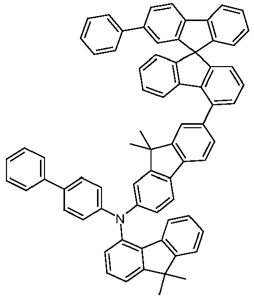 |
| 226 | 227 | 228 |
| 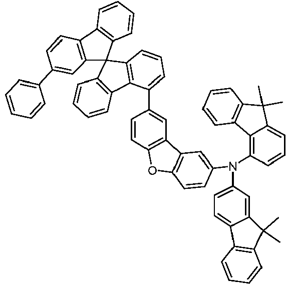 | 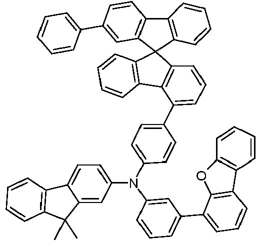 | 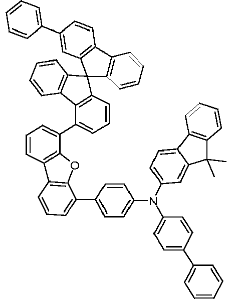 |
| 229 | 230 | 231 |
| 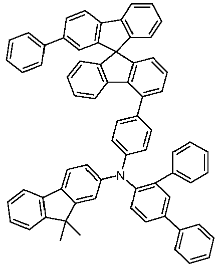 | 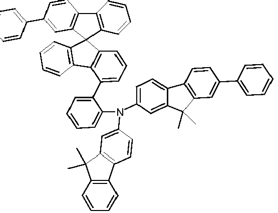 | 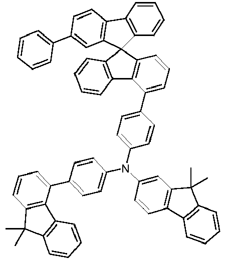 |
| 232 | 233 | 234 |
| 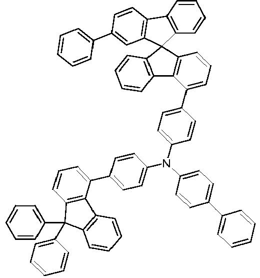 | 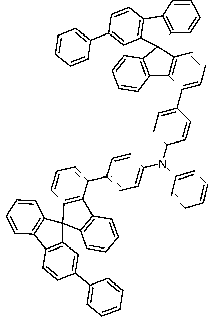 | |
| 235 | 236 | |
| 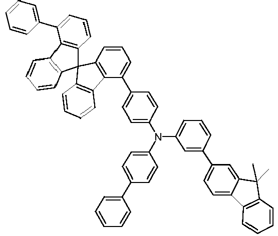 | 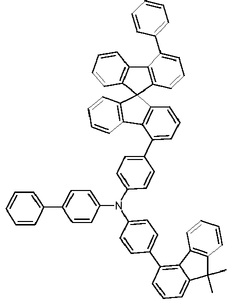 | 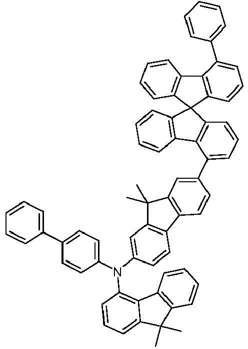 |
| 237 | 238 | 239 |
| 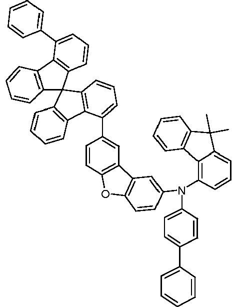 | 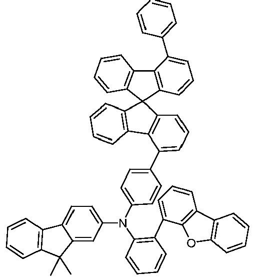 | 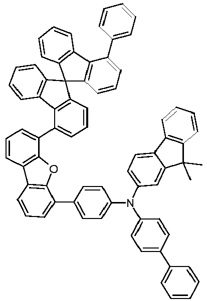 |
| 240 | 241 | 242 |
| 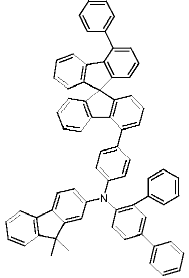 | 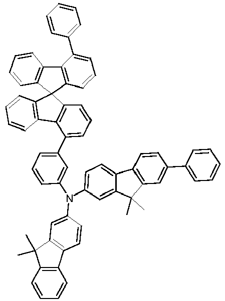 | 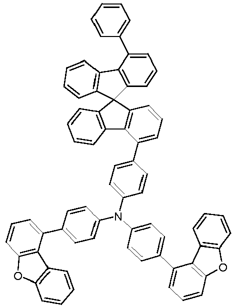 |
| 243 | 244 | 245 |

The compounds according to the invention can be prepared by synthetic steps known to the person skilled in the art, such as, for example, bromination, Ullmann arylation, Hartwig-Buchwald coupling, as depicted in Scheme 1 below. 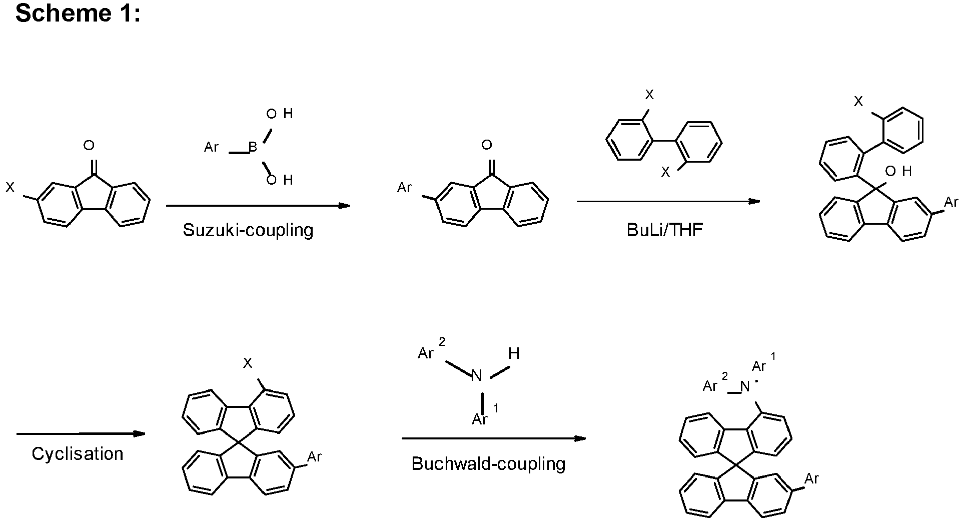
X is a halogen or another leaving group
Ar, Ar¹, Ar² are aromatic or heteroaromatic ring systems

The present invention therefore furthermore relates to a process for the preparation of a compound of the formula (1), characterised in that the diarylamino group is introduced by a C-N coupling reaction between a 1- or 3- or 4-halogenated spirobifluorene substituted by a group R as defined above and a diarylamine.

The compounds according to the invention described above, in particular compounds which are substituted by reactive leaving groups, such as chlorine, bromine, iodine, tosylate, triflate, boronic acid or boronic acid ester, can be used as monomers for the preparation of corresponding oligomers, dendrimers or polymers. The oligomerisation or polymerisation here is preferably carried out via the halogen functionality or the boronic acid functionality.

The invention therefore furthermore relates to oligomers, polymers or dendrimers comprising one or more compounds of the formula (1), where the bond(s) to the polymer, oligomer or dendrimer may be localised at any desired positions in formula (1) substituted by R² to R⁵. Depending on the linking of the compound of the formula (1), the compound is part of a side chain of the oligomer or polymer or part of the main chain. An oligomer in the sense of this invention is taken to mean a compound which is built up from at least three monomer units. A polymer in the sense of the invention is taken to mean a compound which is built up from at least ten monomer units. The polymers, oligomers or dendrimers according to the invention may be conjugated, partially conjugated or non-conjugated. The oligomers or polymers according to the invention may be linear, branched or dendritic. In the structures linked in a linear manner, the units of the formula (1) may be linked directly to one another or linked to one another via a divalent group, for example via a substituted or unsubstituted alkylene group, via a heteroatom or via a divalent aromatic or heteroaromatic group. In branched and dendritic structures, three or more units of the formula (1) may, for example, be linked via a trivalent or polyvalent group, for example via a trivalent or polyvalent aromatic or heteroaromatic group, to give a branched or dendritic oligomer or polymer. The same preferences as described above for compounds of the formula (1) apply to the recurring units of the formula (1) in oligomers, dendrimers and polymers.

For the preparation of the oligomers or polymers, the monomers according to the invention are homopolymerised or copolymerised with further monomers. Suitable and preferred comonomers are selected from fluorenes (for example in accordance with EP 842208 or WO 00/22026), spirobifluorenes (for example in accordance with EP 707020, EP 894107 or WO 06/061181), para-phenylenes (for example in accordance with WO 92/18552), carbazoles (for example in accordance with WO 04/070772 or WO 04/113468), thiophenes (for example in accordance with EP 1028136), dihydrophenanthrenes (for example in accordance with WO 05/014689 or WO 07/006383), cis- and trans-indenofluorenes (for example in accordance with WO 04/041901 or WO 04/113412), ketones (for example in accordance with WO 05/040302), phenanthrenes (for example in accordance with WO 05/104264 or WO 07/017066) or also a plurality of these units. The polymers, oligomers and dendrimers usually also contain further units, for example emitting (fluorescent or phosphorescent) units, such as, for example, vinyltriarylamines (for example in accordance with WO 07/068325) or phosphorescent metal complexes (for example in accordance with WO 06/003000), and/or charge-transport units, in particular those based on triarylamines.

The polymers and oligomers according to the invention are generally prepared by polymerisation of one or more types of monomer, at least one monomer of which results in recurring units of the formula (1) in the polymer. Suitable polymerisation reactions are known to the person skilled in the art and are described in the literature. Particularly suitable and preferred polymerisation reactions which result in C-C or C-N links are the following:
(A) SUZUKI polymerisation;
(B) YAMAMOTO polymerisation;
(C) STILLE polymerisation; and
(D) HARTWIG-BUCHWALD polymerisation.

The way in which the polymerisation can be carried out by these methods and the way in which the polymers can then be separated off from the reaction medium and purified is known to the person skilled in the art and is described in detail in the literature, for example in WO 2003/048225, WO 2004/037887 and WO 2004/037887.

The present invention thus also relates to a process for the preparation of the polymers, oligomers and dendrimers according to the invention, which is characterised in that they are prepared by SUZUKI polymerisation, YAMAMOTO polymerisation, STILLE polymerisation or HARTWIG-BUCHWALD polymerisation. The dendrimers according to the invention can be prepared by processes known to the person skilled in the art or analogously thereto. Suitable processes are described in the literature, such as, for example, in Frechet, Jean M. J.; Hawker, Craig J., "Hyperbranched polyphenylene and hyperbranched polyesters: new soluble, three-dimensional, reactive polymers", Reactive & Functional Polymers (1995), 26(1-3), 127-36; Janssen, H. M.; Meijer, E. W., "The synthesis and characterization of dendritic molecules", Materials Science and Technology (1999), 20 (Synthesis of Polymers), 403-458; Tomalia, Donald A., "Dendrimer molecules", Scientific American (1995), 272(5), 62-6; WO 02/067343 A1 and WO 2005/026144 A1.

The compounds according to the invention are suitable for use in an electronic device. An electronic device here is taken to mean a device which comprises at least one layer which comprises at least one organic compound. However, the component here may also comprise inorganic materials or also layers built up entirely from inorganic materials.

The present invention therefore furthermore relates to the use of the compounds according to the invention in an electronic device, in particular in an organic electroluminescent device.

The present invention still furthermore relates to an electronic device comprising at least one compound according to the invention. The preferences stated above likewise apply to the electronic devices.

The electronic device is preferably selected from the group consisting of organic electroluminescent devices (organic light-emitting diodes, OLEDs), organic integrated circuits (O-ICs), organic field-effect transistors (O-FETs), organic thin-film transistors (O-TFTs), organic light-emitting transistors (O-LETs), organic solar cells (O-SCs), organic dye-sensitised solar cells (ODSSCs), organic optical detectors, organic photoreceptors, organic field-quench devices (O-FQDs), light-emitting electrochemical cells (LECs), organic laser diodes (O-lasers) and organic plasmon emitting devices (D. M. Koller et al., Nature Photonics 2008, 1-4), but preferably organic electroluminescent devices (OLEDs), particularly preferably phosphorescent OLEDs.

The organic electroluminescent devices and the light-emitting electrochemical cells can be employed for various applications, for example for monochromatic or polychromatic displays, for lighting applications or for medical and/or cosmetic applications, for example in phototherapy.

The organic electroluminescent device comprises a cathode, an anode and at least one emitting layer. Apart from these layers, it may also comprise further layers, for example in each case one or more hole-injection layers, hole-transport layers, hole-blocking layers, electron-transport layers, electron-injection layers, exciton-blocking layers, electron-blocking layers and/or charge-generation layers. Interlayers, which have, for example, an exciton-blocking function, may likewise be introduced between two emitting layers. However, it should be pointed out that each of these layers does not necessarily have to be present.

The organic electroluminescent device here may comprise one emitting layer or a plurality of emitting layers. If a plurality of emission layers is present, these preferably have in total a plurality of emission maxima between 380 nm and 750 nm, resulting overall in white emission, i.e. various emitting compounds which are able to fluoresce or phosphoresce are used in the emitting layers. Particular preference is given to systems having three emitting layers, where the three layers exhibit blue, green and orange or red emission (for the basic structure see, for example, WO 2005/011013). It is possible here for all emitting layers to be fluorescent or for all emitting layers to be phosphorescent or for one or more emitting layers to be fluorescent and one or more other layers to be phosphorescent.

The compound according to the invention in accordance with the embodiments indicated above can be employed here in different layers, depending on the precise structure. Preference is given to an organic electroluminescent device comprising a compound of the formula (1) or the preferred embodiments as hole-transport material in a hole-transport or hole-injection or exciton-blocking layer or as matrix material for fluorescent or phosphorescent emitters, in particular for phosphorescent emitters. The preferred embodiments indicated above also apply to the use of the materials in organic electronic devices.

In a preferred embodiment of the invention, the compound of the formula (1) or the preferred embodiments is employed as hole-transport or hole-injection material in a hole-transport or hole-injection layer. The emitting layer here can be fluorescent or phosphorescent. A hole-injection layer in the sense of the present invention is a layer which is directly adjacent to the anode. A hole-transport layer in the sense of the present invention is a layer which is located between a hole-injection layer and an emitting layer.

In still a further preferred embodiment of the invention, the compound of the formula (1) or the preferred embodiments is employed in an exciton-blocking layer. An exciton-blocking layer is taken to mean a layer which is directly adjacent to an emitting layer on the anode side.

The compound of the formula (1) or the preferred embodiments is particularly preferably employed in a hole-transport or exciton-blocking layer.

If the compound of the formula (1) is employed as a hole-transport material in a hole-tranport layer, a hole-injection layer or an exciton-blocking layer, then the compound of formula (1) can be used in such a layer as a single material, i.e. in a proportion of 100%, or the compound of formula (1) can be used in combination with one or more further compounds in such a layer. According to a preferred embodiment, the organic layer comprising the compound of formula (1) additionally comprises one or more p-dopants. Preferred p-dopant for the present invention are organic compounds that can accept electrons (electron acceptors) and can oxidize one or more of the other compounds present in the mixture.

Particularly preferred embodiments of p-dopants are described in WO 2011/073149, EP 1968131, EP 2276085, EP 2213662, EP 1722602, EP 2045848, DE 102007031220, US 8044390, US 8057712, WO 2009/003455, WO 2010/094378, WO 2011/120709, US 2010/0096600, WO 2012/095143 and DE 102012209523.

Particularly preferred as p-dopants are quinodimethane compounds, azaindenofluorendione, azaphenalene, azatriphenylene, I₂, metal halides, preferably transition metal halides, metal oxides, preferably metal oxides containing at least one transition metal or a metal of the 3rd main group and transition metal complexes, preferably complexes of Cu, Co, Ni, Pd and Pt with ligands containing at least one oxygen atom as binding site. Also preferred are transition metal oxides as dopants, preferably oxides of rhenium, molybdenum and tungsten, particularly preferably Re₂O₇, MoO₃, WO₃ and ReO₃.

The p-dopants are preferably distributed substantially uniformly in the p-doped layers. This can be achieved for example by co-evaporation of the p-dopant and of the hole-transport material matrix.

Particularly preferred p-dopants are selected from the compounds (D-1) to (D-13):

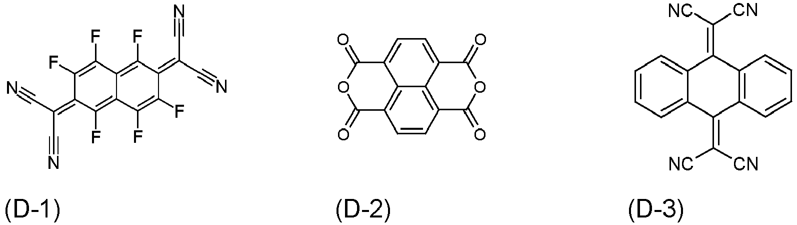

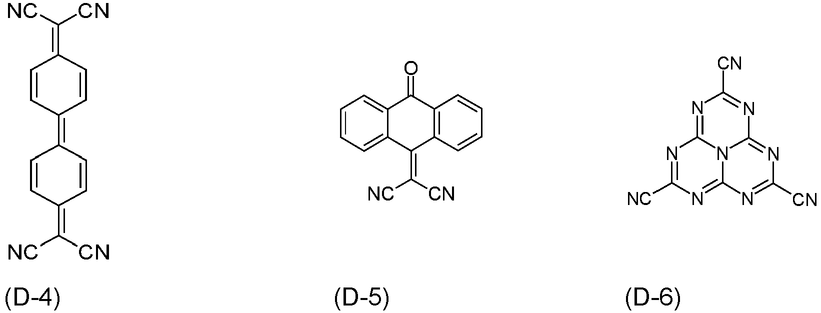

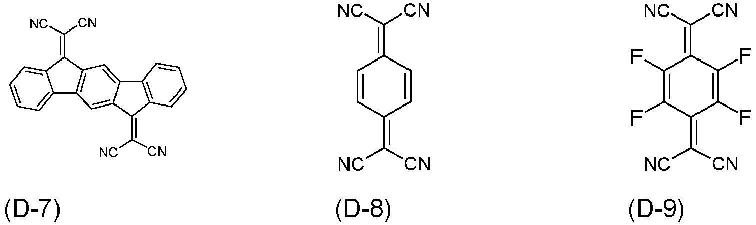

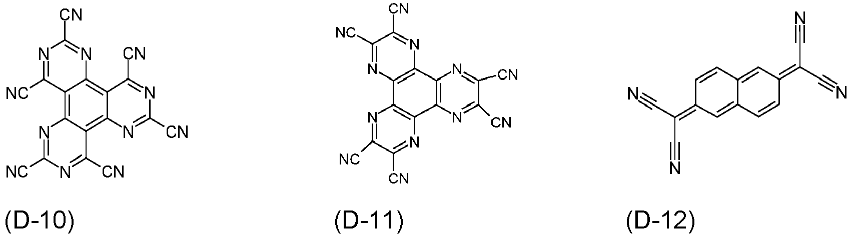

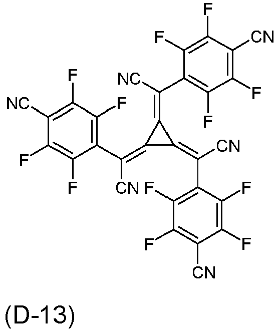

In an embodiment of the invention, the compound of the formula (1) or the preferred embodiments is used in a hole-transport or -injection layer in combination with a layer which comprises a hexaazatriphenylene derivative, in particular hexacyanohexaazatriphenylene (for example in accordance with EP 1175470). Thus, for example, preference is given to a combination which looks as follows: anode - hexaazatriphenylene derivative - hole-transport layer, where the hole-transport layer comprises one or more compounds of the formula (1) or the preferred embodiments. It is likewise possible in this structure to use a plurality of successive hole-transport layers, where at least one hole-transport layer comprises at least one compound of the formula (1) or the preferred embodiments. A further preferred combination looks as follows: anode - hole-transport layer - hexaazatriphenylene derivative - hole-transport layer, where at least one of the two hole-transport layers comprises one or more compounds of the formula (1) or the preferred embodiments. It is likewise possible in this structure to use a plurality of successive hole-transport layers instead of one hole-transport layer, where at least one hole-transport layer comprises at least one compound of the formula (1) or the preferred embodiments.

In a further preferred embodiment of the invention, the compound of the formula (1) or the preferred embodiments is employed as matrix material for a fluorescent or phosphorescent compound, in particular for a phosphorescent compound, in an emitting layer. The organic electroluminescent device here may comprise one emitting layer or a plurality of emitting layers, where at least one emitting layer comprises at least one compound according to the invention as matrix material.

If the compound of the formula (1) or the preferred embodiments is employed as matrix material for an emitting compound in an emitting layer, it is preferably employed in combination with one or more phosphorescent materials (triplet emitters). Phosphorescence in the sense of this invention is taken to mean the luminescence from an excited state having a spin multiplicity > 1, in particular from an excited triplet state. For the purposes of this application, all luminescent complexes containing transition metals or lanthanoids, in particular all luminescent iridium, platinum and copper complexes, are to be regarded as phosphorescent compounds.

The mixture comprising the matrix material, which comprises the compound of the formula (1) or the preferred embodiments, and the emitting compound comprises between 99.9 and 1% by weight, preferably between 99 and 10% by weight, particularly preferably between 97 and 60% by weight, in particular between 95 and 80% by weight, of the matrix material, based on the entire mixture comprising emitter and matrix material. Correspondingly, the mixture comprises between 0.1 and 99% by weight, preferably between 1 and 90% by weight, particularly preferably between 3 and 40% by weight, in particular between 5 and 20% by weight, of the emitter, based on the entire mixture comprising emitter and matrix material. The limits indicated above apply, in particular, if the layer is applied from solution. If the layer is applied by vacuum evaporation, the same numerical values apply, with the percentage in this case being indicated in % by vol. in each case.

A particularly preferred embodiment of the present invention is the use of the compound of the formula (1) or the preferred embodiments as matrix material for a phosphorescent emitter in combination with a further matrix material. Particularly suitable matrix materials which can be employed in combination with the compounds of the formula (1) or the preferred embodiments are aromatic ketones, aromatic phosphine oxides or aromatic sulfoxides or sulfones, for example in accordance with WO 2004/013080, WO 2004/093207, WO 2006/005627 or WO 2010/006680, triarylamines, carbazole derivatives, for example CBP (N,N-biscarbazolylbiphenyl), m-CBP or the carbazole derivatives disclosed in WO 2005/039246, US 2005/0069729, JP 2004/288381, EP 1205527 or WO 2008/086851, indolocarbazole derivatives, for example in accordance with WO 2007/063754 or WO 2008/056746, indenocarbazole derivatives, for example in accordance with WO 2010/136109 or WO 2011/000455, aza-carbazole derivatives, for example in accordance with EP 1617710, EP 1617711, EP 1731584, JP 2005/347160, bipolar matrix materials, for example in accordance with WO 2007/137725, silanes, for example in accordance with WO 2005/111172, azaboroles or boronic esters, for example in accordance with WO 2006/117052, triazine derivatives, for example in accordance with WO 2010/015306, WO 2007/063754 or WO 08/056746, zinc complexes, for example in accordance with EP 652273 or WO 2009/062578, fluorene derivatives, for example in accordance with WO 2009/124627, diazasilole or tetraazasilole derivatives, for example in accordance with WO 2010/054729, diazaphosphole derivatives, for example in accordance with WO 2010/054730, or bridged carbazole derivatives, for example in accordance with US 2009/0136779, WO 2010/050778, WO 2011/042107 or WO 2011/088877. It is furthermore possible to use an electronically neutral co-host which has neither hole-transporting nor electron-transporting properties, as described, for example, in WO 2010/108579.

It is likewise possible to use two or more phosphorescent emitters in the mixture. In this case, the emitter, which emits at shorter wavelength, acts as co-host in the mixture.

Suitable phosphorescent compounds (= triplet emitters) are, in particular, compounds which emit light, preferably in the visible region, on suitable excitation and in addition contain at least one atom having an atomic number greater than 20, preferably greater than 38 and less than 84, particularly preferably greater than 56 and less than 80, in particular a metal having this atomic number. The phosphorescent emitters used are preferably compounds which contain copper, molybdenum, tungsten, rhenium, ruthenium, osmium, rhodium, iridium, palladium, platinum, silver, gold or europium, in particular compounds which contain iridium, platinum or copper.

Examples of the emitters described above are revealed by the applications WO 2000/70655, WO 2001/41512, WO 2002/02714, WO 2002/15645, EP 1191613, EP 1191612, EP 1191614, WO 2005/033244, WO 2005/019373, US 2005/0258742, WO 2009/146770, WO 2010/015307, WO 2010/031485, WO 2010/054731, WO 2010/054728, WO 2010/086089, WO 2010/099852, WO 2010/102709, WO 2011/157339 or WO 2012/007086. In general, all phosphorescent complexes as used in accordance with the prior art for phosphorescent OLEDs and as are known to the person skilled in the art in the area of organic electroluminescence are suitable, and the person skilled in the art will be able to use further phosphorescent complexes without inventive step.

In a further embodiment of the invention, the organic electroluminescent device according to the invention does not comprise a separate hole-injection layer and/or hole-transport layer and/or hole-blocking layer and/or electron-transport layer, i.e. the emitting layer is directly adjacent to the hole-injection layer or the anode, and/or the emitting layer is directly adjacent to the electron-transport layer or the electron-injection layer or the cathode, as described, for example, in WO 2005/053051. It is furthermore possible to use a metal complex which is identical or similar to the metal complex in the emitting layer as hole-transport or hole-injection material directly adjacent to the emitting layer, as described, for example, in WO 2009/030981.

It is furthermore possible to use the compound of the formula (1) or the preferred embodiments both in a hole-transport layer or exciton-blocking layer and as matrix in an emitting layer.

In the further layers of the organic electroluminescent device according to the invention, it is possible to use all materials as usually employed in accordance with the prior art. The person skilled in the art will therefore be able, without inventive step, to employ all materials known for organic electroluminescent devices in combination with the compounds of the formula (1) according to the invention or the preferred embodiments.

Preference is furthermore given to an organic electroluminescent device, characterised in that one or more layers are applied by means of a sublimation process, in which the materials are vapour-deposited in vacuum sublimation units at an initial pressure of usually less than 10⁻⁵ mbar, preferably less than 10⁻⁶ mbar. However, it is also possible for the initial pressure to be even lower, for example less than 10⁻⁷ mbar.

Preference is likewise given to an organic electroluminescent device, characterised in that one or more layers are applied by means of the OVPD (organic vapour phase deposition) process or with the aid of carrier-gas sublimation, in which the materials are applied at a pressure between 10⁻⁵ mbar and 1 bar. A special case of this process is the OVJP (organic vapour jet printing) process, in which the materials are applied directly through a nozzle and thus structured (for example M. S. Arnold et al., Appl. Phys. Lett. 2008, 92, 053301).

Preference is furthermore given to an organic electroluminescent device, characterised in that one or more layers are produced from solution, such as, for example, by spin coating, or by means of any desired printing process, such as, for example, LITI (light induced thermal imaging, thermal transfer printing), ink-jet printing, screen printing, flexographic printing, offset printing or nozzle printing. Soluble compounds, which are obtained, for example, by suitable substitution, are necessary for this purpose. These processes are also particularly suitable for the compounds according to the invention, since these generally have very good solubility in organic solvents.

Also possible are hybrid processes, in which, for example, one or more layers are applied from solution and one or more further layers are applied by vapour deposition. Thus, for example, the emitting layer can be applied from solution and the electron-transport layer by vapour deposition.

These processes are generally known to the person skilled in the art and can be applied by him without inventive step to organic electroluminescent devices comprising the compounds according to the invention.

The processing of the compounds according to the invention from the liquid phase, for example by spin coating or by printing processes, requires formulations of the compounds according to the invention. These formulations can be, for example, solutions, dispersions or mini-emulsions. It may be preferred to use mixtures of two or more solvents for this purpose. Suitable and preferred solvents are, for example, toluene, anisole, o-, m- or p-xylene, methyl benzoate, dimethylanisole, mesitylene, tetralin, veratrol, THF, methyl-THF, THP, chlorobenzene, dioxane or mixtures of these solvents.

The present invention therefore furthermore relates to a formulation, in particular a solution, dispersion or mini-emulsion, comprising at least one compound of the formula (1) or the preferred embodiments indicated above and at least one solvent, in particular an organic solvent. The way in which solutions of this type can be prepared is known to the person skilled in the art and is described, for example, in WO 2002/072714, WO 2003/019694 and the literature cited therein.

The present invention furthermore relates to mixtures comprising at least one compound of the formula (1) or the preferred embodiments indicated above and at least one further compound. The further compound can be, for example, a fluorescent or phosphorescent dopant if the compound according to the invention is used as matrix material. The mixture may then also additionally comprise a further material as additional matrix material.

The invention is explained in greater detail by the following examples, without wishing to restrict it thereby. On the basis of the descriptions, the person skilled in the art will be able to carry out the invention throughout the range disclosed and prepare further compounds according to the invention without inventive step and use them in electronic devices or use the process according to the invention.

### Examples (embodiments which are not according to the claims are marked with #):

### A) Synthesis examples

The following syntheses are carried out under a protective-gas atmosphere, unless indicated otherwise. The starting materials can be purchased from ALDRICH or ABCR. The numbers in square brackets in the case of the starting materials known from the literature are the corresponding CAS numbers.

### Example 1

### Synthesis of bis-(biphenyl-2-yl)-(2'-phenyl-9,9'-spirobifluoren-4-yl)-amine (1-1) and derivatives (1-2) to (1-28)

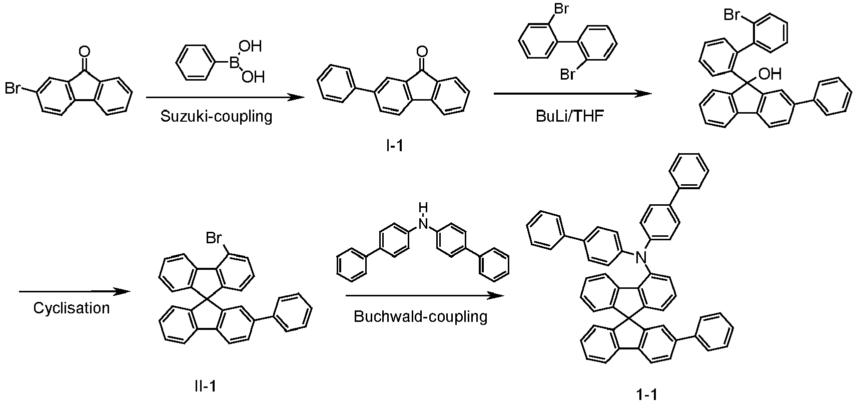

### Synthesis of 2'-phenyl-9H-fluoren-9-one (intermediate 1-1)

50 g (193 mmol) of 2-bromo-9H-fluoren-9-one (25,9 g, 212 mmol) of phenyl boronic acid, 2,23 g (1,93 mmol) of Pd(PPh₃)₄, 25,9 g (386 mmol) of sodium carbonate are dissolved in 460 mL of toluene, 230 mL of water and 170 ml of ethanol. The reaction mixture is refluxed and agitated under an argon atmosphere for 12 hours and after cooling to room temperature, the mixture is filtered through Celite. The filtrate is evaporated *in vacuo,* and the residue is crystallised from heptane. The crude product is extracted in a Soxhlet extractor (toluene) and purified by recrystallization in toluene/heptane. The product is isolated in the form of an off-white solid (49 g, 85% of theory. The following compounds are synthesized analogously:

| Ex. | Bromo-fluorenone | Aryl-fluorenone | Product | Yield |
|---|---|---|---|---|
| I-1 | 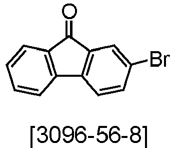 | 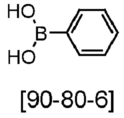 | 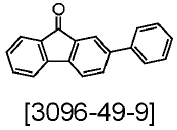 | 85% |
| I-2 | 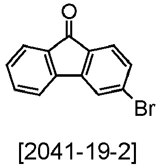 | 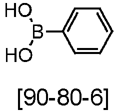 | 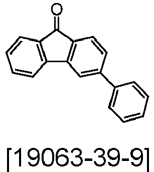 | 85% |
| I-3 | 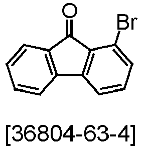 | 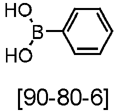 | 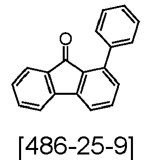 | 90% |
| I-4 | 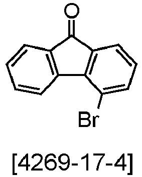 | 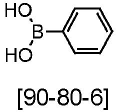 | 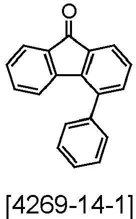 | 90% |
| I-5 | 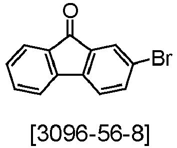 | 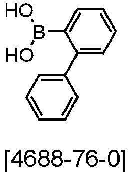 | 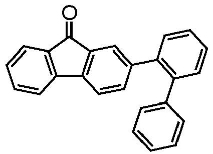 | 81% |
| I-6 | 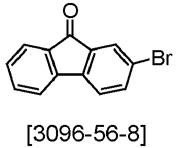 | 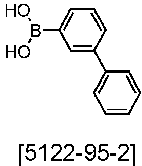 | 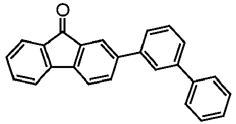 | 81% |
| I-7 | 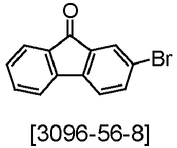 | 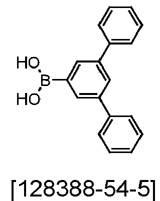 | 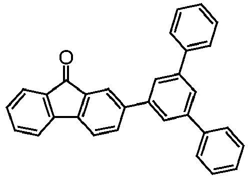 | 90% |
| I-8 | 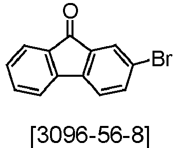 | 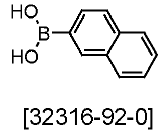 | 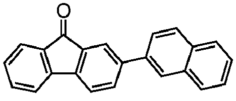 | 87% |
| I-9 | 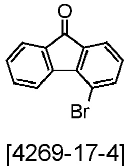 | 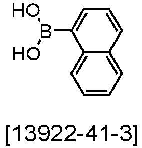 | 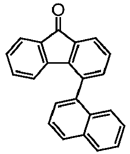 | 85% |
| I-10 | 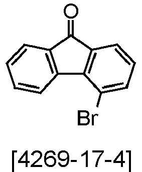 | 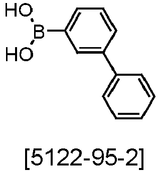 | 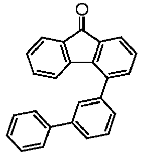 | 80% |
| I-11 | 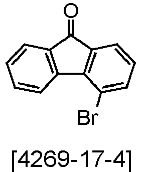 | 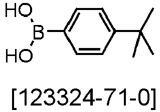 | 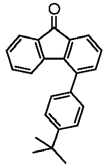 | 80% |
| I-12 | 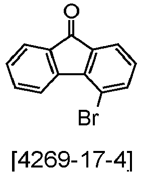 | 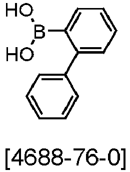 | 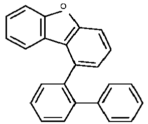 | 88% |
| I-13 | 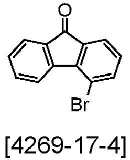 | 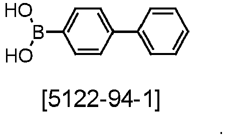 | 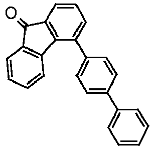 | 79% |
| I-14 | 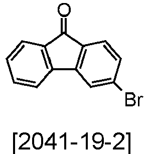 | 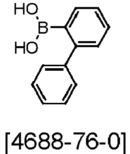 | 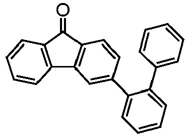 | 85% |

### Synthesis of 4-bromo-spiro-2'-phenyl-9,9'-bifluorene (intermediate II-1)

A solution of 2, 2'-dibromo-biphenyl (35 g, 110 mmol) in THF (400ml) is treated with 61 mL of n-BuLi (1,8 M in hexane, 110 mmol) under argon at minus 78 °C. The mixture is stirred for 30 minutes. A solution of 2-Phenyl-fluoren-9-one (CAS: 3096-49-9) (28,2 g, 110 mmol) in 100 mL THF is added dropwise. The reaction proceeds at -78 °C for 30 minutes and then is stirred at room temperature overnight. The reaction is quenched with water and the solid is filtered. Without further purification, a mixture of the alcohol, acetic acid (400 mL) and concentrated HCl (20 mL) is refluxed for 2 hours. After cooling, the mixture is filtered and washed with water and dried under vacuum. The product is isolated in the form of a white solid (45 g, 86% of theory).

The synthesis of further halogenated aryl-substituted-spirobifluorene derivatives is carried out analogously:

| Ex. | Bromo-biphenyl | Aryl-fluorenone | Product | Yield |
|---|---|---|---|---|
| II-1 | 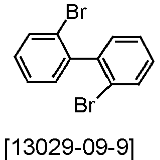 | 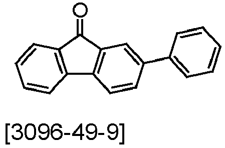 | 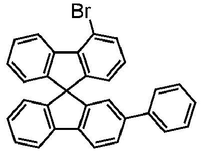 | 86% |
| II-2 | 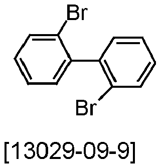 | 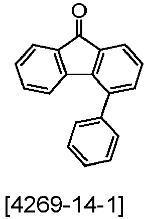 | 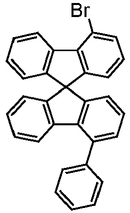 | 84% |
| II-3 | 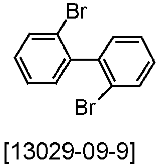 | 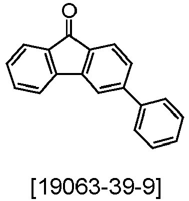 | 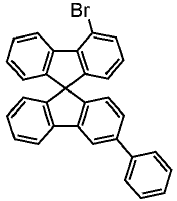 | 90% |
| II-4 | 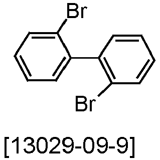 | 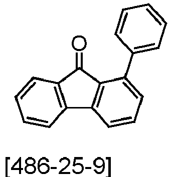 | 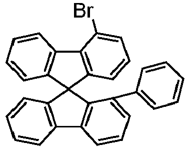 | 90% |
| II-5 | 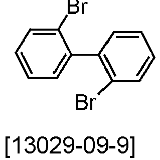 | 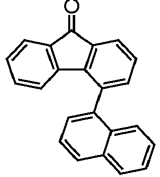 | 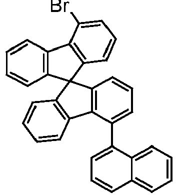 | 66% |
| II-6 | 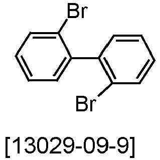 | 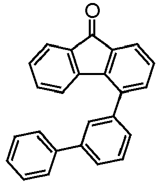 | 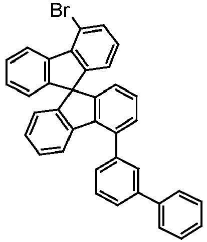 | 81% |
| II-7 | 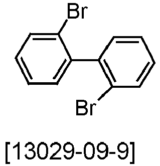 | 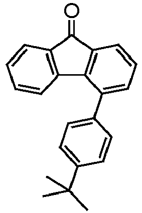 | 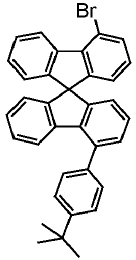 | 77% |
| II-8 | 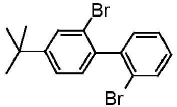 | 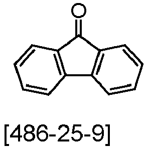 | 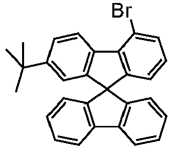 | 85% |
| II-9 | 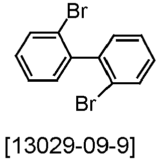 | 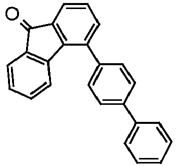 | 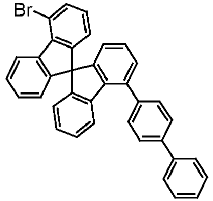 | 81% |
| II-10 | 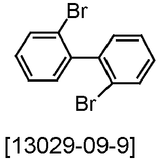 | 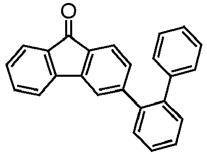 | 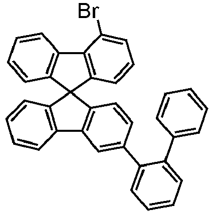 | 87% |
| II-11 | 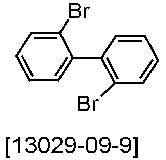 | 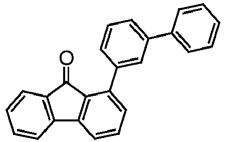 | 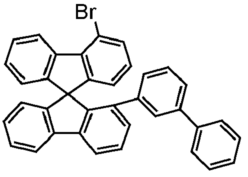 | 75% |
| II-12 | 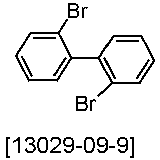 | 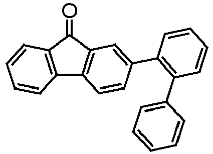 | 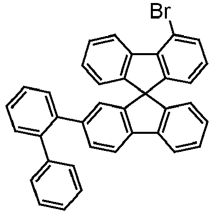 | 80% |
| II-13 | 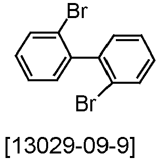 | 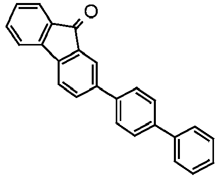 | 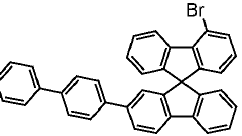 | 76% |
| II-14 | 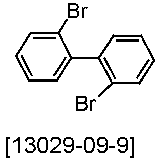 | 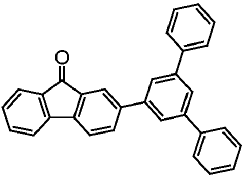 | 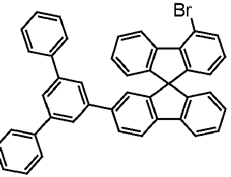 | 80% |
| II-15 | 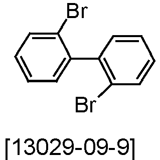 | 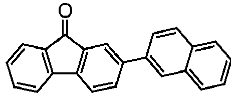 | 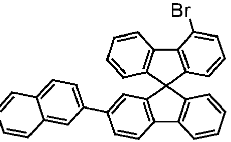 | 90% |
| II-16 | 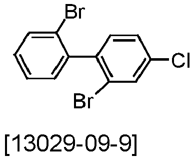 | 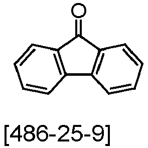 | 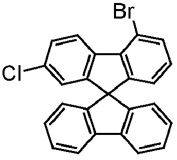 | 65% |
| II-17 | 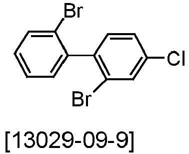 | 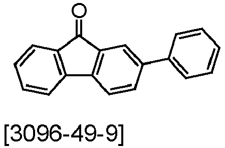 | 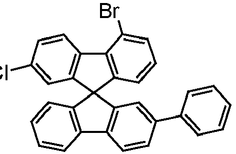 | 68% |
| II-18 | 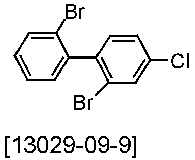 | 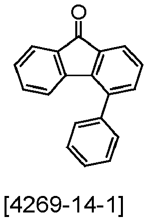 | 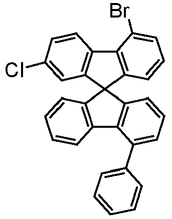 | 65% |
| II-19 | 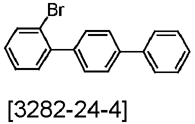 | 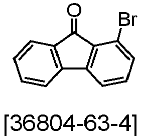 | 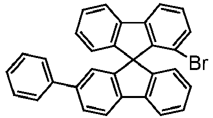 | 77% |
| II-20 | 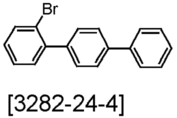 | 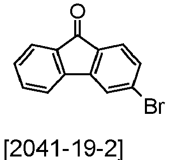 | 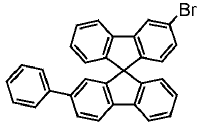 | 63% |
| II-21 | 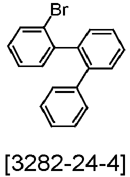 | 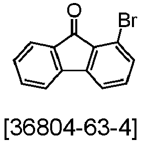 | 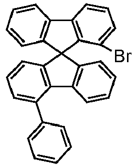 | 65% |
| II-22 | 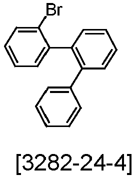 | 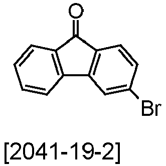 | 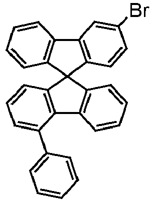 | 74% |

### Synthesis of bis-(biphenyl-2-yl)-(2'-phenyl-9,9'-spirobifluoren-4-yl)-amine (1-1)

Tri-tert-butylphosphine (1,27 ml of a 1.0 M solution in toluene, 1,27 mmol), palladium acetate (286 mg, 1.27 mmol) and sodium tert-butoxide (8.16 g, 84,9 mmol) are added to a solution of bis-biphenyl-2-yl-amine (13,6 g, 42.4 mmol) and 4-bromo-2'-phenyl-9,9'-spirobifluorene (20.0 g, 42.4 mmol) in degassed toluene (400 ml), and the mixture is heated under reflux for 3 h. The reaction mixture is cooled to room temperature, extended with toluene and filtered through Celite. The filtrate is evaporated *in vacuo,* and the residue is crystallised from toluene/heptane. The crude product is extracted in a Soxhlet extractor (toluene) and purified by recrystallization in heptane/toluene (21 g, 70% of theory). After sublimation *in vacuo,* the product is isolated in the form of an off-white solid.

The following compounds are obtained analogously:

| **Ex.** | **Halogenated-spiro** | **Amine** | **Product** | **Yield** |
|---|---|---|---|---|
| **1-1** | 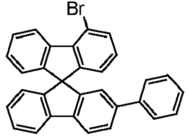 | 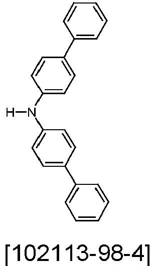 | 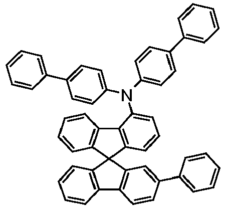 | 70% |
| **1-2** | 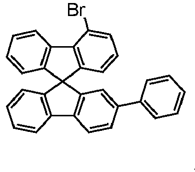 | 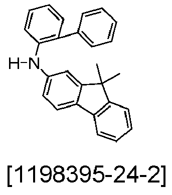 | 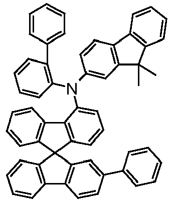 | 72% |
| **1-3** | 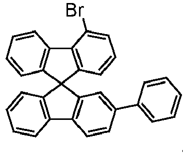 | 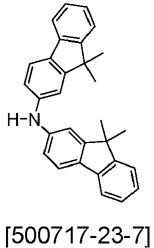 | 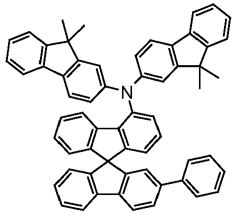 | 55% |
| **1-4** | 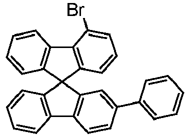 | 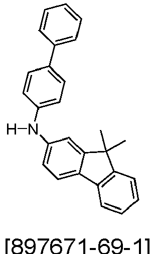 | 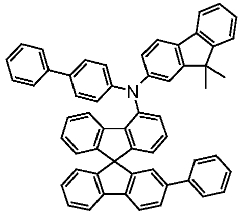 | 64% |
| **1-5** | 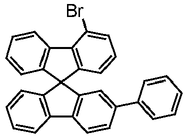 | 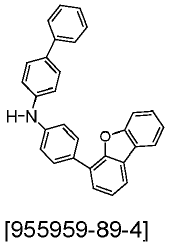 | 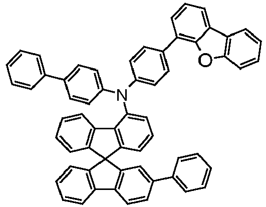 | 56% |
| **1-6** | 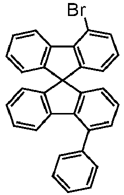 | 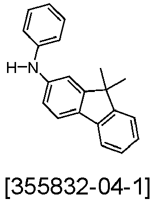 | 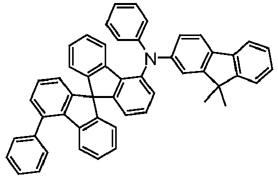 | 73% |
| **1-7** | 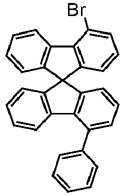 | 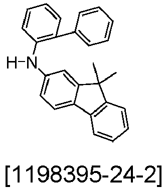 | 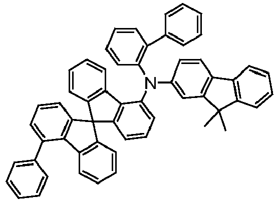 | 65% |
| **1-8** | 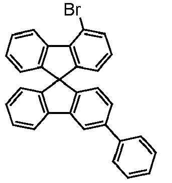 | 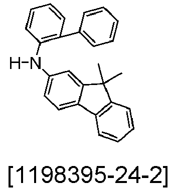 | 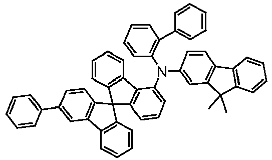 | 67% |
| **1-9** | 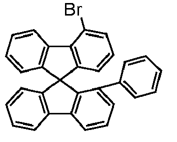 | 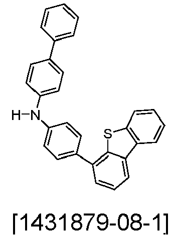 | 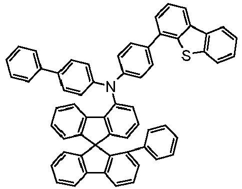 | 66% |
| **1-10** | 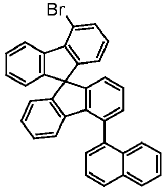 | 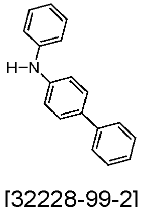 | 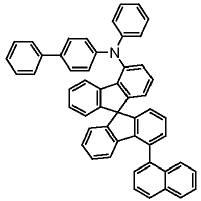 | 50% |
| **1-11** | 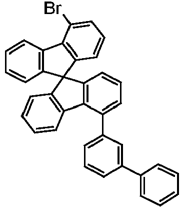 | 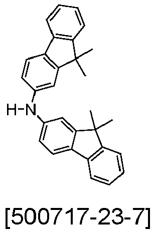 | 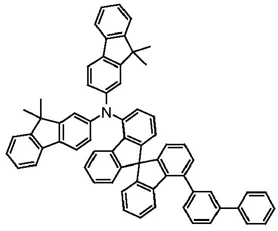 | 62% |
| **1-12#** | 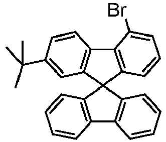 | 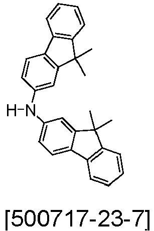 | 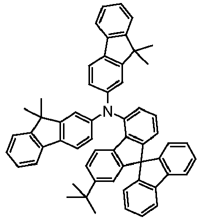 | 71% |
| **1-13** | 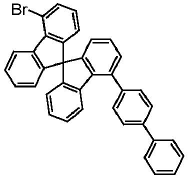 | 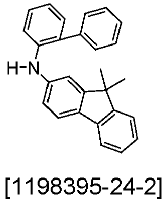 | 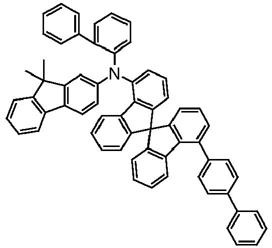 | 69% |
| **1-14** | 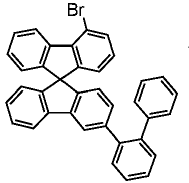 | 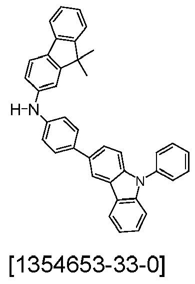 | 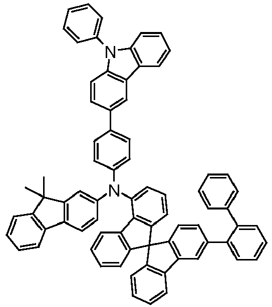 | 57% |
| **1-15#** | 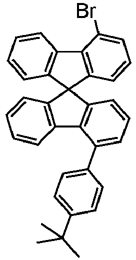 | 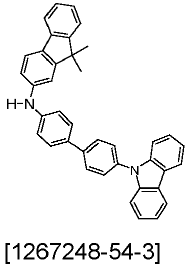 | 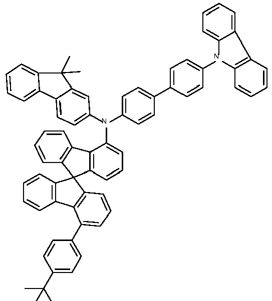 | 65% |
| **1-16** | 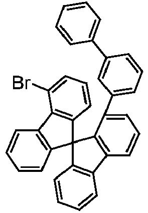 | 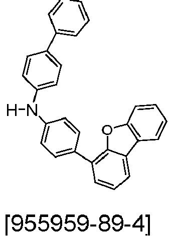 | 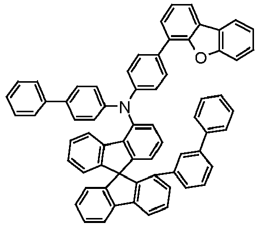 | 71% |
| **1-17** | 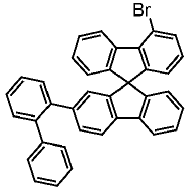 | 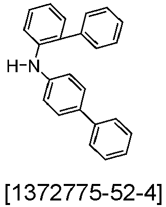 | 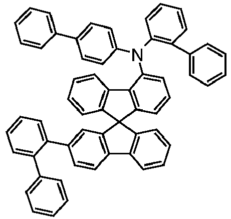 | 71% |
| **1-18** | 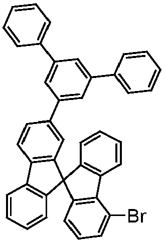 | 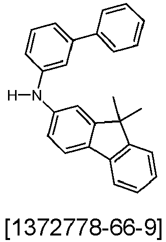 | 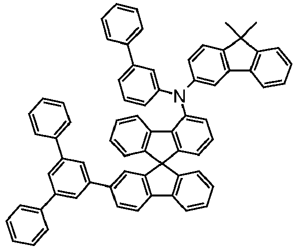 | 72% |
| **1-19** | 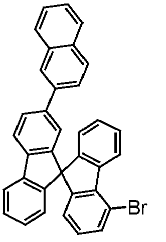 | 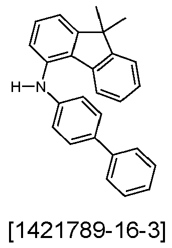 | 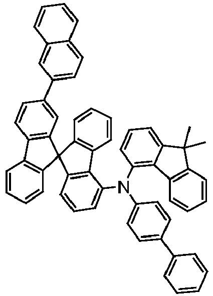 | 62% |
| **1-20** | 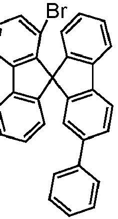 | 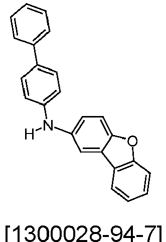 | 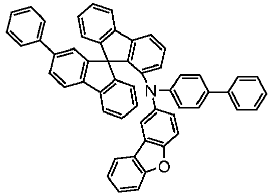 | 45% |
| **1-21** | 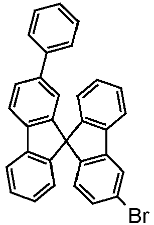 | 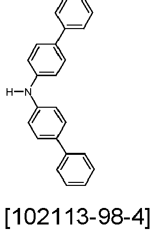 | 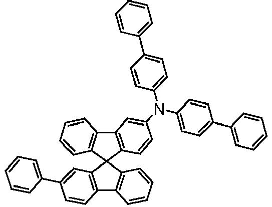 | 66% |
| **1-22** | 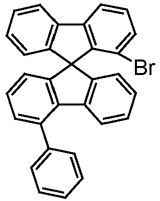 | 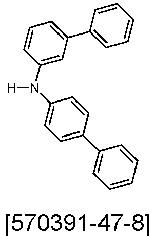 | 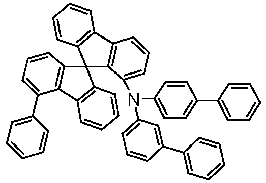 | 36% |
| **1-23** | 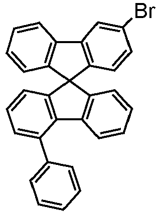 | 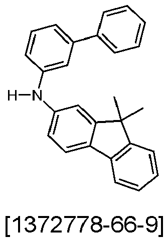 | 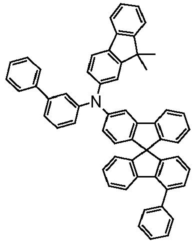 | 71% |
| **1-24** | 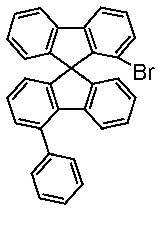 | 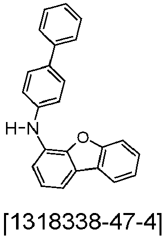 | 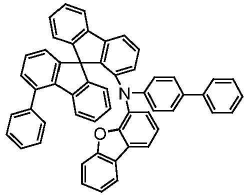 | 39% |
| **1-25#** | 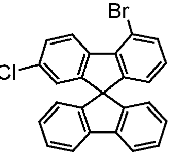 | 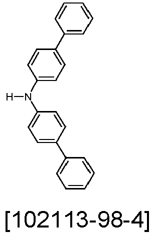 | 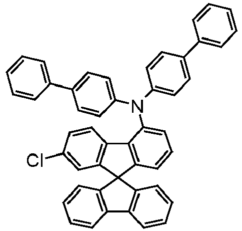 | 65% |
| **1-26#** | 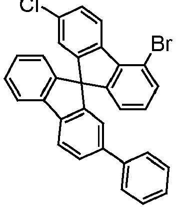 | 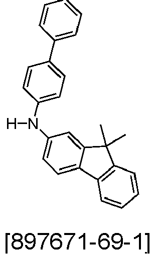 | 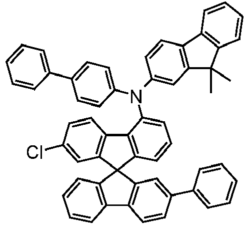 | 73% |
| **1-27#** | 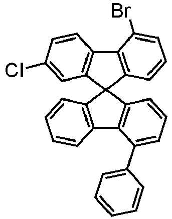 | 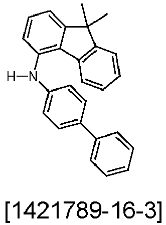 | 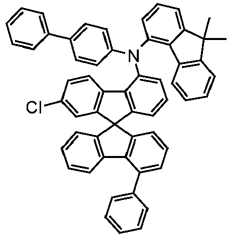 | 70% |
| **1-28** | 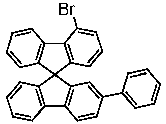 | 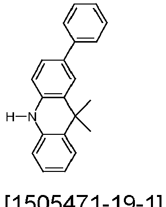 | 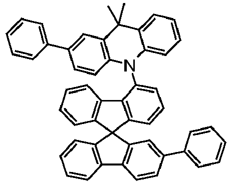 | 68% |

### Example 2

### Synthesis of Synthesis of Biphenyl-4-yl-(9,9-dimethyl-9H-fluoren-2-yl)-[2'-phenyl-4-(9,9'-spiro-bifluoren-4-yl)-phenyl]-amine (2-1) and the derivatives (2-2) to (2-15)

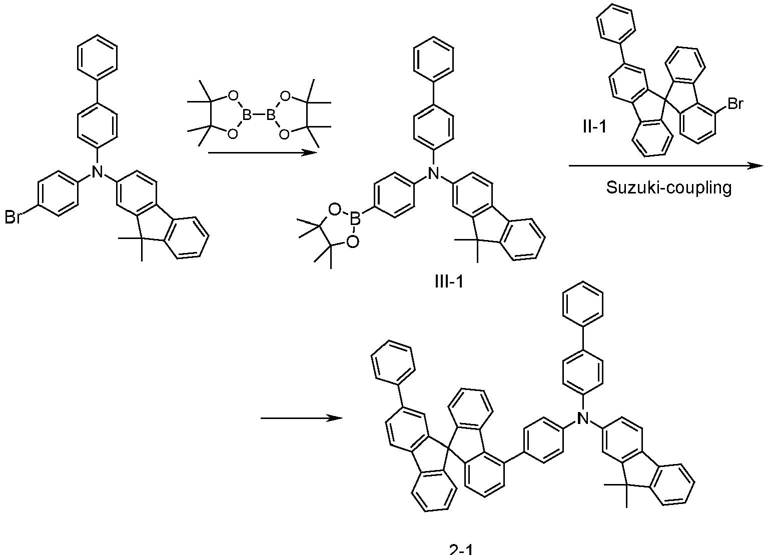

### Synthesis of Biphenyl-4-yl-(9,9-dimethyl-9H-fluoren-2-yl (4,4,5,5tetramethyl-[1,3,2]dioxaborolan-2-yl)-phenyl]-amine

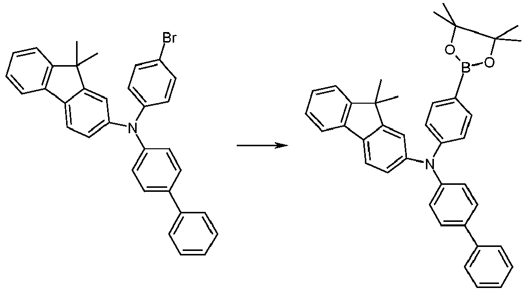

102 g (198 mmol) of biphenyl-4-yl-(4-bromo-phenyl)-(9,9-dimethyl-9H-fluoren-2-yl)-amine, 4,8 g (5,9 mmol) of Pd(dppf)Cl₂, 61,6 g (238 mmol) of bis(pinacolato)diboron and 58,3 g (594 mmol) of potassium acetate are dissolved in 1300 mL of 1,4-dioxane. The reaction mixture is refluxed and agitated under an argon atmosphere for 12 hours and after cooling to room temperature, the mixture is filtered through Celite. The filtrate is evaporated *in vacuo,* and the residue is crystallised from heptane. The product is isolated in the form of a pale-yellow solid (87 g, 78% of theory).

### Synthesis of Synthesis of Biphenyl-4-yl-(9,9-dimethyl-9H-fluoren-2-yl)-[2'-phenyl-4-(9,9'-spiro-bifluoren-4-yl)-phenyl]-amine (2-1)

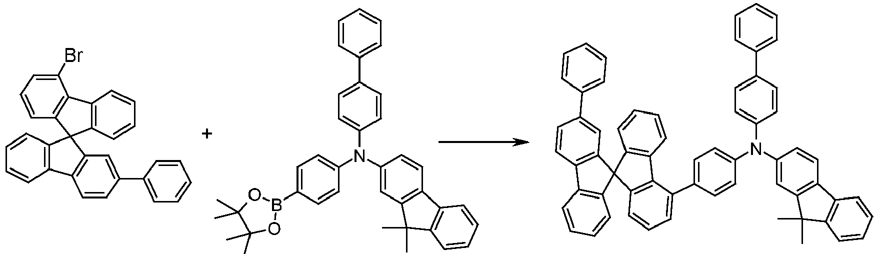

59.1 g (101.8 mmol) of Biphenyl-4-yl-(9,9-dimethyl-9H-fluoren-2-yl (4,4,5,5-tetramethyl-[1,3,2]dioxaborolan-2-yl)-phenyl]-amine, 50.2g (101.8 mmol) of 4-brom-2'-phenyl-spirobifluorene, 2.43 g (2.10 mmol) of Pd(PPh3)4, and 59.3 g (205.6 mmol) of sodium carbonate are dissolved in 600 mL of toluene and 100 ml water. The reaction mixture is refluxed and agitated under an argon atmosphere for 12 hours and after cooling to room temperature, the mixture is filtered through Celite. The filtrate is evaporated *in vacuo,* and the residue is crystallised from heptane. The crude product is extracted in a Soxhlet extractor (toluene) and purified by recrystallisation (42 g, 50% of theory). After sublimation *in vacuo, t*he product is isolated in the form of a white solid.

The following compounds are synthesized analogously:

| **Ex.** | Halogenated-spiro | Amine | Product | Yield |
|---|---|---|---|---|
| **2-1** | 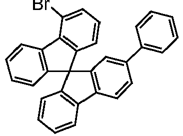 | 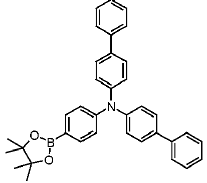 | 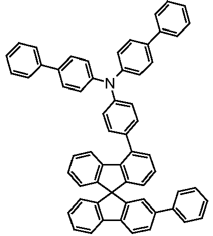 | 50% |
| **2-2** | 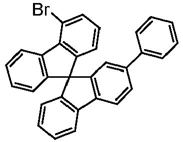 | 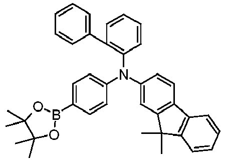 | 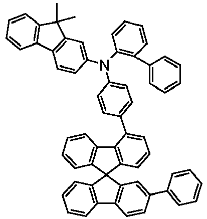 | 55% |
| **2-3** | 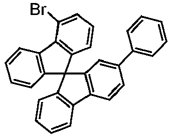 | 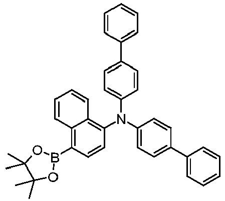 | 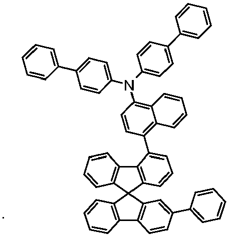 | 60% |
| **2-4** | 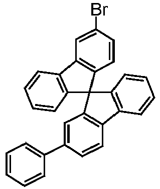 | 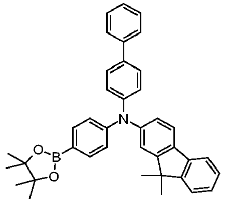 | 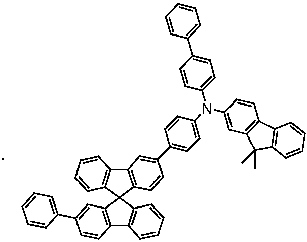 | 63% |
| **2-5** | 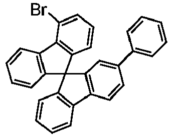 | 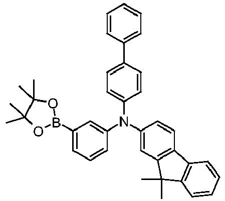 | 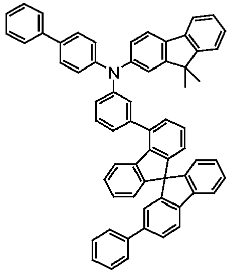 | 70% |
| **2-6** | 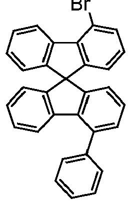 | 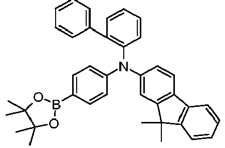 | 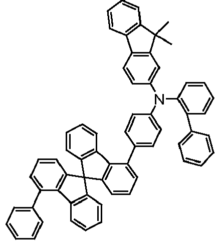 | 75% |
| **2-7** | 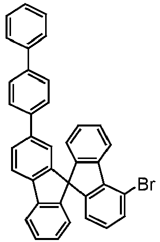 | 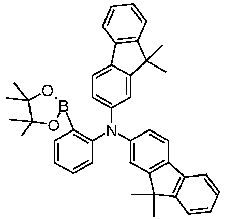 | 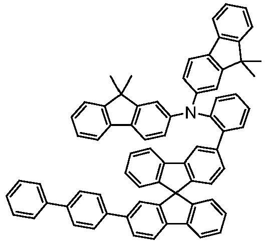 | 55% |
| **2-8** | 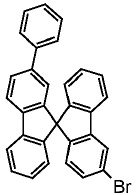 | 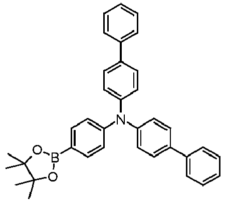 | 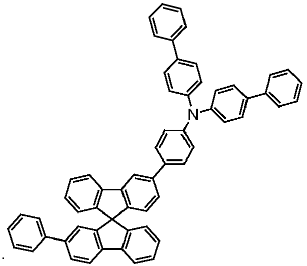 | 64% |
| **2-9** | 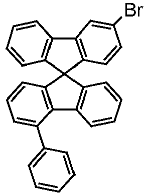 | 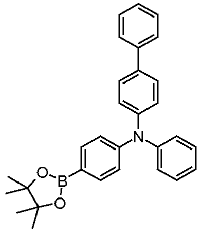 | 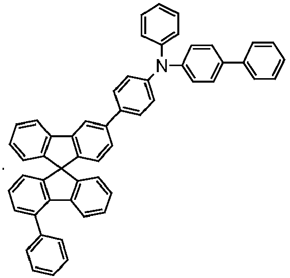 | 60% |
| **2-10** | 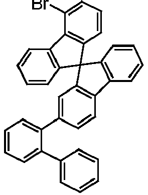 | 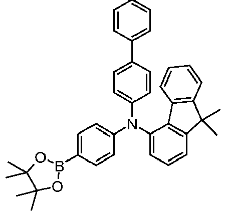 | 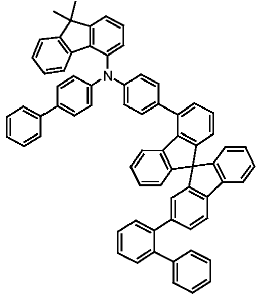 | 59% |
| **2-11 #** | 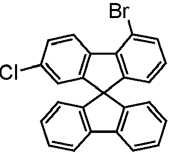 | 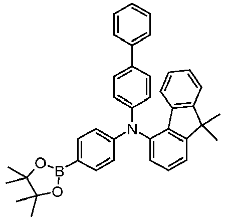 | 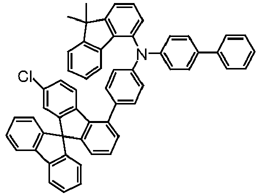 | 75% |
| **2-12 #** | 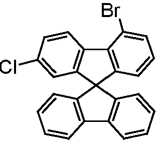 I | 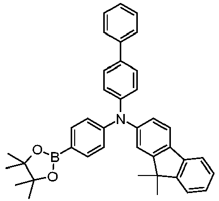 | 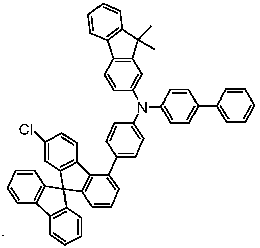 | 81% |
| **2-13 #** | 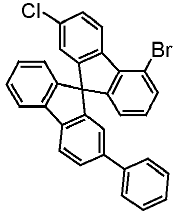 | 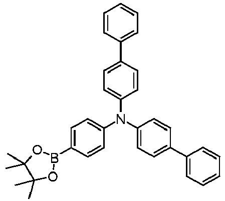 | 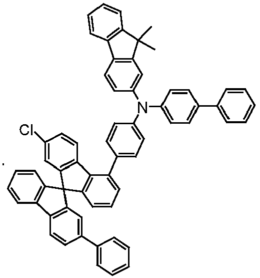 | 65% |
| **2-14 #** | 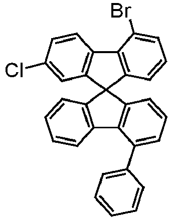 | 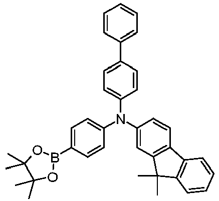 | 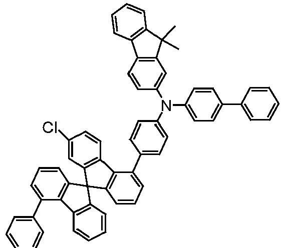 | 67% |
| **2-15** | 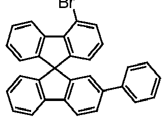 | 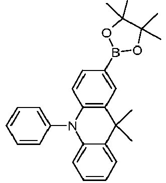 | 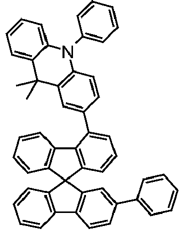 | 58% |
| **2-16** | 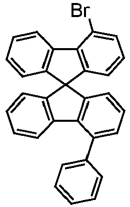 | 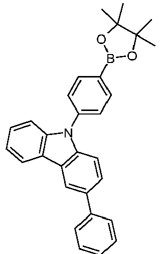 | 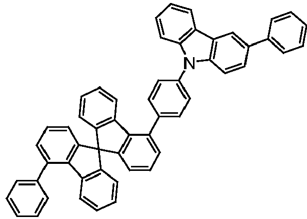 | 62% |

### Example 3

### Synthesis of bis-(biphenyl-2-yl)-(7-phenyl-9,9'-spirobifluoren-4-yl)-amine (3-1) and derivatives (3-2) bis (3-8)

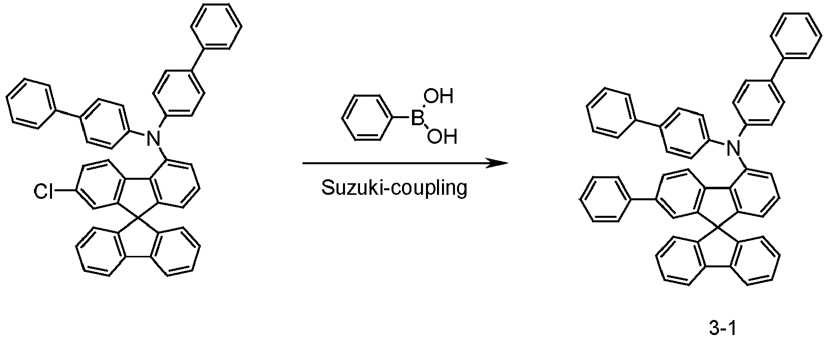

30g (44,8 mmol) of bis-(biphenyl-2-yl)-(7-chloro-9,9'-spirobifluoren-4-yl)-amine, 9,6 g (47,0 mmol) of phenyl-boronic acid, 1,66 g (2,25mmol) of PdCl₂(Cy)₃, and 13,6 g (89,5 mmol) of cesium fluoride are dissolved in 500 mL of dioxane. The reaction mixture is refluxed and agitated under an argon atmosphere for 12 hours and after cooling to room temperature, the mixture is filtered through Celite. The filtrate is evaporated *in vacuo,* and the residue is crystallised from heptane. The crude product is extracted in a Soxhlet extractor (toluene) and purified by zone sublimation *in vacuo.* The product is isolated in the form of a off-white solid (14g, 46% of theory).

The following compounds are synthesized analogously:

| Ex. | Halogenated-spiro | Amine | Product | Yield |
|---|---|---|---|---|
| **3-1** | 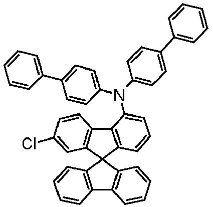 | 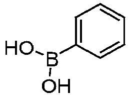 | 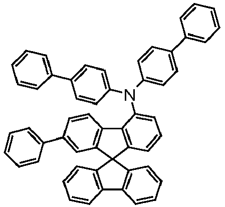 | 46% |
| **3-2** | 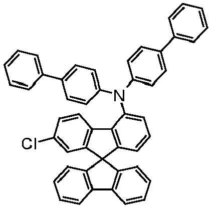 | 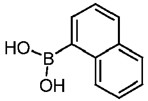 | 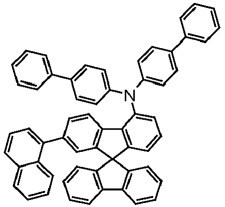 | 52% |
| **3-3 #** | 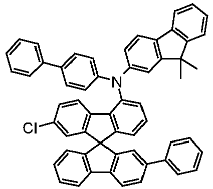 | 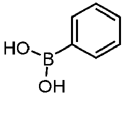 | 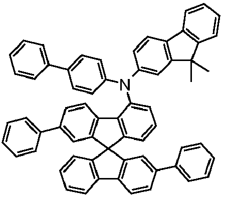 | 43% |
| **3-4 #** | 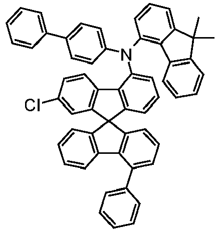 | 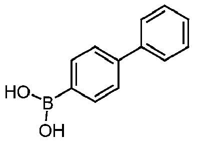 | 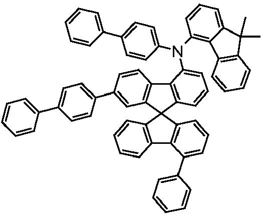 | 48% |
| **3-5** | 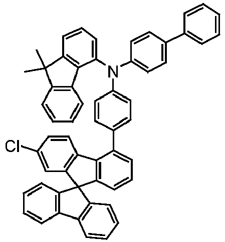 | 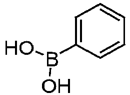 | 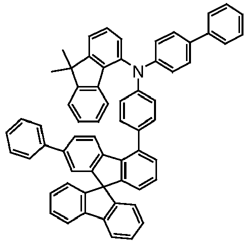 | 70% |
| **3-6** | 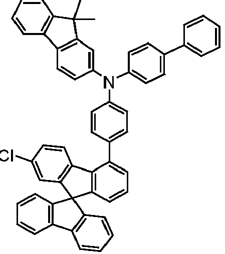 I | 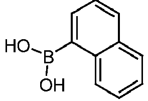 | 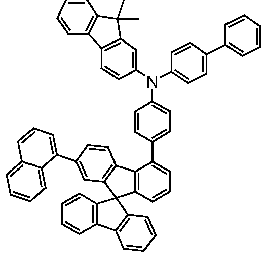 | 65% |
| **3-7 #** | 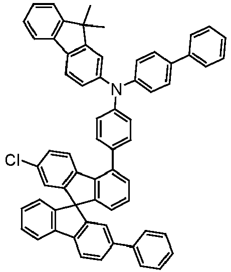 | 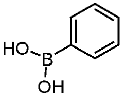 | 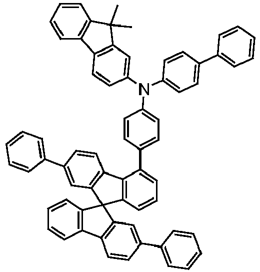 | 55% |
| **3-8 #** | 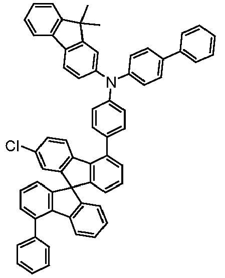 | 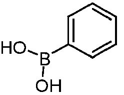 | 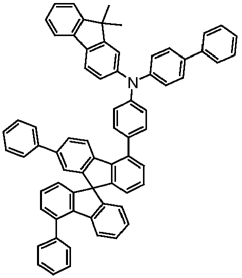 | 64% |

### B) Devices examples

OLEDs according to the invention and OLEDs in accordance with the prior art are produced by a general process in accordance with WO 2004/058911, which is adapted to the circumstances described here (layer-thickness variation, materials).

The data for various OLEDs are presented in Examples E1 to E7 below (see Tables 1 to 6). The substrates used are glass plates coated with structured ITO (indium tin oxide) in a thickness of 50nm. The OLEDs basically have the following layer structure: substrate / hole-injection layer (HIL) / hole-transport layer (HTL) / hole-injection layer (HTL2) / electron-blocking layer (EBL) / emission layer (EML) / electron-transport layer (ETL) / electron-injection layer (EIL) and finally a cathode. The cathode is formed by an aluminium layer with a thickness of 100nm. The precise structure of the OLEDs is shown in tables 1, 3 and 5. The materials required for the production of the OLEDs are shown in table 7.

All materials are applied by thermal vapour deposition in a vacuum chamber. The emission layer here always consists of at least one matrix material (host material) and an emitting dopant (emitter), which is admixed with the matrix material or matrix materials in a certain proportion by volume by co-evaporation. An expression such as H1:SEB (5%) here means that material H1 is present in the layer in a proportion by volume of 95% and SEB is present in the layer in a proportion of 5%. Analogously, other layers may also consist of a mixture of two or more materials.

The OLEDs are characterised by standard methods. For this purpose, the electroluminescence spectra and the external quantum efficiency (EQE, measured in per cent) as a function of the luminous density, calculated from current/voltage/luminous density characteristic lines (IUL characteristic lines) assuming Lambert emission characteristics, and the lifetime are determined. The expression EQE @ 10mA/cm² denotes the external quantum efficiency at an operating current density of 10mA/cm². LT80 @ 60mA/cm² is the lifetime until the OLED has dropped from its initial luminance of i.e. 5000cd/m² to 80% of the initial intensity, i.e. to 4000cd/m² without using any acceleration factor. The data for the various OLEDs containing inventive and comparative materials are summarised in tables 2, 4 and 6.

### Use of compounds according to the invention as hole-transport materials in fluorescent OLEDs

In particular, compounds according to the invention are suitable as HIL, HTL, EBL or matrix material in the EML in OLEDs. They are suitable as a single layer, but also as mixed component as HIL, HTL, EBL or within the EML. Compared with components from prior art (V1 to V8), the samples comprising the compounds according to the invention exhibit both higher efficiencies and also improved lifetimes both in singlet blue and also in triplet green.

| **Table 1: Structure of the OLEDs** | | | | | | | |
|---|---|---|---|---|---|---|---|
| **Ex.** | **HIL** | **HTL** | **HTL2** | **EBL** | **EML** | **ETL** | **EIL** |
| | Thickness / nm | Thickness / nm | Thickness / nm | Thickness / nm | Thickness / nm | Thickness / nm | Thicknes s / nm |
| E1 | HIM:F4TCNQ(5 %) 20nm | HIM 210nm | HTM2:F4TC NQ(5%) 20nm | HTM2 20nm | H2:TEG(10 %) 30 nm | ETM:LiQ(50%) 40 nm | LiQ 1nm |
| V1 | HIM:F4TCNQ(5 %) 20nm | HIM 210nm | HTMV2:F4T CNQ(5%) 20nm | HTMV2 20nm | H2:TEG(10 %) 30 nm | ETM:LiQ(50%) 40 nm | LiQ 1 nm |
| V2 | HIM:F4TCNQ(5 %) 20nm | HIM 210nm | HTMV5:F4T CNQ(5%) 20nm | HTMV5 20nm | H2:TEG(10 %) 30 nm | ETM:LiQ(50%) 40 nm | LiQ 1nm |

| **Table 2: Data for the OLEDs** | | | |
|---|---|---|---|
| **Ex.** | **U @ 2mA/cm²** | **EQE @ 2mA/cm²** | **LT80 @ 20mA/cm²** |
| | [V] | % | [h] |
| E1 | 3.3 | 19.2 | 190 |
| V1 | 3.6 | 17.5 | 140 |
| V2 | 3.7 | 18.7 | 100 |

| **Table 3: Structure of the OLEDs** | | | | | | | |
|---|---|---|---|---|---|---|---|
| **Ex.** | **HIL** | **HTL** | **HTL2** | **EBL** | **EML** | **ETL** | **EIL** |
| | Thickness / nm | Thickness / nm | Thickness / nm | Thickness / nm | Thickness / nm | Thickness / nm | Thicknes s / nm |
| E2 | HIM:F4TCNQ(5%) | HIM | HTM3:F4TCNQ(5%) | HTM3 | H1:SEB(3%) | ETM:LiQ(40%) | LiQ |
| | 20nm | 155nm | 20nm | 20nm | 20 nm | 30 nm | 1nm |
| E3 | HIM:F4TCNQ(5%) | HIM | HTM5:F4TCNQ(5%) | HTM5 | H1:SEB(3%) | ETM:LiQ(40%) | LiQ |
| | 20nm | 155nm | 20nm | 20nm | 20 nm | 30 nm | 1nm |
| V3 | HIM:F4TCNQ(5%) | HIM | HTMV3:F4TCNQ(5%) | HTMV3 | H1:SEB(3%) | ETM:LiQ(40%) | LiQ |
| | 20nm | 155nm | 20nm | 20nm | 20 nm | 30 nm | 1nm |
| V4 | HIM:F4TCNQ(5%) | HIM | HTMV6:F4TCNQ(5%) | HTMV6 | H1:SEB(3%) | ETM:LiQ(40%) | LiQ |
| | 20nm | 155nm | 20nm | 20nm | 20 nm | 30 nm | 1 nm |

| **Table 4: Data for the OLEDs** | | | |
|---|---|---|---|
| **Ex.** | **U @ 10mA/cm²** | **EQE @ 10mA/cm²** | **LT80 @ 60mA/cm²** |
| | [V] | % | [h] |
| E2 | 3.8 | 7.3 | 440 |
| E3 | 3.8 | 7.2 | 420 |
| V3 | 3.9 | 7.1 | 410 |
| V4 | 3.7 | 7.1 | 370 |

| **Table 5: Structure of the OLEDs** | | | | | | |
|---|---|---|---|---|---|---|
| ***Ex.*** | ***HIL*** | ***HTL*** | ***EBL*** | ***EML*** | ***ETL*** | ***EIL*** |
| | Thickness / nm | Thickness / nm | Thickness / nm | Thickness / nm | Thickness / nm | Thickness / nm |
| E4 | HTM1:F4TCNQ(5%) | HTM1 | EBM1 | H1:SEB(5%) | ETM:LiQ(50%) | LiQ |
| | 20 nm | 180 nm | 10 nm | 20 nm | 30 nm | 1 nm |
| E5 | HTM4:F4TCNQ(5%) | HTM4 | EBM1 | H1:SEB(5%) | ETM:LiQ(50%) | LiQ |
| | 20 nm | 180nm | 10 nm | 20 nm | 30 nm | 1 nm |
| V5 | HTMV1 :F4TCNQ(5%) | HTMV1 | EBM1 | H1:SEB(5%) | ETM:LiQ(50%) | LiQ |
| | 20 nm | 180 nm | 10 nm | 20 nm | 30 nm | 1 nm |
| V6 | HTMV4:F4TCNQ(5%) | HTMV4 | EBM1 | H1:SEB(5%) | ETM:LiQ(50%) | LiQ |
| | 20 nm | 180nm | 10 nm | 20 nm | 30 nm | 1 nm |
| E6 | HTM3:F4TCNQ(5%) | HTM3 | EBM1 | H1:SEB(5%) | ETM:LiQ(50%) | LiQ |
| | 20 nm | 180nm | 10 nm | 20 nm | 30 nm | 1 nm |
| E7 | HTM5:F4TCNQ(5%) | HTM5 | EBM1 | H1:SEB(5%) | ETM:LiQ(50%) | LiQ |
| | 20 nm | 180nm | 10 nm | 20 nm | 30 nm | 1 nm |
| V7 | HTMV3:F4TCNQ(5%) | HTMV3 | EBM1 | H1:SEB(5%) | ETM:LiQ(50%) | LiQ |
| | 20 nm | 180 nm | 10 nm | 20 nm | 30 nm | 1 nm |
| V8 | HTMV6:F4TCNQ(5%) | HTMV6 | EBM1 | H1:SEB(5%) | ETM:LiQ(50%) | LiQ |
| | 20 nm | 180nm | 10 nm | 20 nm | 30 nm | 1 nm |

| **Table 6: Data for the OLEDs** | | | |
|---|---|---|---|
| **Ex.** | **U @ 10mA/cm²** | **EQE @ 10mA/cm²** | **LT80 @ 60mA/cm²** |
| | [V] | % | [h] |
| E4 | 4.1 | 8.2 | 300 |
| E5 | 3.9 | 8.0 | 310 |
| V5 | 4.1 | 7.9 | 250 |
| V6 | 4.3 | 8.1 | 270 |
| E6 | 4.2 | 8.2 | 350 |
| E7 | 4.2 | 8.2 | 340 |
| V7 | 4.9 | 7.2 | 290 |
| V8 | 4.3 | 8.1 | 280 |

| **Table 7 - Structures of the materials used** | | |
|---|---|---|
| 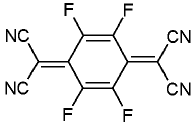 | 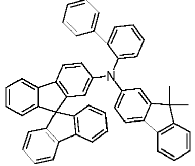 | 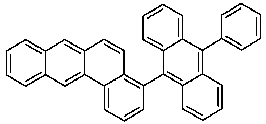 |
| F4TCNQ | HIM | H1 |
| 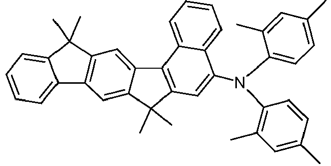 | 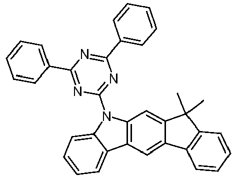 | 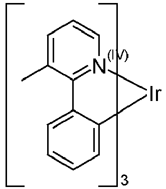 |
| SEB | H2 | TEG |
| 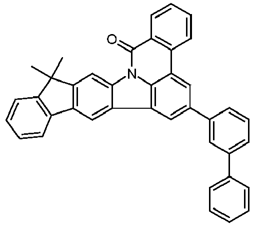 | 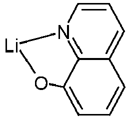 | 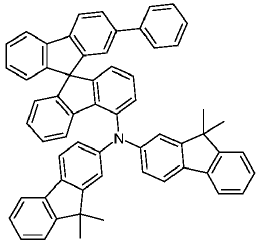 |
| ETM | LiQ | HTM1 |
| 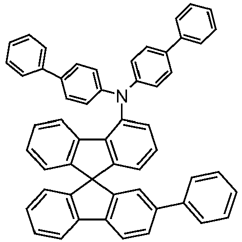 | 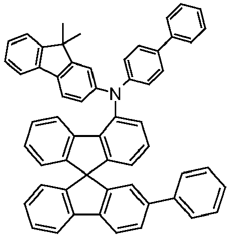 | 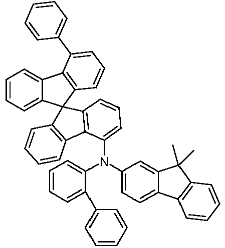 |
| HTM2 | HTM3 | HTM4 |
| 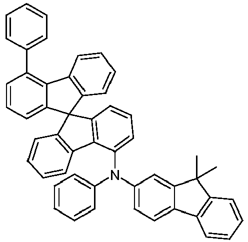 | 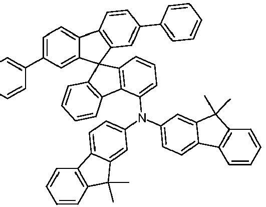 | 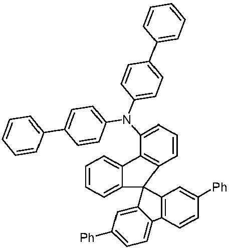 |
| HTM5 | HTMV1 | HTMV2 |
| 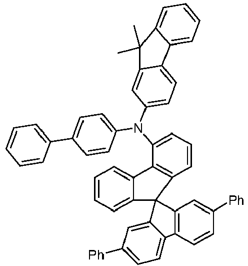 | 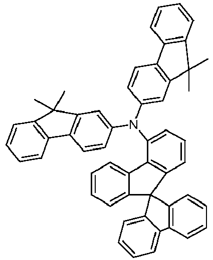 | 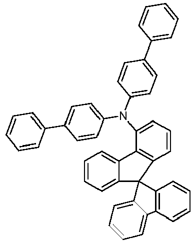 |
| HTMV3 | HTMV4 | HTMV5 |
| 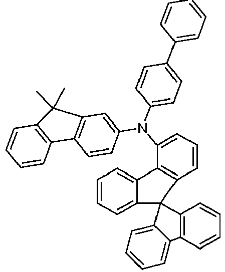 | 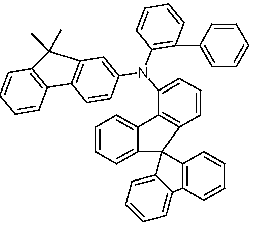 | |
| HTMV6 | EBM1 | |

### Example 1

OLED devices with the structures shown in table 1 are produced. Table 2 shows the performance data of the examples described. The device is a triplet green device with comparison of HTM2, HTMV2 and HTMV5 as material in the electron blocking layer (EBL). It can be shown, that voltage of the device @ 2mA/cm² with inventive material is with 3.3V at least 0.3V lower than the comparative examples. The efficiency of the stack including the same material HTM2 has a higher external quantum efficiency @ 2mA/cm² of 19.2% than both of the comparative examples (17.5% and 18.7%). Even in lifetime the inventive example is much better than the references. The device with HTM2 has a lifetime down to 80% of its initial brightness @ 20mA/cm² constant driving current density of 190h. The two comparative examples achieve 140h and 100h respectively.

### Example 2

OLED devices with a singlet blue emissive layer are produced. The exact structure is defined in table 3. The overall data and trends in this stack is comparable to Example 1. The voltages of all three devices are closer together than in the green stack. Experiment V4 shows a little bit better, V3 a little bit worse voltage at 10mA/cm² than the two inventive examples. The efficiency depicted in column 3 of table 4 shows again the advantage of the inventive materials in experiment E2 and E3, which are at 7.3% and 7.2% instead of 7.1% of the comparative examples. In the blue device the lifetime is measured at a constant current of 60mA/cm². LT80 of the inventive materials are 440h and 420h respectively, whereas the comparative examples only last 410 and 370h.

### Example 3

Finally a third type of OLED stack was produced. This is shown in table 5 with 4 inventive structures as well as 4 comparative examples. The material is used as a doped hole injection material and as hole transport material. Structurewise one needs to compare E4 and E5 as inventive examples against V5 and V6. The inventive examples have the lowest driving voltage @ 10mA/cm² (4.1V and 3.9V vs. 4.1V and 4.3V), the highest external quantum efficiency @ 10mA/cm² (8.2% and 8.0% vs. 7.9% and 8.1%) and the best lifetime for LT80 @60mA/cm² (300h and 310h vs. 250h and 270h). In the second case the comparison needs to be done between inventive materials in E6 and E7 and comparative examples in V7 and V8. The voltage of the device including the inventive materials is 4.2V and 4.2V respectively @ 10mA/cm² driving current density. Comparative materials are at 4.9V and 4.3V. The EQE @ 10mA/cm² is 8.2% and 8.2% for the inventive examples and 7.2% as well as 8.1%, respectively, for the comparative examples. Lifetime is analogous with 350h and 340h for LT80 @ 60mA/cm² for the inventive examples and 290h and 280h for the comparative ones.

## Claims

1. Compound of the formula (1), 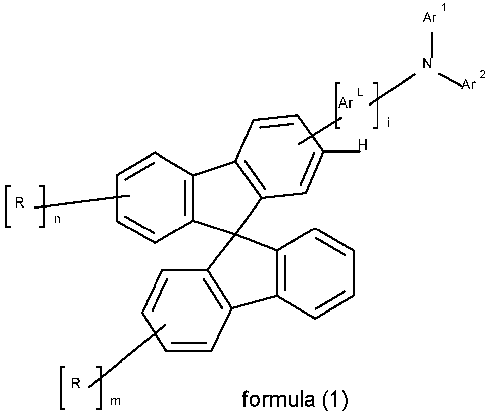 where the following applies to the symbols and indices used:
R is an aromatic ring system selected from phenyl, biphenyl, terphenyl and naphthyl;
Ar¹, Ar² are on each occurrence, identically or differently, an aromatic or heteroaromatic ring system having 5 to 60 aromatic ring atoms, which may in each case also be substituted by one or more radicals R²; Ar¹ and Ar² here may also be connected via a single bond or a divalent bridge selected from -N(R²)-, -O-, -S-, -C(R²)2-, -C(R²)₂-C(R²)₂-, -Si(R²)₂- or -B(R²)-;
Ar^{L} is an aromatic or heteroaromatic ring system having 5 to 40 aromatic ring atoms, which may in each case be substituted by one or more radicals R²;
R² are selected on each occurrence, identically or differently, from the group consisting of H, D, F, Cl, Br, I, CHO, CN, C(=O)Ar³, P(=O)(Ar³)₂, S(=O)Ar³, S(=O)₂Ar³, NO₂, Si(R³)₃, B(OR³)₂, OSO₂R³, straight-chain alkyl, alkoxy or thioalkyl groups having 1 to 40 C atoms or branched or cyclic alkyl, alkoxy or thioalkyl groups having 3 to 40 C atoms, each of which may be substituted by one or more radicals R³, where in each case one or more non-adjacent CH₂ groups may be replaced by R³C=CR³, C=C, Si(R³)₂, Ge(R³)₂, Sn(R³)₂, C=O, C=S, C=Se, P(=O)(R³), SO, SO₂, O, S or CONR³ and where one or more H atoms may be replaced by D, F, Cl, Br, I, CN or NO₂, aromatic or heteroaromatic ring systems having 5 to 60 aromatic ring atoms, which may in each case be substituted by one or more radicals R³, and aryloxy groups having 5 to 60 aromatic ring atoms, which may be substituted by one or more radicals R³, where two or more adjacent substituents R² may optionally form a mono- or polycyclic, aliphatic ring system or aromatic ring system, which may be substituted by one or more radicals R³;
R³ is selected on each occurrence, identically or differently, from the group consisting of H, D, F, Cl, Br, I, CHO, CN, C(=O)Ar³, P(=O)(Ar³)₂, S(=O)Ar³, S(=O)₂Ar³, NO₂, Si(R⁴)₃, B(OR⁴)₂, OSO₂R⁴, straight-chain alkyl, alkoxy or thioalkyl groups having 1 to 40 C atoms or a branched or cyclic alkyl, alkoxy or thioalkyl groups having 3 to 40 C atoms, each of which may be substituted by one or more radicals R⁴, where in each case one or more non-adjacent CH₂ groups may be replaced by R⁴C=CR⁴, C≡C, Si(R⁴)₂, Ge(R⁴)₂, Sn(R⁴)₂, C=O, C=S, C=Se, P(=O)(R⁴), SO, SO₂, O, S or CONR⁴ and where one or more H atoms may be replaced by D, F, Cl, Br, I, CN or NO₂, aromatic or heteroaromatic ring systems having 5 to 60 aromatic ring atoms, which may in each case be substituted by one or more radicals R⁴, and aryloxy groups having 5 to 60 aromatic ring atoms, which may be substituted by one or more radicals R⁴, where two or more adjacent substituents R³ may optionally form a mono- or polycyclic, aliphatic ring system or aromatic ring system, which may be substituted by one or more radicals R⁴;
R⁴ is selected on each occurrence, identically or differently, from the group consisting of H, D, F, Cl, Br, I, CN, straight-chain alkyl, alkoxy or thioalkyl groups having 1 to 20 C atoms or branched or cyclic alkyl, alkoxy or thioalkyl groups having 3 to 20 C atoms, where in each case one or more non-adjacent CH₂ groups may be replaced by SO, SO₂, O or S and where one or more H atoms may be replaced by D, F, Cl, Br or I, and aromatic or heteroaromatic ring systems having 5 to 24 C atoms;
Ar³ is an aromatic or heteroaromatic ring system having 5 to 24 aromatic ring atoms, which may in each case be substituted by one or more radicals R⁴;
i is 0 or 1 ;
m, n are, identically or differently, 0, or 1;
and m + n = 1,
where the following compounds are excluded:

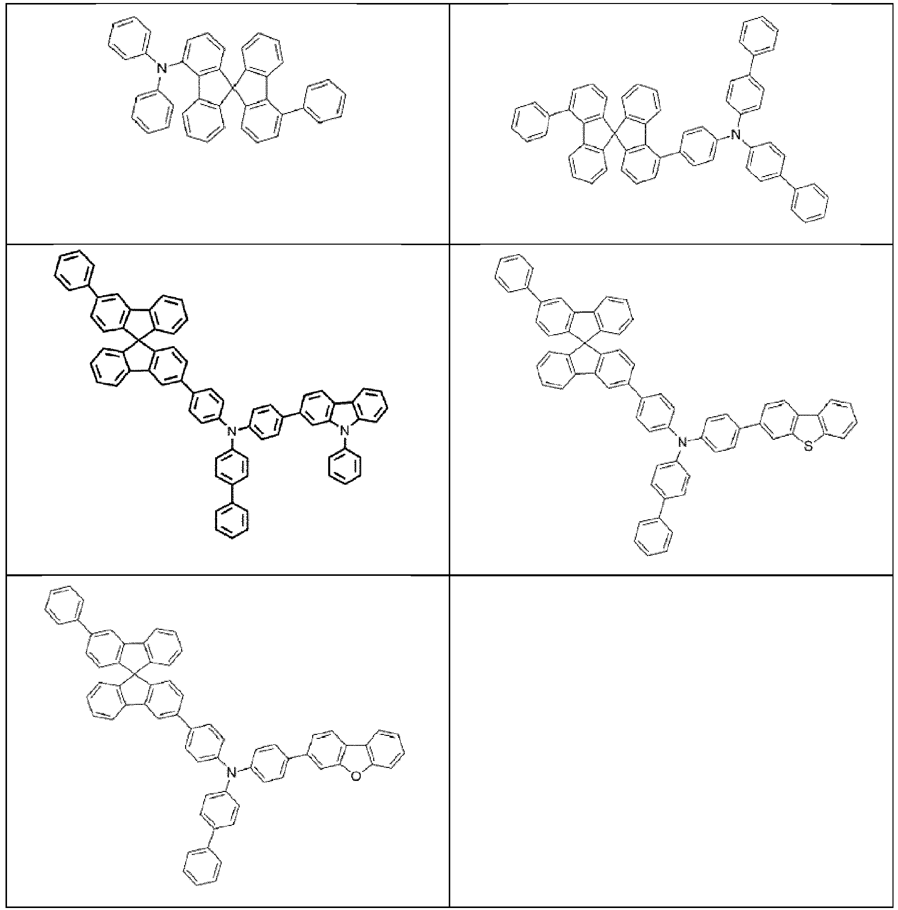

2. Compound according to claim 1, **characterised in that** m = 1 and n = 0.

3. Compound according to Claim 1 or 2, **characterized in that** the groups Ar¹ and Ar² are selected from the groups of formulae (A-1) to (A-48), 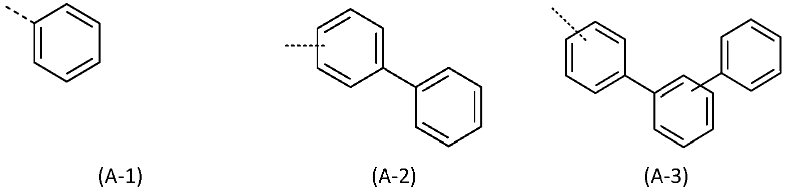 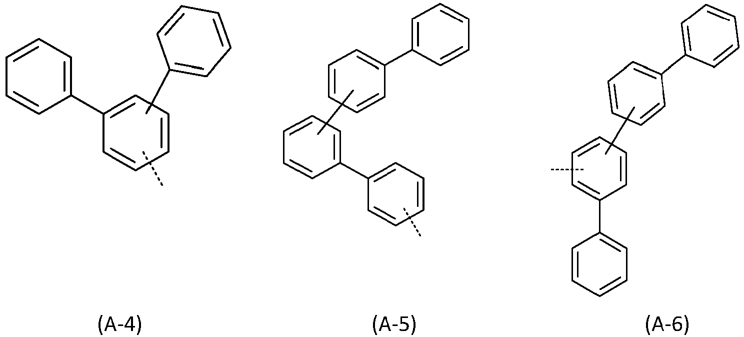 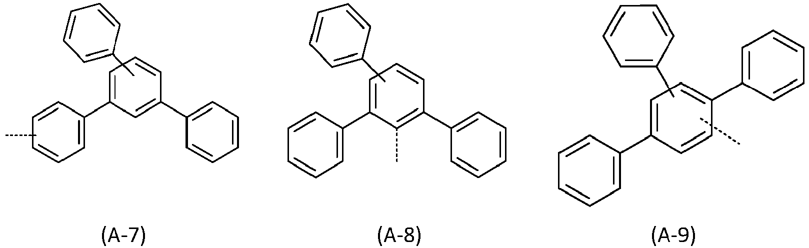 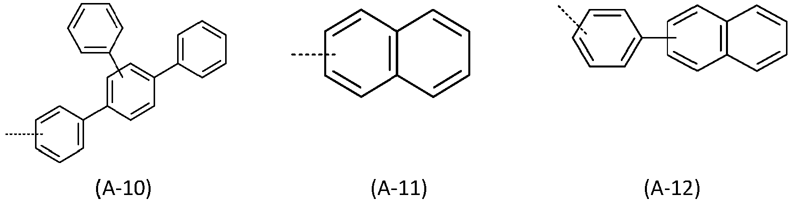 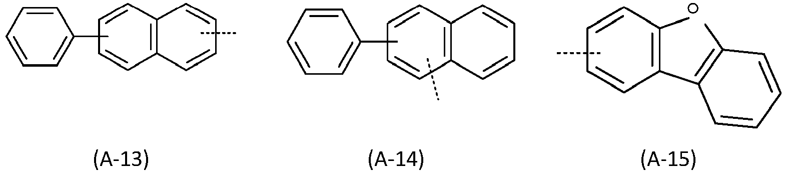 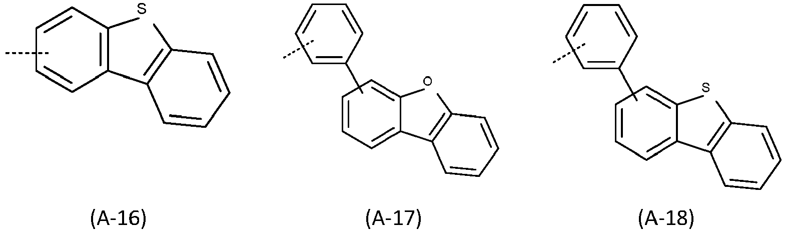 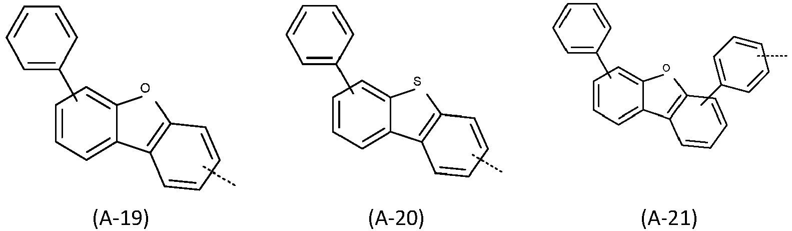 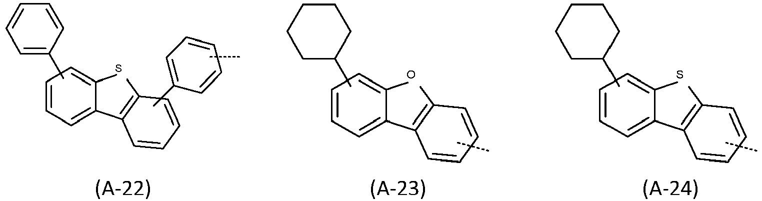 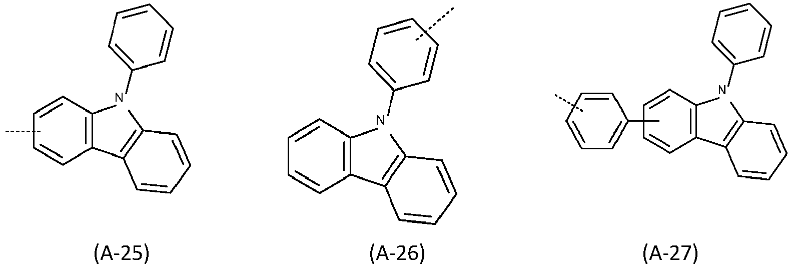 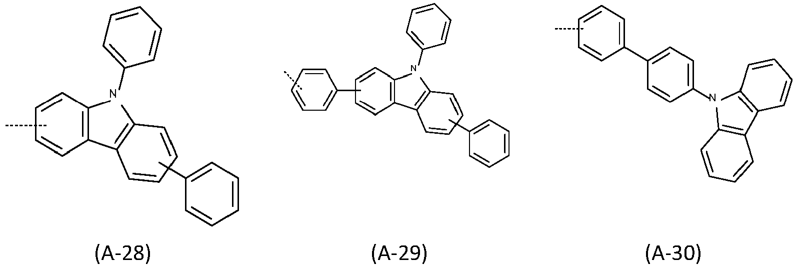 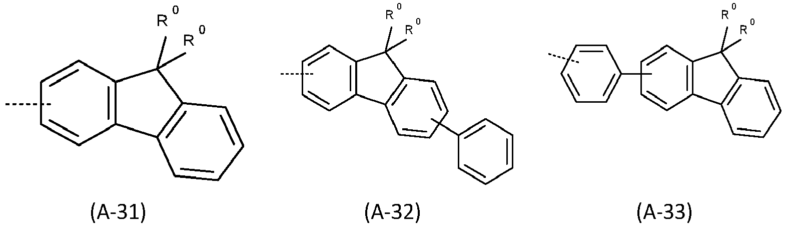 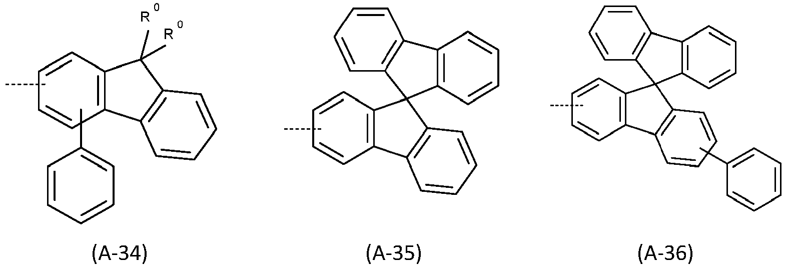 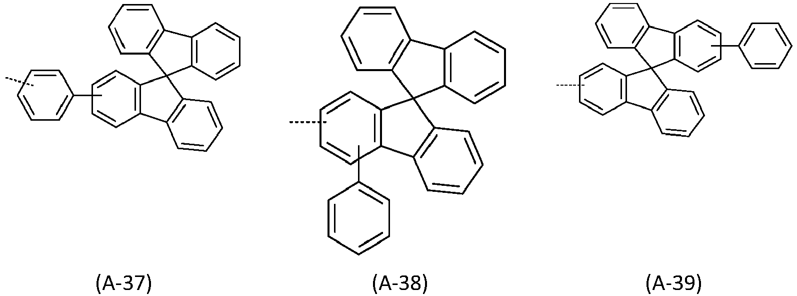 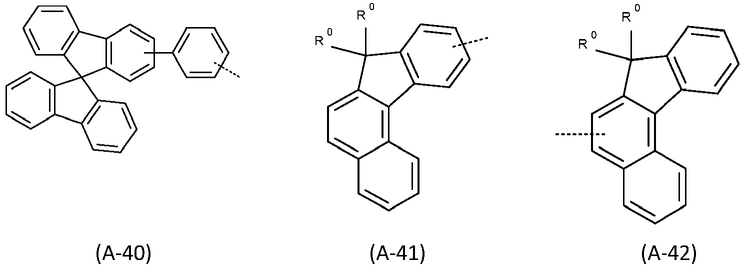 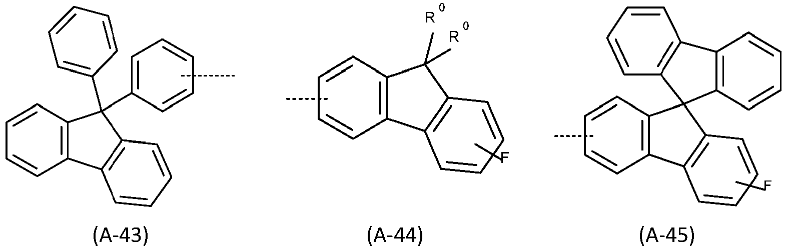 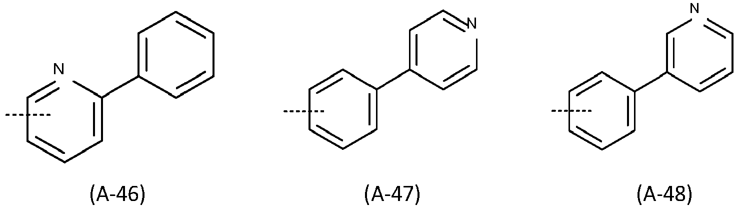 where the dashed bonds indicate the bonds to the nitrogen atom as depicted in formula (1),
where the groups of formulae (A-1) to (A-48) may further be substituted at each free position by a group R² as defined in claim 1, and
where the groups R⁰, in formulae (A-31) to (A-34), (A-41), (A-42) and (A-44), are selected on each occurrence, identically or differently, from the group consisting of H, D, F, CN, Si(R³)₃, straight-chain alkyl groups having 1 to 10 C atoms or branched or cyclic alkyl groups having 3 to 10 C atoms, each of which may be substituted by one or more radicals R³, and aryl or heteroaryl groups having 5 to 18 aromatic ring atoms, which may in each case be substituted by one or more radicals R³, where two adjacent substituents R⁰ may optionally form a mono- or polycyclic, aliphatic ring system or aromatic ring system, which may be substituted by one or more radicals R³.

4. Compound according to one or more of the preceding claims, **characterised in that** Ar^{L} is selected from aromatic or heteroaromatic ring systems having 5 to 18 aromatic ring atoms, which may in each case be substituted by one or more radicals R².

5. Compound according to one or more of the preceding claims, **characterised in that** R² are selected, identically or differently on each occurrence, from the group consisting of H, D, F, CN, straight-chain alkyl or alkoxy groups having 1 to 10 C atoms or branched or cyclic alkyl or alkoxy groups having 3 to 10 C atoms, each of which may be substituted by one or more radicals R³, where one or more non-adjacent CH₂ groups may be replaced by O and where one or more H atoms may be replaced by F, and aromatic or heteroaromatic ring systems having 5 to 24 aromatic ring atoms, which may in each case be substituted by one or more radicals R³.

6. Compound according to one or more of the preceding claims, selected from the compounds of the formulae (1-1) to (1-24) and (I-31) to (I-54), 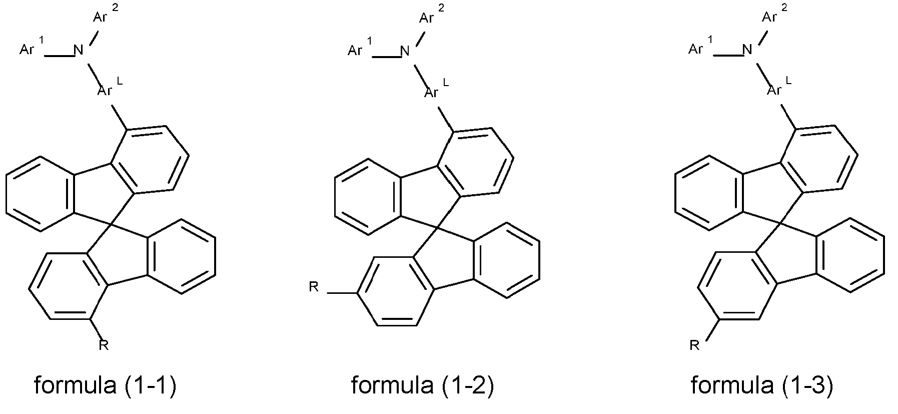 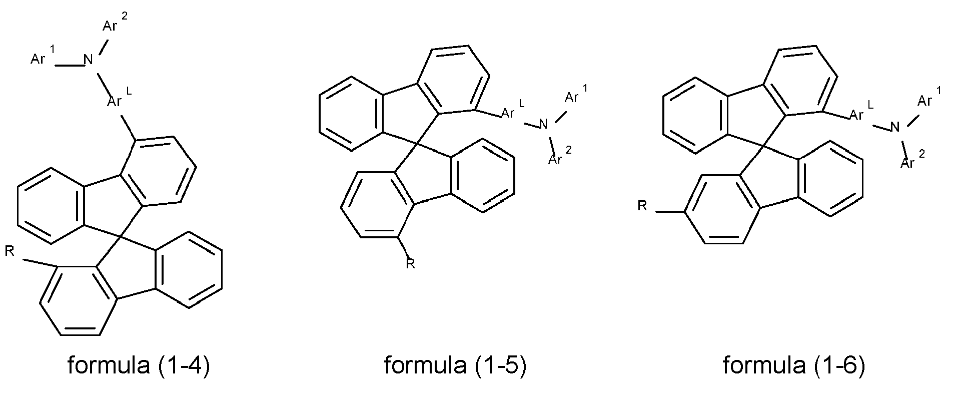 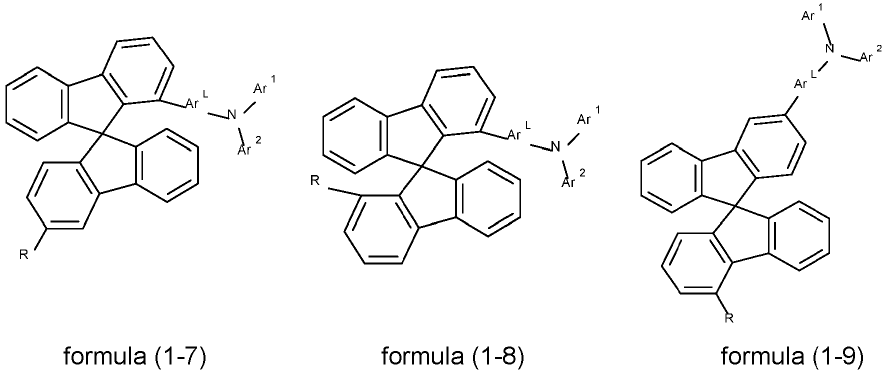 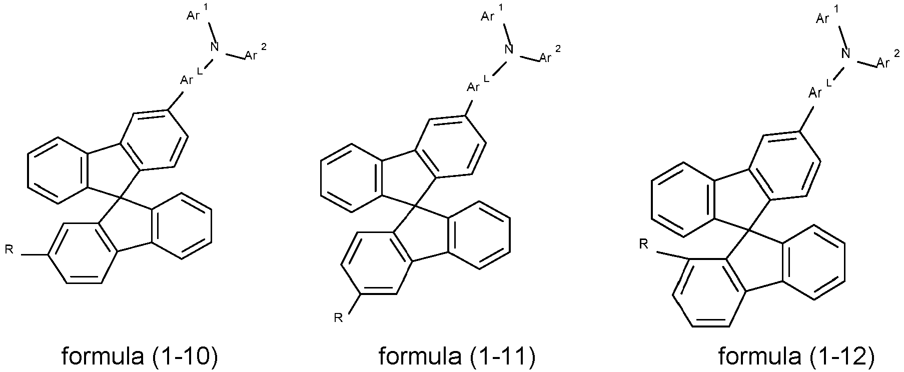 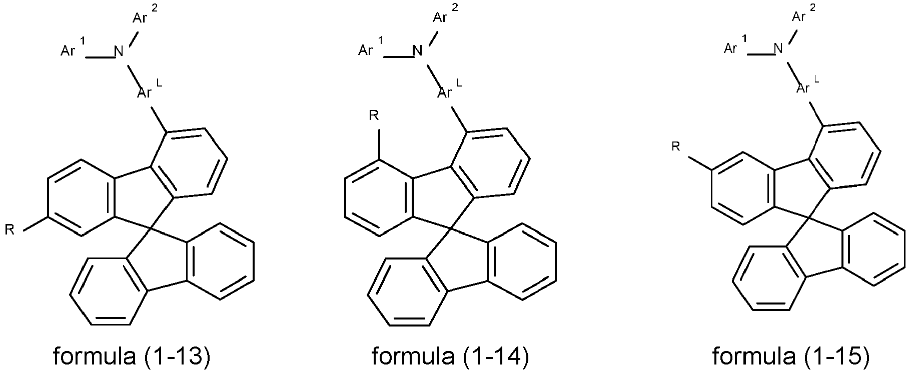 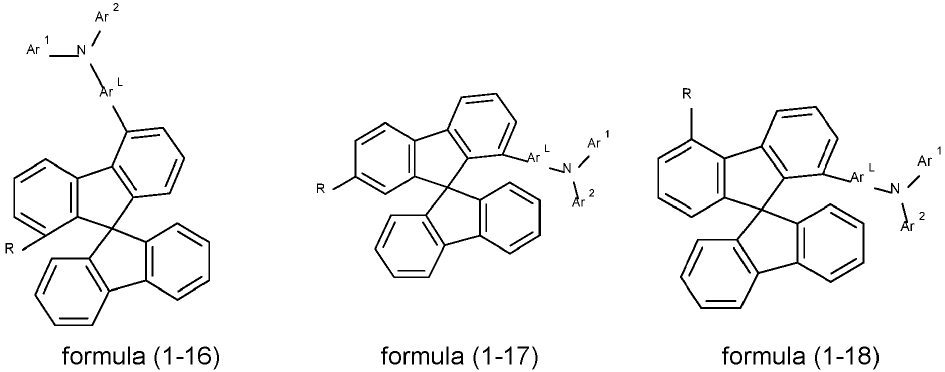 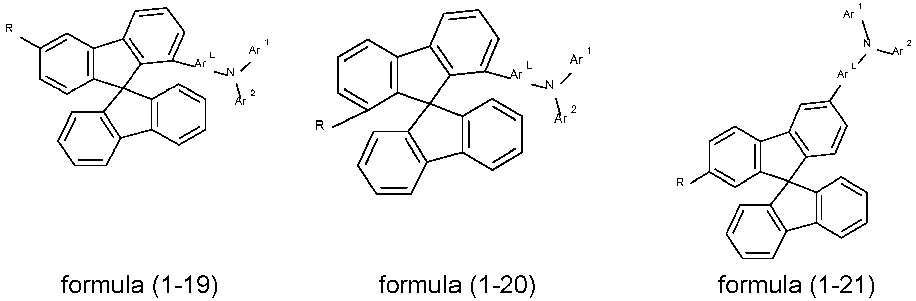 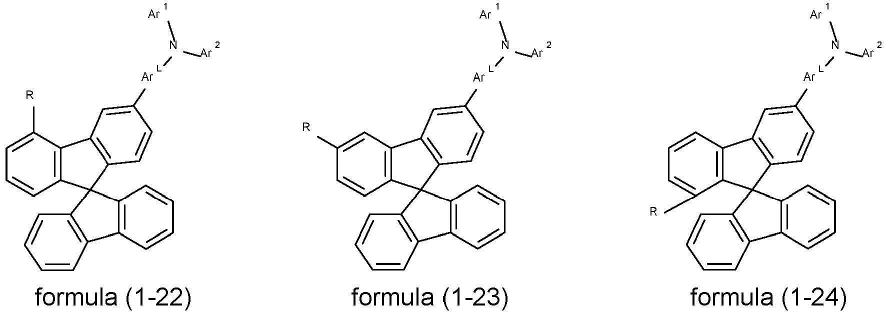 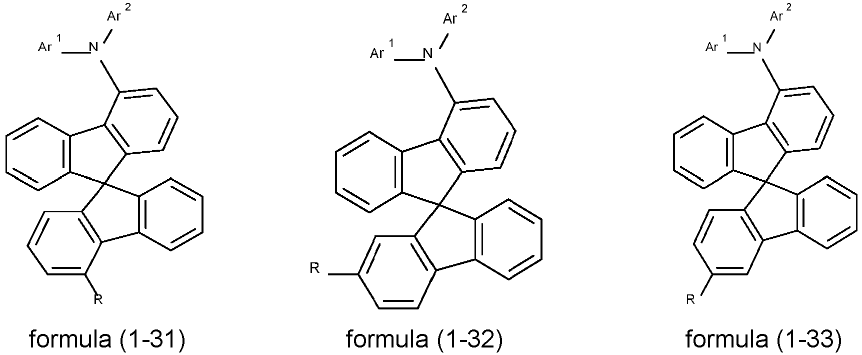 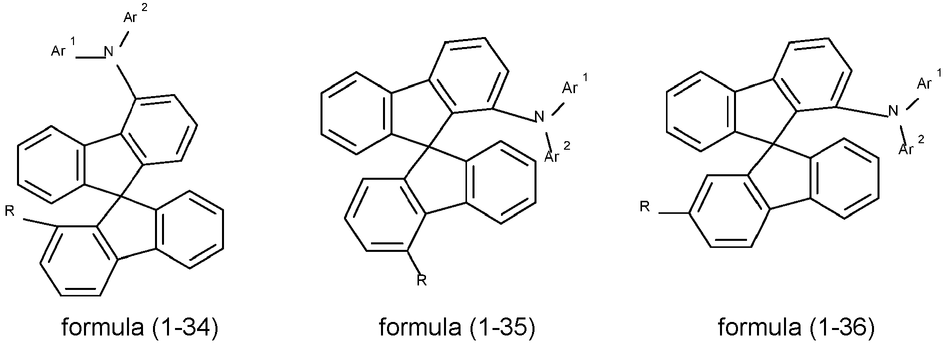 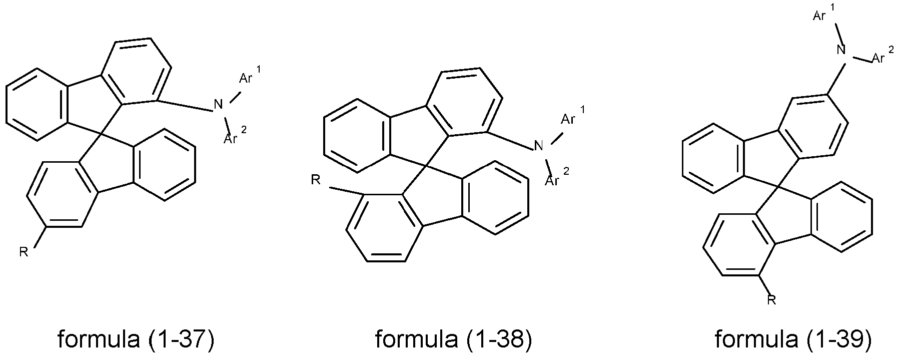 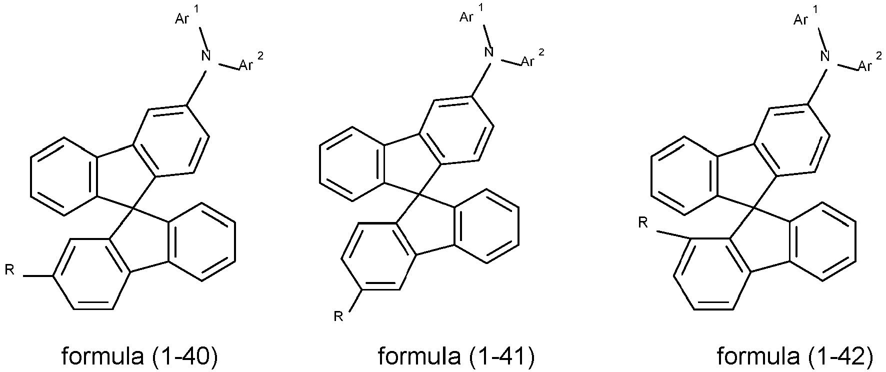 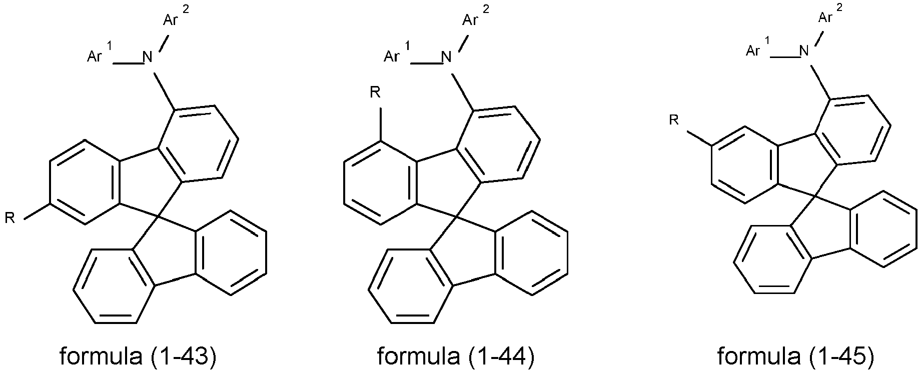 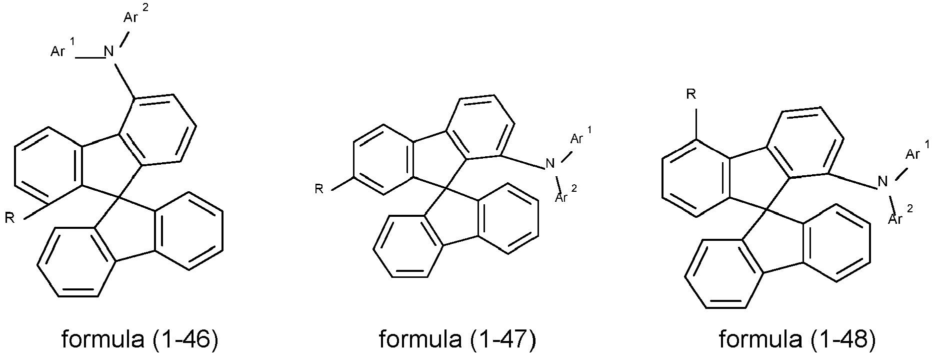 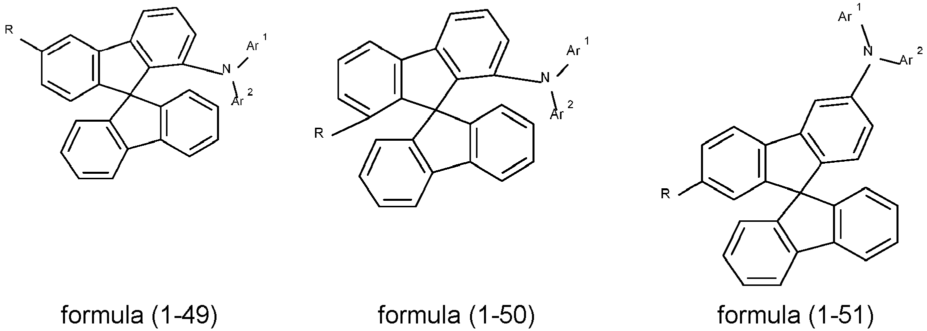 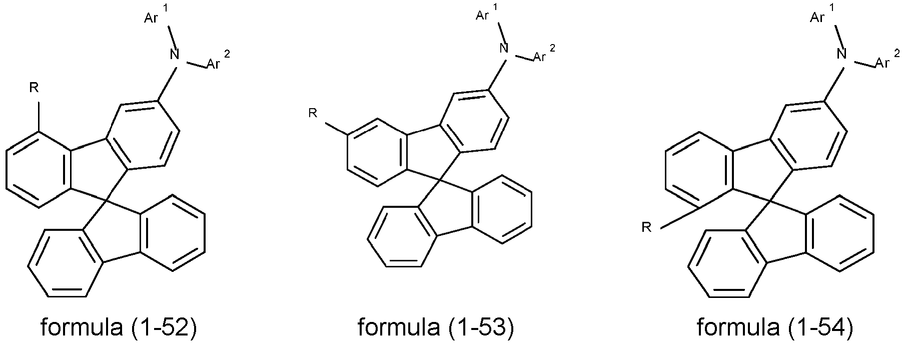 where the symbols and indices used have the meaning as given in claim 1.

7. Process for the preparation of a compound according to one or more of claims 1 to 6, where index i=0, **characterised in that** a diarylamino group is introduced by a C-N coupling reaction between a diarylamine compound and a halogenated spirobifluorene substituted by a group R as defined in claim 1.

8. Formulation comprising at least one compound, according to one or more of claims 1 to 6, and at least one solvent.

9. Use of a compound according to one or more of claims 1 to 6 in an electronic device, in particular in an organic electroluminescent device.

10. Electronic device comprising at least one compound according to one or more of claims 1 to 6, preferably selected from the group consisting of organic electroluminescent devices, organic integrated circuits, organic field-effect transistors, organic thin-film transistors, organic light-emitting transistors, organic solar cells, dye-sensitised organic solar cells, organic optical detectors, organic photoreceptors, organic field-quench devices, light-emitting electrochemical cells, organic laser diodes and organic plasmon emitting devices.

11. Electronic device according to claim 10, which is an organic electroluminescent device, **characterised in that** the compound according to one or more of claims 1 to 6 is employed as hole-transport material in a hole-transport or hole-injection or exciton-blocking or electron-blocking layer or as matrix material for fluorescent or phosphorescent emitters.

## Patentansprüche

1. Verbindung der Formel (1), 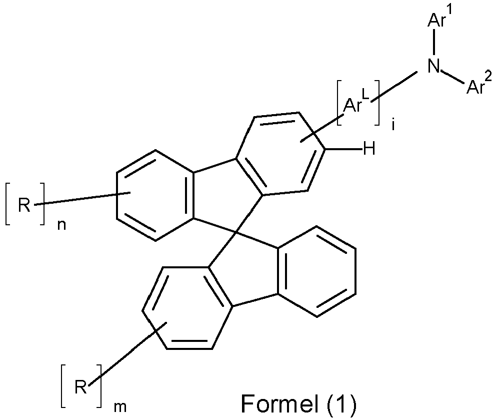 wobei für die verwendeten Symbole und Indizes Folgendes gilt:
R ist ein aus Phenyl, Biphenyl, Terphenyl und Naphthyl ausgewähltes aromatisches Ringsystem;
Ar¹, Ar² sind bei jedem Auftreten gleich oder verschieden ein aromatisches oder heteroaromatisches Ringsystem mit 5 bis 60 aromatischen Ringatomen, das jeweils auch durch einen oder mehrere Reste R² substituiert sein kann; dabei können Ar¹ und Ar² auch über eine Einfachbindung oder eine aus -N(R²)-, -O-, -S-, -C(R²)₂-, -C(R²)₂-C(R²)₂-, -Si(R²)₂- oder -B(R²)- ausgewählte zweiwertige Brücke verbunden sein;
Ar^{L} ist ein aromatisches oder heteroaromatisches Ringsystem mit 5 bis 40 aromatischen Ringatomen, das jeweils durch einen oder mehrere Reste R² substituiert sein kann;
R² sind bei jedem Auftreten gleich oder verschieden ausgewählt aus der Gruppe bestehend aus H, D, F, Cl, Br, I, CHO, CN, C(=O)Ar³, P(=O)(Ar³)₂, S(=O)Ar³, S(=O)₂Ar³, NO², Si(R³)₃, B(OR³)₂, OSO₂R³, geradkettigen Alkyl-, Alkoxy- oder Thioalkylgruppen mit 1 bis 40 C-Atomen oder verzweigten oder cyclischen Alkyl-, Alkoxy- oder Thioalkylgruppen mit 3 bis 40 C-Atomen, die jeweils durch einen oder mehrere Reste R³ substituiert sein können, wobei jeweils eine oder mehrere nicht benachbarte CH₂-Gruppen durch R³C=CR³, C≡C, Si(R³)₂, Ge(R³)₂, Sn(R³)₂, C=O, C=S, C=Se, P(=O)(R³), SO, SO₂, O, S oder CONR³ ersetzt sein können und wobei ein oder mehrere H-Atome durch D, F, Cl, Br, I, CN oder NO₂ ersetzt sein können, aromatischen oder heteroaromatischen Ringsystemen mit 5 bis 60 aromatischen Ringatomen, die jeweils durch einen oder mehrere Reste R³ substituiert sein können, und Aryloxygruppen mit 5 bis 60 aromatischen Ringatomen, die durch einen oder mehrere Reste R³ substituiert sein können, wobei zwei oder mehr benachbarte Substituenten R² gegebenenfalls ein mono- oder polycyclisches, aliphatisches Ringsystem oder aromatisches Ringsystem bilden können, das durch einen oder mehrere Reste R³ substituiert sein kann;
R³ ist bei jedem Auftreten gleich oder verschieden ausgewählt aus der Gruppe bestehend aus H, D, F, Cl, Br, I, CHO, CN, C(=O)Ar³, P(=O)(Ar³)₂, S(=O)Ar³, S(=O)₂Ar³, NO², Si(R⁴)₃, B(OR⁴)₂, OSO₂R⁴, geradkettigen Alkyl-,
Alkoxy- oder Thioalkylgruppen mit 1 bis 40 C-Atomen oder verzweigten oder cyclischen Alkyl-, Alkoxy- oder Thioalkylgruppen mit 3 bis 40 C-Atomen, die jeweils durch einen oder mehrere Reste R⁴ substituiert sein können, wobei jeweils eine oder mehrere nicht benachbarte CH₂-Gruppen durch R⁴C=CR⁴, C≡C, Si(R⁴)₂, Ge(R⁴)₂, Sn(R⁴)₂, C=O, C=S, C=Se, P(=O)(R⁴), SO, SO₂, O, S oder CONR⁴ ersetzt sein können und wobei ein oder mehrere H-Atome durch D, F, Cl, Br, I, CN oder NO₂ ersetzt sein können, aromatischen oder heteroaromatischen Ringsystemen mit 5 bis 60 aromatischen Ringatomen, die jeweils durch einen oder mehrere Reste R⁴ substituiert sein können, und Aryloxygruppen mit 5 bis 60 aromatischen Ringatomen, die durch einen oder mehrere Reste R⁴ substituiert sein können, wobei zwei oder mehr benachbarte Substituenten R³ gegebenenfalls ein mono- oder polycyclisches, aliphatisches Ringsystem oder aromatisches Ringsystem bilden können, das durch einen oder mehrere Reste R⁴ substituiert sein kann;
R⁴ ist bei jedem Auftreten gleich oder verschieden ausgewählt aus der Gruppe bestehend aus H, D, F, Cl, Br, I, CN, geradkettigen Alkyl-, Alkoxy- oder Thioalkylgruppen mit 1 bis 20 C-Atomen oder verzweigten oder cyclischen Alkyl-, Alkoxy- oder Thioalkylgruppen mit 3 bis 20 C-Atomen, wobei jeweils eine oder mehrere nicht benachbarte CH₂-Gruppen durch SO, SO₂, O oder S ersetzt sein können und wobei ein oder mehrere H-Atome durch D, F, Cl, Br oder I ersetzt sein können, und aromatischen oder heteroaromatischen Ringsystemen mit 5 bis 24 C-Ringatomen;
Ar³ ist ein aromatisches oder heteroaromatisches Ringsystem mit 5 bis 24 aromatischen Ringatomen, das jeweils durch einen oder mehrere Reste R⁴ substituiert sein kann;
i ist 0 oder 1;
m, n sind gleich oder verschieden 0 oder 1;
und m + n = 1,
wobei die folgenden Verbindungen ausgeschlossen sind:

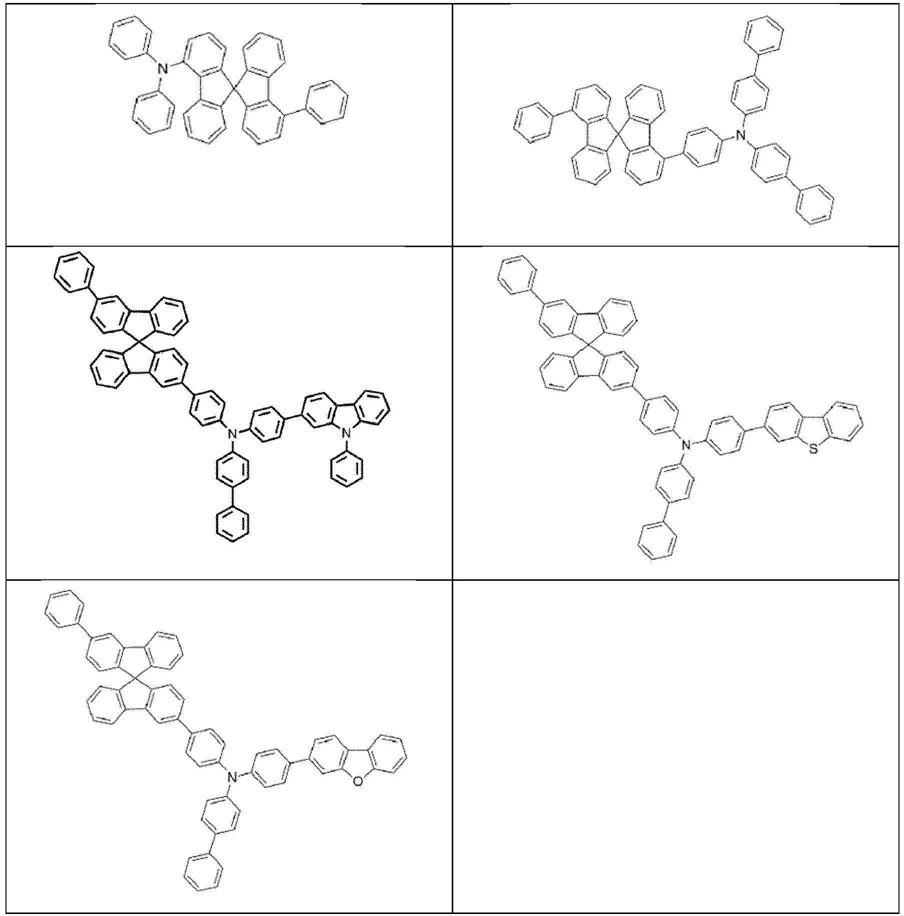

2. Verbindung nach Anspruch 1, **dadurch gekennzeichnet, dass** m = 1 und n=0.

3. Verbindung nach Anspruch 1 oder 2, **dadurch gekennzeichnet, dass** die Gruppen Ar¹ und Ar² ausgewählt sind aus den Gruppen der Formeln (A-1) bis (A-48), 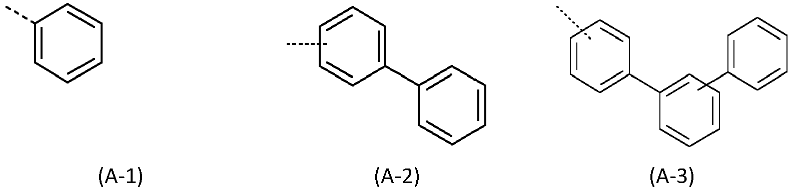 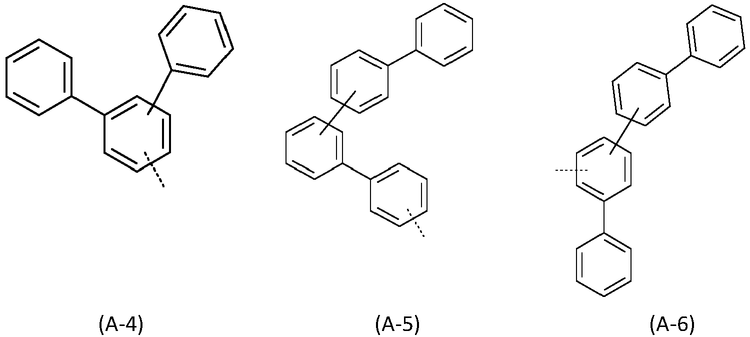 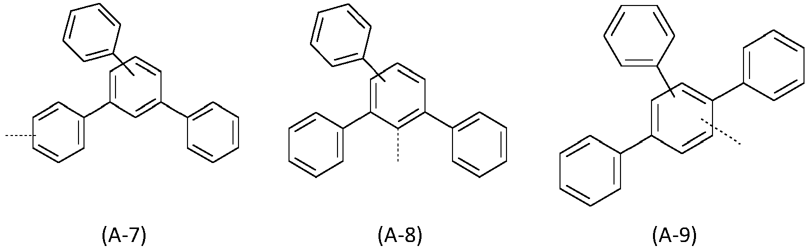 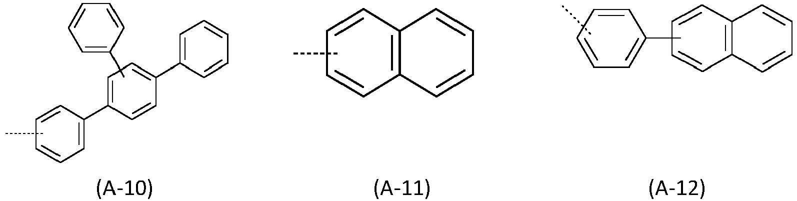 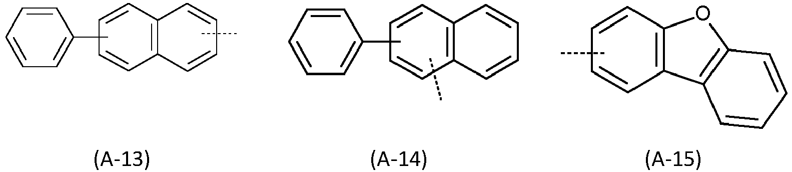 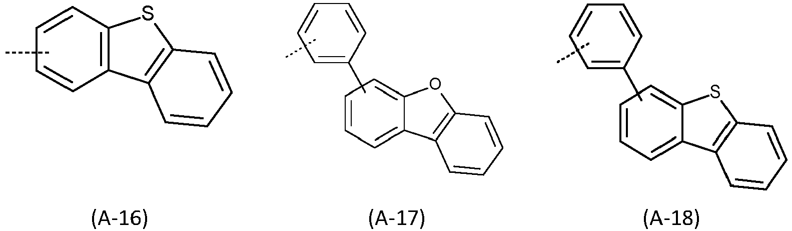 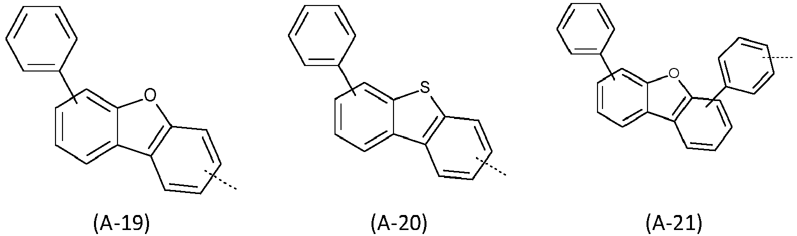 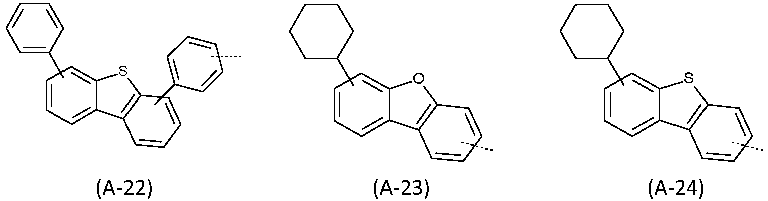 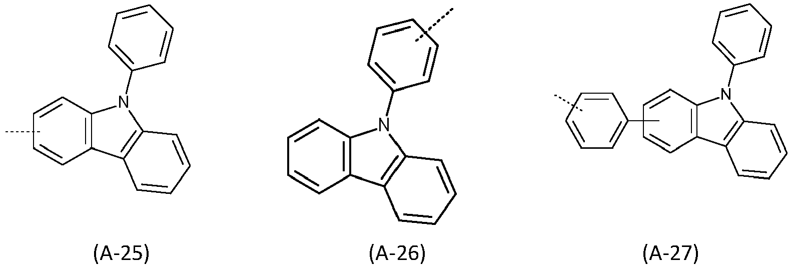 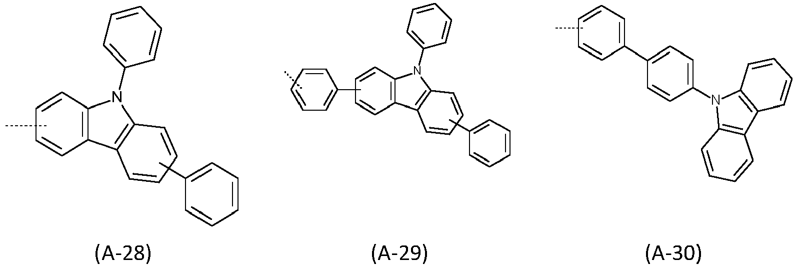 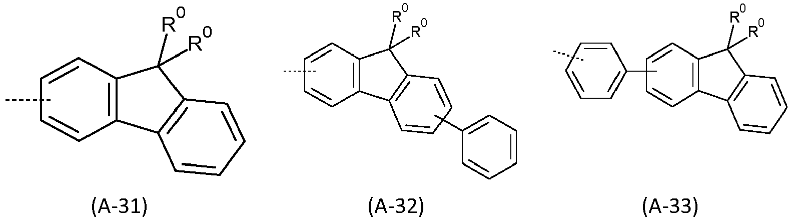 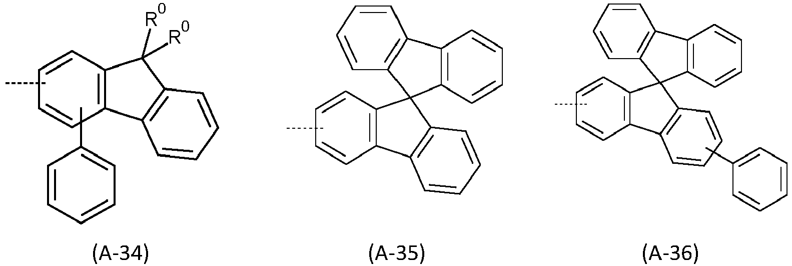 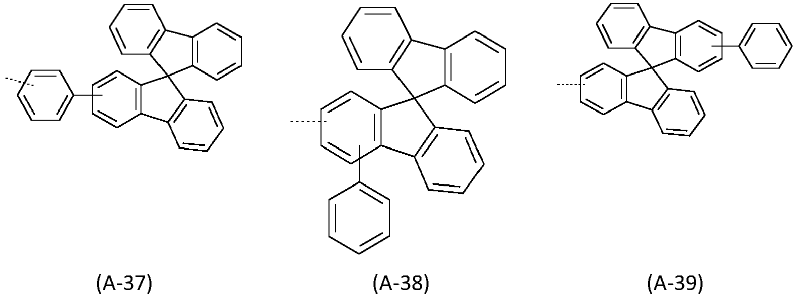 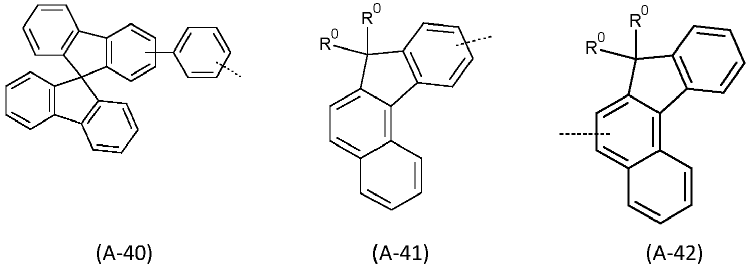 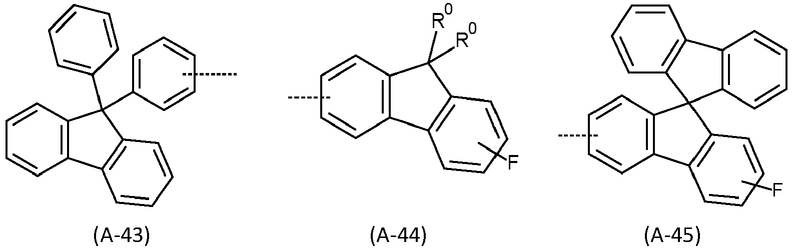 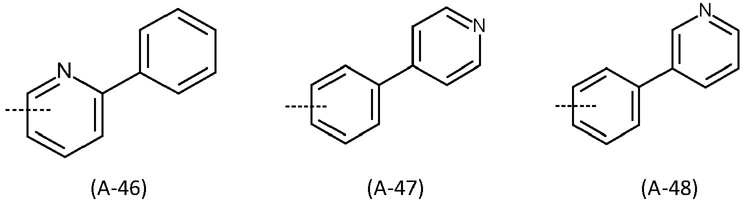 wobei die gestrichelten Bindungen die Bindungen an das Stickstoffatom wie in Formel (1) dargestellt angeben,
wobei die Gruppen der Formeln (A-1) bis (A-48) in jeder freien Position weiter durch eine Gruppe R² wie in Anspruch 1 definiert substituiert sein können und
wobei die Gruppen R⁰, in den Formeln (A-31) bis (A-34), (A-41), (A-42) und (A-44), bei jedem Auftreten gleich oder verschieden ausgewählt sind aus der Gruppe bestehend aus H, D, F, CN, Si(R³)₃, geradkettigen Alkylgruppen mit 1 bis 10 C-Atomen oder verzweigten oder cyclischen Alkylgruppen mit 3 bis 10 C-Atomen, die jeweils durch einen oder mehrere Reste R³ substituiert sein können, und Aryl- oder Heteroarylgruppen mit 5 bis 18 aromatischen Ringatomen, die jeweils durch einen oder mehrere Reste R³ substituiert sein können, wobei zwei benachbarte Substituenten R⁰ gegebenenfalls ein mono- oder polycyclisches, aliphatisches Ringsystem oder aromatisches Ringsystem bilden können, das durch einen oder mehrere Reste R³ substituiert sein kann.

4. Verbindung nach einem oder mehreren der vorhergehenden Ansprüche, **dadurch gekennzeichnet, dass** Ar^{L} aus aromatischen oder heteroaromatischen Ringsystemen mit 5 bis 18 aromatischen Ringatomen ausgewählt ist, die jeweils durch einen oder mehrere Reste R² substituiert sein können.

5. Verbindung nach einem oder mehreren der vorhergehenden Ansprüche, **dadurch gekennzeichnet, dass** R² gleich oder verschieden bei jedem Auftreten ausgewählt sind aus der Gruppe bestehend aus H, D, F, CN, geradkettigen Alkyl- oder Alkoxygruppen mit 1 bis 10 C-Atomen oder verzweigten oder cyclischen Alkyl- oder Alkoxygruppen mit 3 bis 10 C-Atomen, die jeweils durch einen oder mehrere Reste R³ substituiert sein können, wobei eine oder mehrere nicht benachbarte CH₂-Gruppen durch O ersetzt sein können und wobei ein oder mehrere H-Atome durch F ersetzt sein können, und aromatischen oder heteroaromatischen Ringsystemen mit 5 bis 24 aromatischen Ringatomen, die jeweils durch einen oder mehrere Reste R³ substituiert sein können.

6. Verbindung nach einem oder mehreren der vorhergehenden Ansprüche, ausgewählt aus den Verbindungen der Formeln (1-1) bis (1-24) und (1-31) bis (1-54), 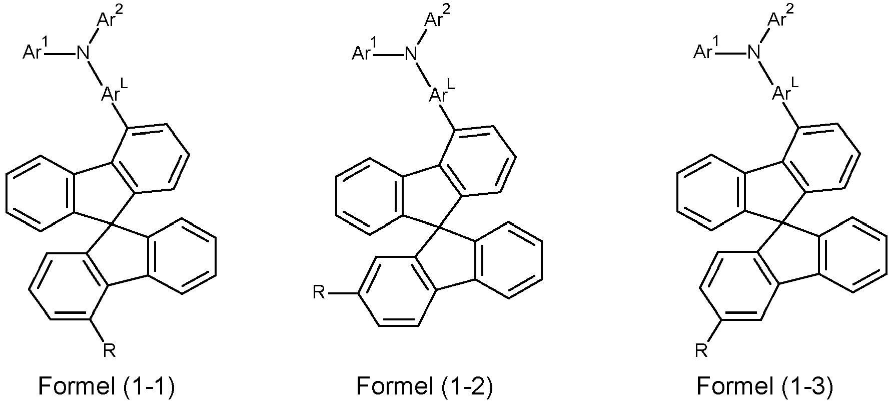 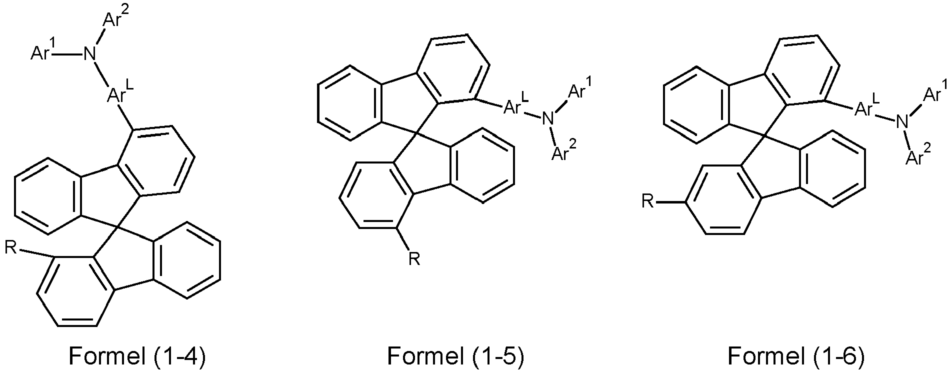 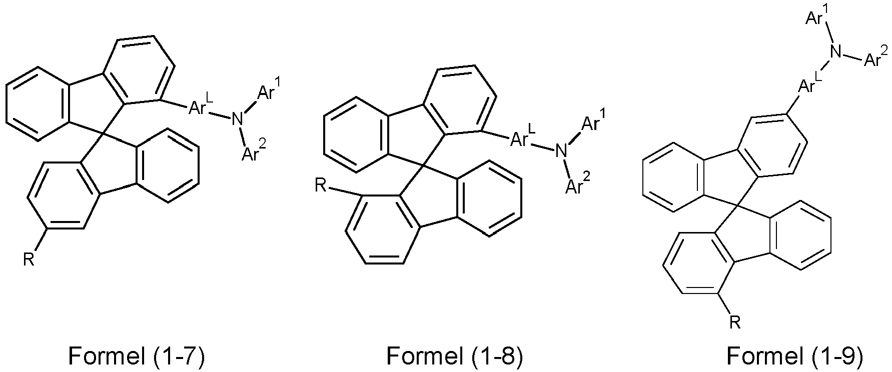 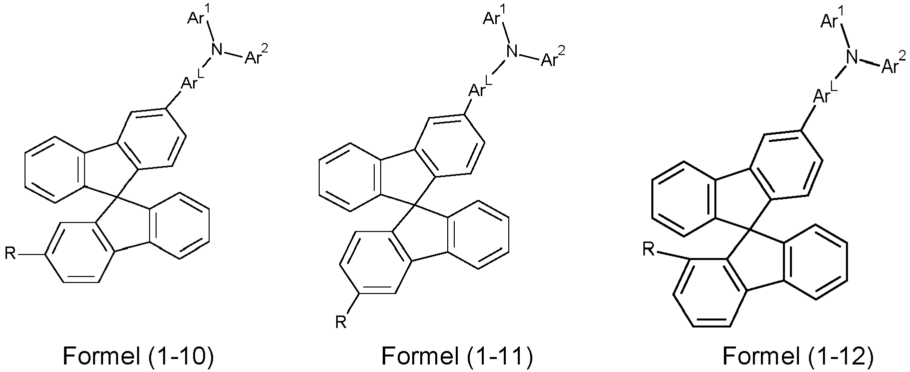 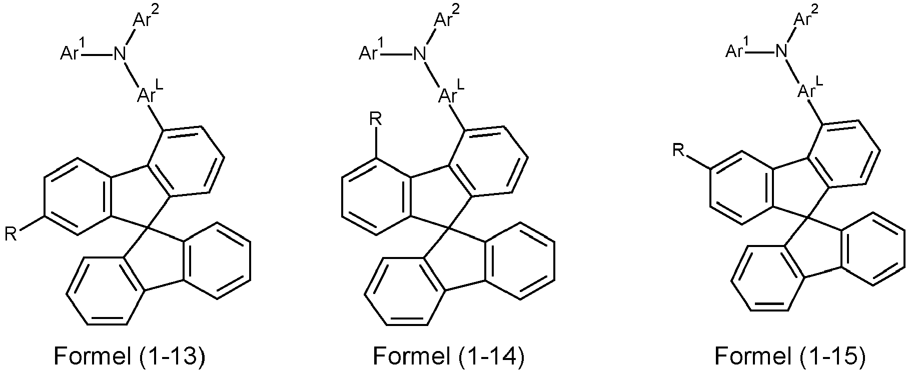 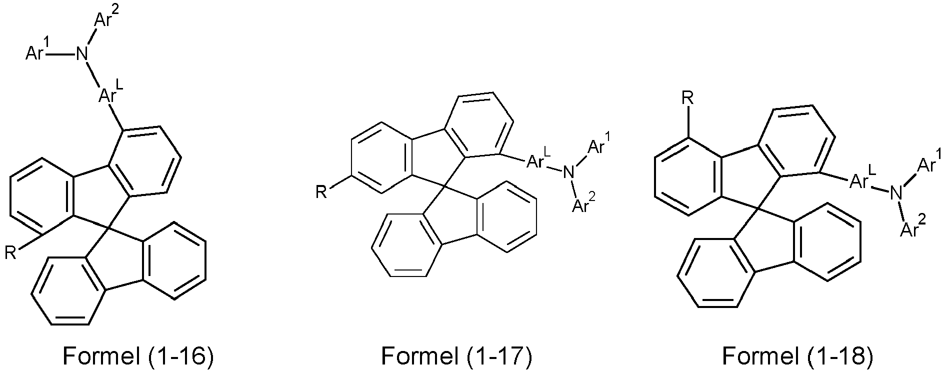 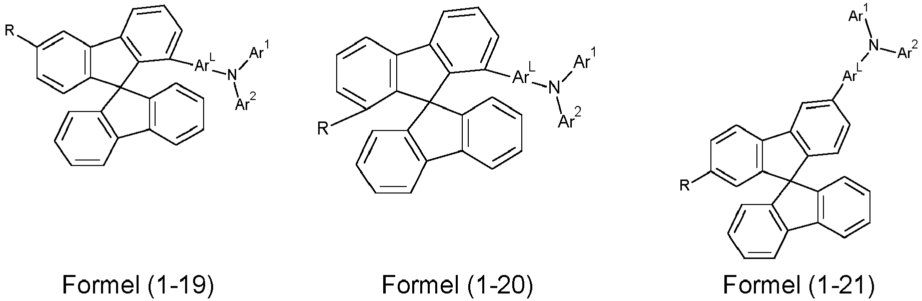 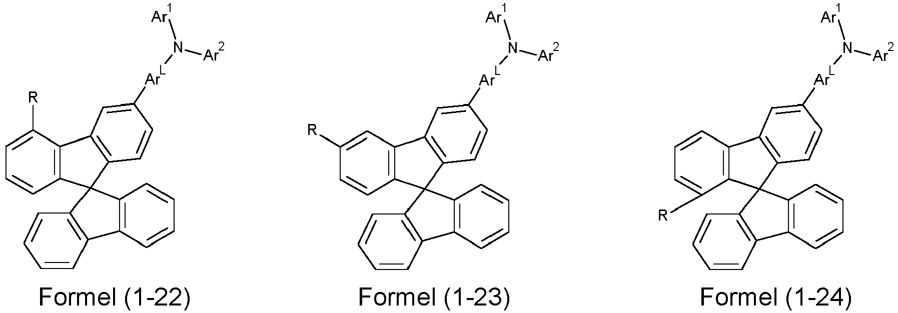 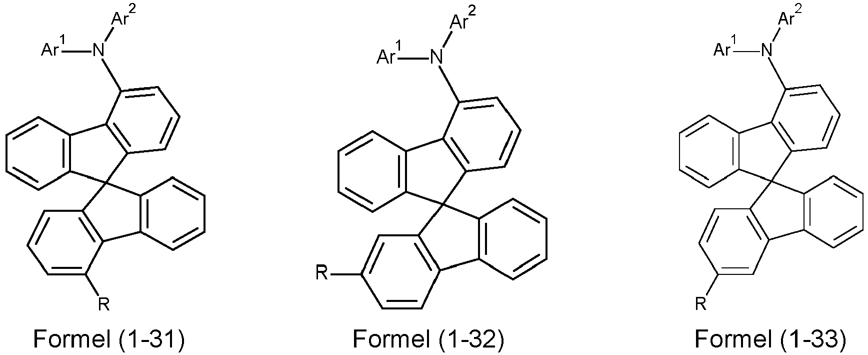 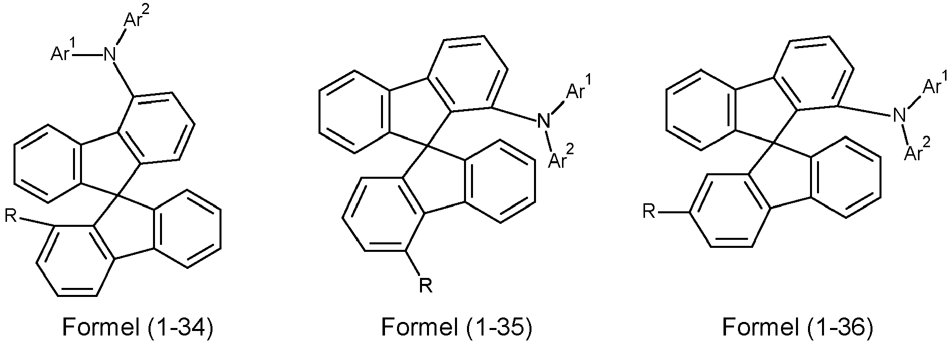 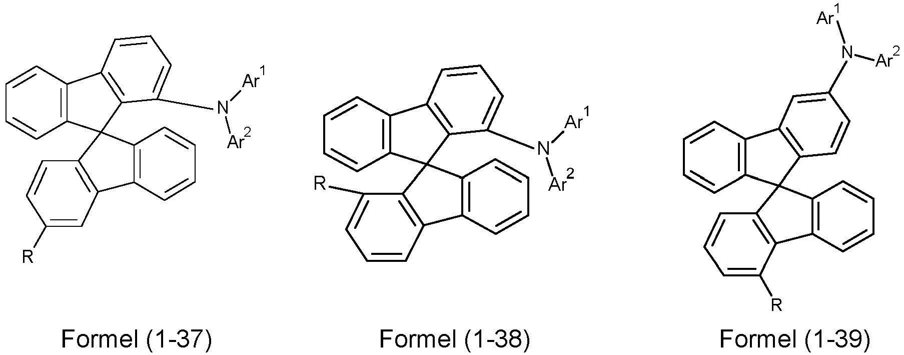 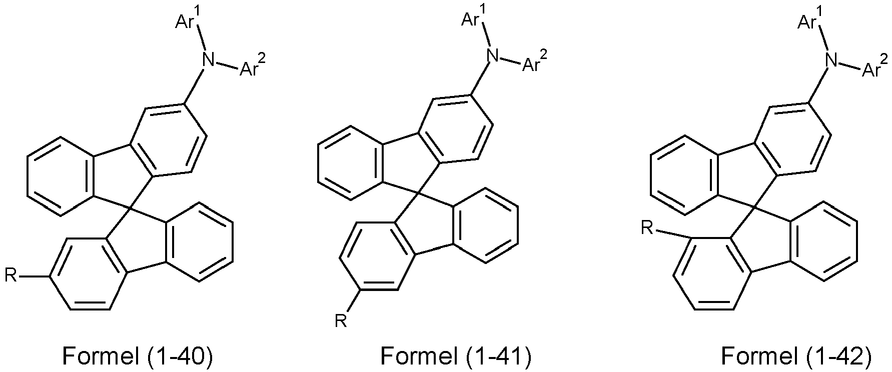 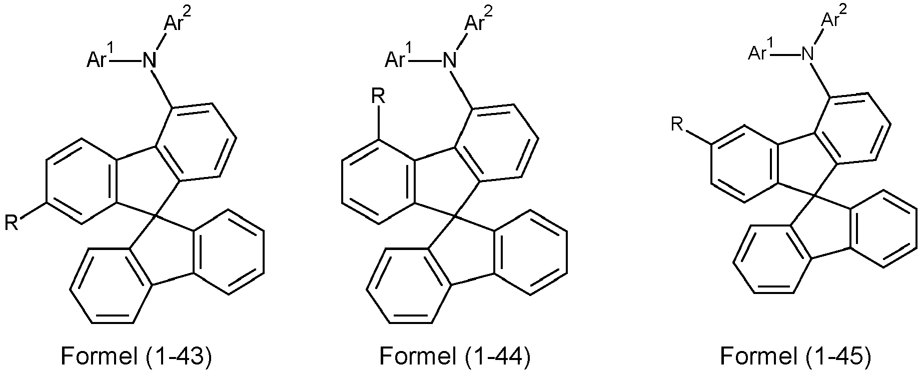 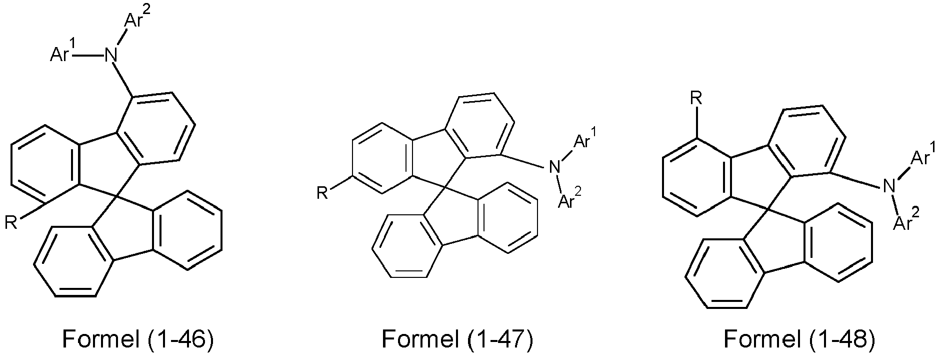 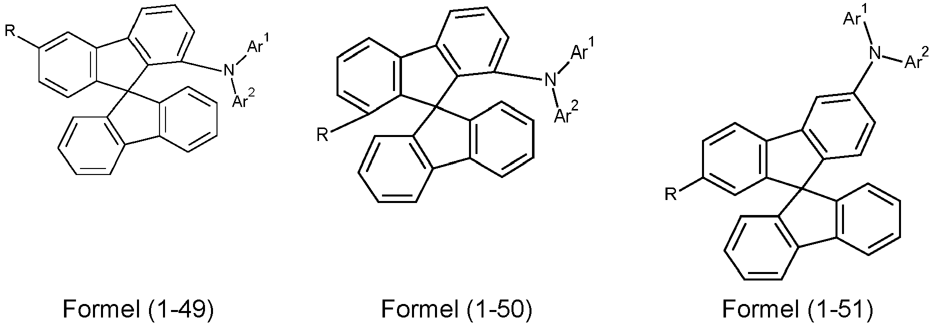 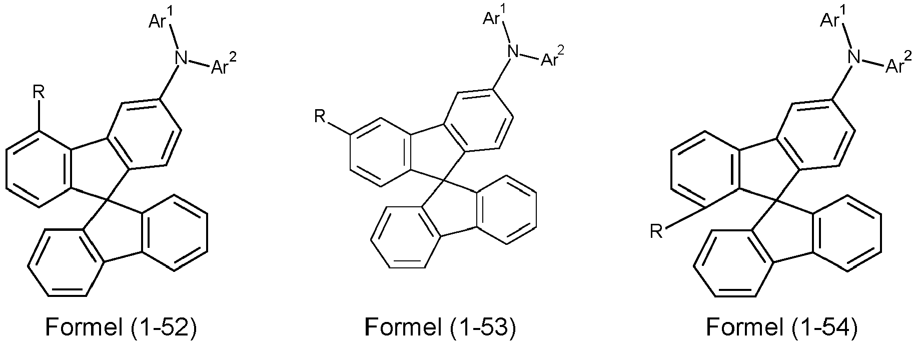 wobei die verwendeten Symbole und Indizes die wie in Anspruch 1 gegebene Bedeutung besitzen.

7. Verfahren zur Herstellung einer Verbindung nach einem oder mehreren der Ansprüche 1 bis 6, wobei der Index i=0, **dadurch gekennzeichnet, dass** eine Diarylaminogruppe durch eine C-N-Kupplungsreaktion zwischen einer Diarylaminverbindung und einem durch eine Gruppe R wie in Anspruch 1 definiert substituierten, halogenierten Spirobifluoren eingeführt wird.

8. Formulierung enthaltend mindestens eine Verbindung nach einem oder mehreren der Ansprüche 1 bis 6 sowie mindestens ein Lösungsmittel.

9. Verwendung einer Verbindung nach einem oder mehreren der Ansprüche 1 bis 6 in einer elektronischen Vorrichtung, insbesondere in einer organischen Elektrolumineszenzvorrichtung.

10. Elektronische Vorrichtung enthaltend mindestens eine Verbindung nach einem oder mehreren der Ansprüche 1 bis 6, vorzugsweise ausgewählt aus der Gruppe bestehend aus organischen Elektrolumineszenzvorrichtungen, organischen integrierten Schaltungen, organischen Feldeffekttransistoren, organischen Dünnschichttransistoren, organischen lichtemittierenden Transistoren, organischen Solarzellen, farbstoffsensibilisierten organischen Solarzellen, organischen optischen Detektoren, organischen Photorezeptoren, organischen Feld-Quench-Devices, lichtemittierenden elektrochemischen Zellen, organischen Laserdioden und organischen Plasmon-emittierenden Vorrichtungen.

11. Elektronische Vorrichtung nach Anspruch 10, bei der es sich um eine organische Elektrolumineszenzvorrichtung handelt, **dadurch gekennzeichnet, dass** die Verbindung nach einem oder mehreren der Ansprüche 1 bis 6 als Lochtransportmaterial in einer Lochtransport- oder Lochinjektions- oder Exzitonenblockier- oder Elektronenblockierschicht oder als Matrixmaterial für fluoreszierende oder phosphoreszierende Emitter eingesetzt wird.

## Revendications

1. Composé de la formule (1), 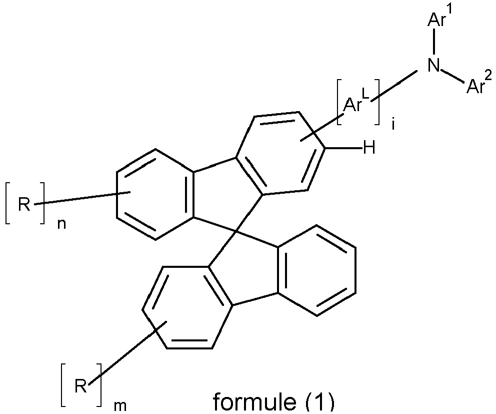 dans laquelle ce qui suit s'applique aux symboles et aux indices qui sont utilisés :
R est un système de cycle aromatique qui est sélectionné parmi phényle, biphényle, terphényle et naphtyle ;
Ar¹, Ar² sont pour chaque occurrence, de manière identique ou différente, un système de cycle aromatique ou hétéroaromatique qui comporte de 5 à 60 atomes de cycle aromatique, lequel peut dans chaque cas également être substitué par un radical ou par plusieurs radicaux R²; Ar¹ et Ar² peuvent ici également être connectés via une liaison simple ou un pont divalent qui est sélectionné parmi -N(R²)-, -O-, -S-, -C(R²)₂-, -C(R²)₂-C(R²)₂-, -Si(R²)₂- ou -B(R²)- ;
Ar^{L} est un système de cycle aromatique ou hétéroaromatique qui comporte de 5 à 40 atomes de cycle aromatique, lequel peut dans chaque cas être substitué par un radical ou par plusieurs radicaux R² ;
R² sont sélectionnés pour chaque occurrence, de manière identique ou différente, parmi le groupe qui est constitué par H, D, F, Cl, Br, I, CHO, CN, C(=O)Ar³, P(=O)(Ar³)₂, S(=O)Ar³, S(=O)₂Ar³, NO², Si(R³)₃, B(OR³)₂, OSO₂R³, les groupes alkyle, alcoxy ou thioalkyle en chaîne droite qui comportent de 1 à 40 atome(s) de C ou les groupes alkyle, alcoxy ou thioalkyle ramifiés ou cycliques qui comportent de 3 à 40 atomes de C, dont chacun peut être substitué par un radical ou par plusieurs radicaux R³, où, dans chaque cas, un ou plusieurs groupe(s) CH₂ non adjacents peut/peuvent être remplacé(s) par R³C=CR³, C≡C, Si(R³)₂, Ge(R³)₂, Sn(R³)₂, C=O, C=S, C=Se, P(=O)(R³), SO, SO₂, O, S ou CONR³ et où un ou plusieurs atome(s) de H peut/peuvent être remplacé(s) par D, F, Cl, Br, I, CN ou NO₂, les systèmes de cycle aromatique ou hétéroaromatique qui comportent de 5 à 60 atomes de cycle aromatique, lesquels peuvent dans chaque cas être substitués par un radical ou par plusieurs radicaux R³, et les groupes aryloxy qui comportent de 5 à 60 atomes de cycle aromatique, lesquels peuvent être substitués par un radical ou par plusieurs radicaux R³, où deux substituants R² adjacents ou plus peuvent en option former un système de cycle aliphatique mono- ou polycyclique ou un système de cycle aromatique, lequel peut être substitué par un radical ou par plusieurs radicaux R³ ;
R³ est sélectionné pour chaque occurrence, de manière identique ou différente, parmi le groupe qui est constitué par H, D, F, Cl, Br, I, CHO, CN, C(=O)Ar³, P(=O)(Ar³)₂, S(=O)Ar³, S(=O)₂Ar³, NO², Si(R⁴)₃, B(OR⁴)₂, OSO₂R⁴, les groupes alkyle, alcoxy ou thioalkyle en chaîne droite qui comportent de 1 à 40 atome(s) de C ou les groupes alkyle, alcoxy ou thioalkyle ramifiés ou cycliques qui comportent de 3 à 40 atomes de C, dont chacun peut être substitué par un radical ou par plusieurs radicaux R⁴, où, dans chaque cas, un ou plusieurs groupe(s) CH₂ non adjacents peut/peuvent être remplacé(s) par R⁴C=CR⁴, C≡C, Si(R⁴)₂, Ge(R⁴)₂, Sn(R⁴)₂, C=O, C=S, C=Se, P(=O)(R⁴), SO, SO₂, O, S ou CONR⁴ et où un ou plusieurs atome(s) de H peut/peuvent être remplacé(s) par D, F, Cl, Br, I, CN ou NO², les systèmes de cycle aromatique ou hétéroaromatique qui comportent de 5 à 60 atomes de cycle aromatique, lesquels peuvent dans chaque cas être substitués par un radical ou par plusieurs radicaux R⁴, et les groupes aryloxy qui comportent de 5 à 60 atomes de cycle aromatique, lesquels peuvent être substitués par un radical ou par plusieurs radicaux R⁴, où deux substituants R³ adjacents ou plus peuvent en option former un système de cycle aliphatique mono- ou polycyclique ou un système de cycle aromatique, lequel peut être substitué par un radical ou par plusieurs radicaux R⁴ ;
R⁴ est sélectionné pour chaque occurrence, de manière identique ou différente, parmi le groupe qui est constitué par H, D, F, Cl, Br, I, CN, les groupes alkyle, alcoxy ou thioalkyle en chaîne droite qui comportent de 1 à 20 atome(s) de C ou les groupes alkyle, alcoxy ou thioalkyle ramifiés ou cycliques qui comportent de 3 à 20 atomes de C, où, dans chaque cas, un ou plusieurs groupe(s) CH₂ non adjacents peut/peuvent être remplacé(s) par SO, SO₂, O ou S et où un ou plusieurs atome(s) de H peut/peuvent être remplacé(s) par D, F, Cl, Br ou I, et les systèmes de cycle aromatique ou hétéroaromatique qui comportent de 5 à 24 atomes de C; Ar³ est un système de cycle aromatique ou hétéroaromatique qui comporte de 5 à 24 atomes de cycle aromatique, lequel peut dans chaque cas être substitué par un radical ou par plusieurs radicaux R^{4;}
i est 0 ou 1 ;
m, n sont, de manière identique ou différente, 0, ou 1 ;
et m + n = 1,
où les composés qui suivent sont exclus :

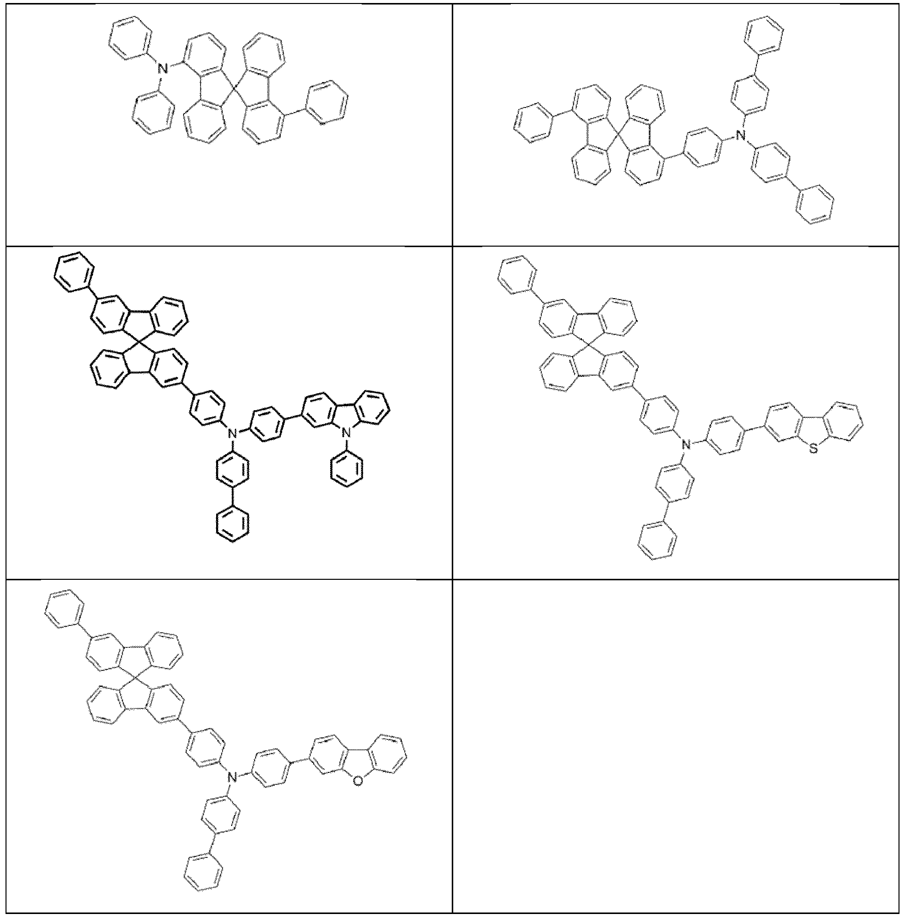

2. Composé selon la revendication 1, **caractérisé en ce que** m = 1 et n=0.

3. Composé selon la revendication 1 ou 2, **caractérisé en ce que** les groupes Ar¹ et Ar² sont sélectionnés parmi les groupes des formules (A-1) à (A-48), 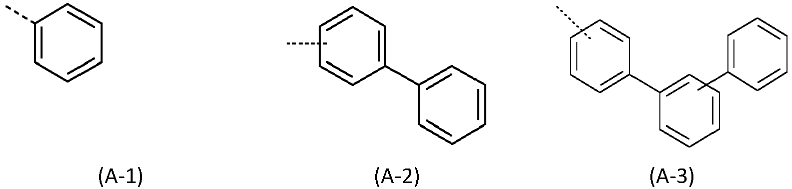 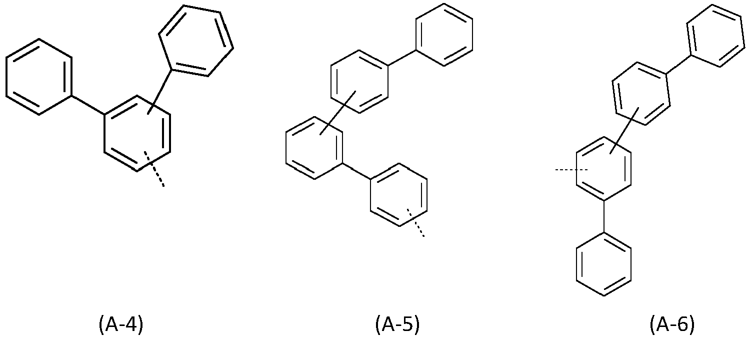 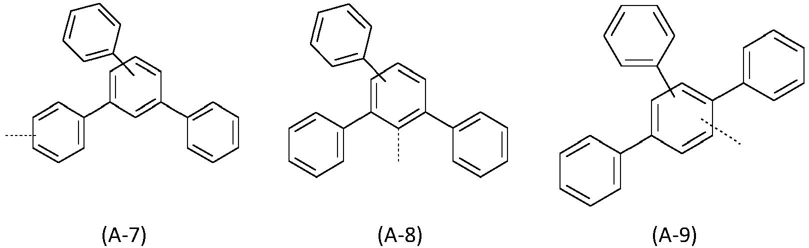 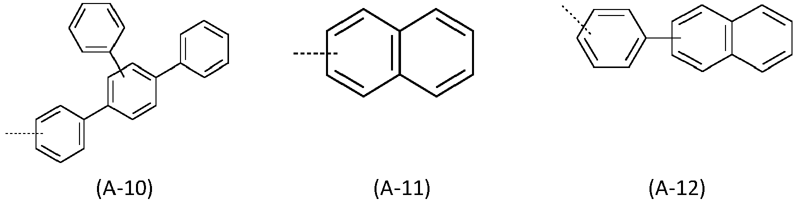 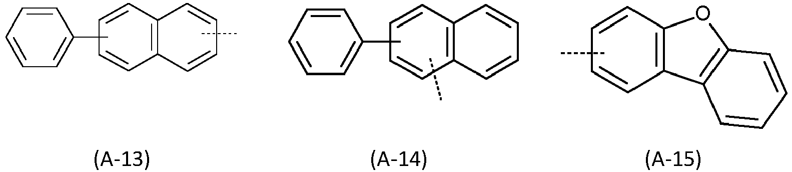 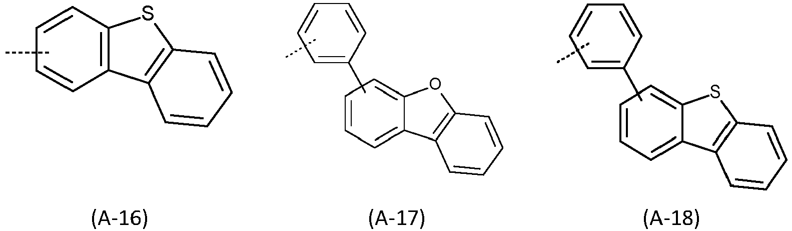 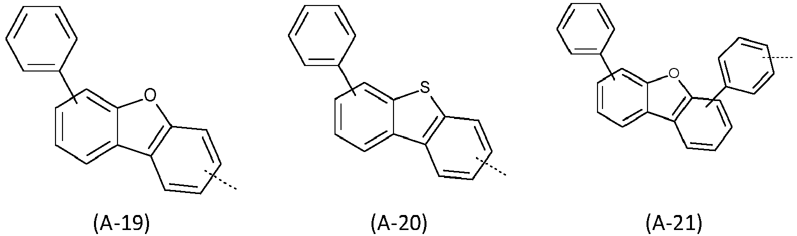 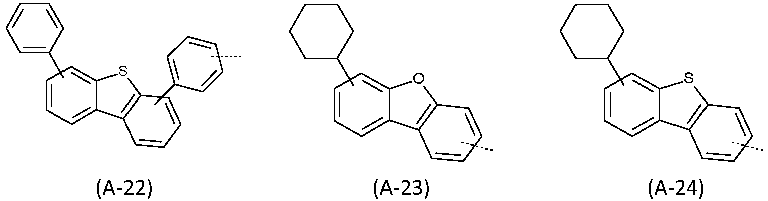 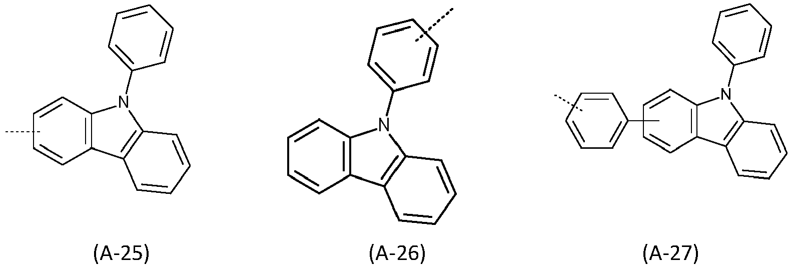 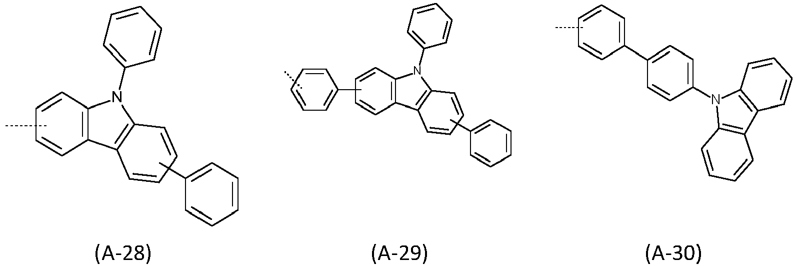 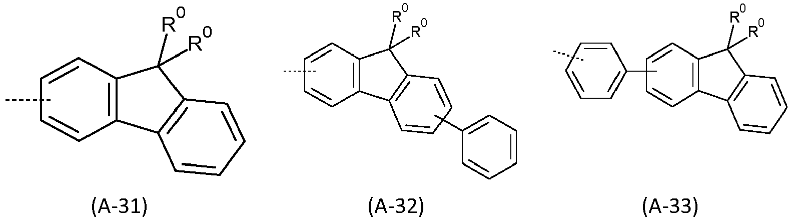 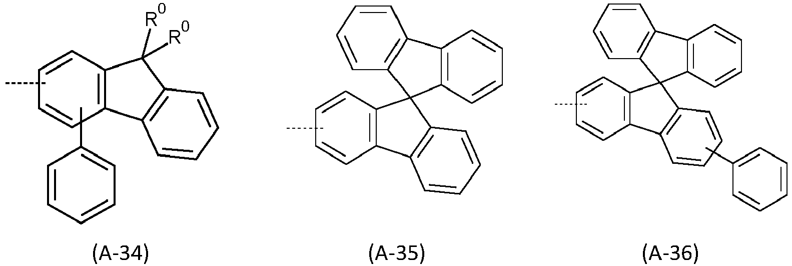 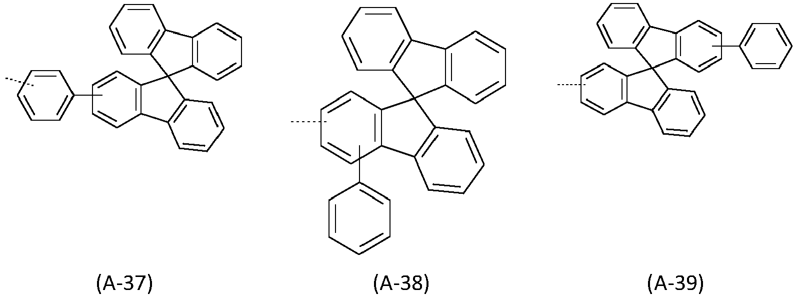 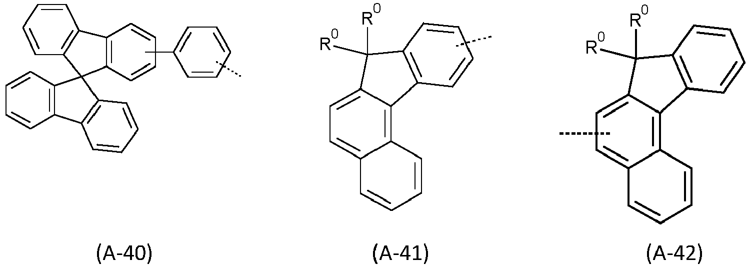 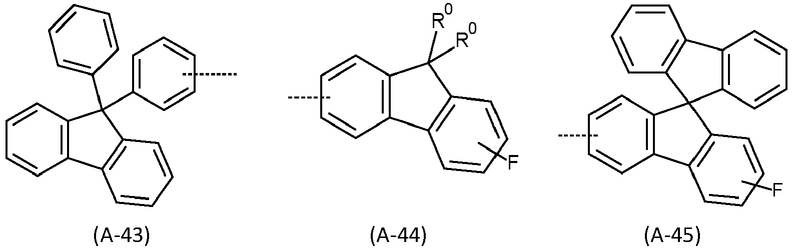 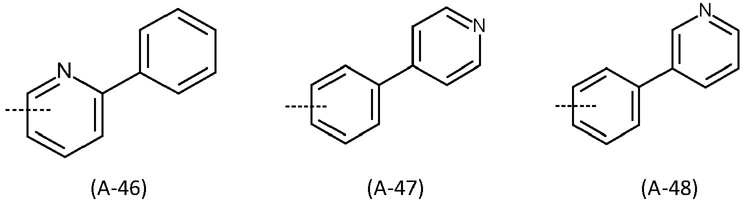 dans lesquelles les liaisons en pointillés indiquent les liaisons sur l'atome d'azote, comme il a été représenté selon la formule (1),
où les groupes des formules (A-1) à (A-48) peuvent en outre être substitués au niveau de chaque position libre par un groupe R² comme il a été défini selon la revendication 1, et
où les groupes R⁰, dans les formules (A-31) à (A-34), (A-41), (A-42) et (A-44), sont sélectionnés pour chaque occurrence, de manière identique ou différente, parmi le groupe qui est constitué par H, D, F, CN, Si(R³)₃, les groupes alkyle en chaîne droite qui comportent de 1 à 10 atome(s) de C ou les groupes alkyle ramifiés ou cycliques qui comportent de 3 à 10 atomes de C, dont chacun peut être substitué par un radical ou par plusieurs radicaux R³, et les groupes aryle ou hétéroaryle qui comportent de 5 à 18 atomes de cycle aromatique, lesquels peuvent dans chaque cas être substitués par un radical ou par plusieurs radicaux R³, où deux substituants R⁰ adjacents peuvent en option former un système de cycle aliphatique mono- ou polycyclique ou un système de cycle aromatique, lequel peut être substitué par un radical ou par plusieurs radicaux R³.

4. Composé selon une ou plusieurs des revendications précédentes, **caractérisé en ce que** Ar^{L} est sélectionné parmi les systèmes de cycle aromatique ou hétéroaromatique qui comportent de 5 à 18 atomes de cycle aromatique, lesquels peuvent dans chaque cas être substitués par un radical ou par plusieurs radicaux R².

5. Composé selon une ou plusieurs des revendications précédentes, **caractérisé en ce que** les R² sont sélectionnés, de manière identique ou différente pour chaque occurrence, parmi le groupe qui est constitué par H, D, F, CN, les groupes alkyle ou alcoxy en chaîne droite qui comportent de 1 à 10 atome(s) de C ou les groupes alkyle ou alcoxy ramifiés ou cycliques qui comportent de 3 à 10 atomes de C, dont chacun peut être substitué par un radical ou par plusieurs radicaux R³, où un ou plusieurs groupe(s) CH₂ non adjacents peut/peuvent être remplacé(s) par O et où un ou plusieurs atome(s) de H peut/peuvent être remplacé(s) par F, et les systèmes de cycle aromatique ou hétéroaromatique qui comportent de 5 à 24 atomes de cycle aromatique, lesquels peuvent dans chaque cas être substitués par un radical ou par plusieurs radicaux R³.

6. Composé selon une ou plusieurs des revendications précédentes, sélectionné parmi les composés des formules (1-1) à (1-24) et (1-31) à (1-54), 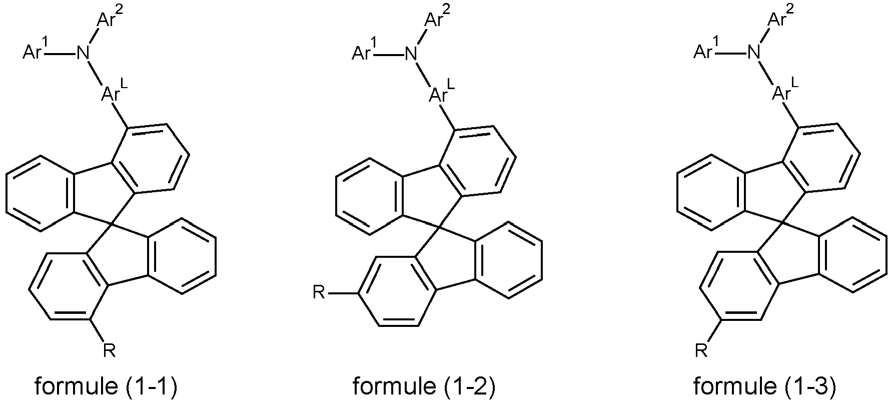 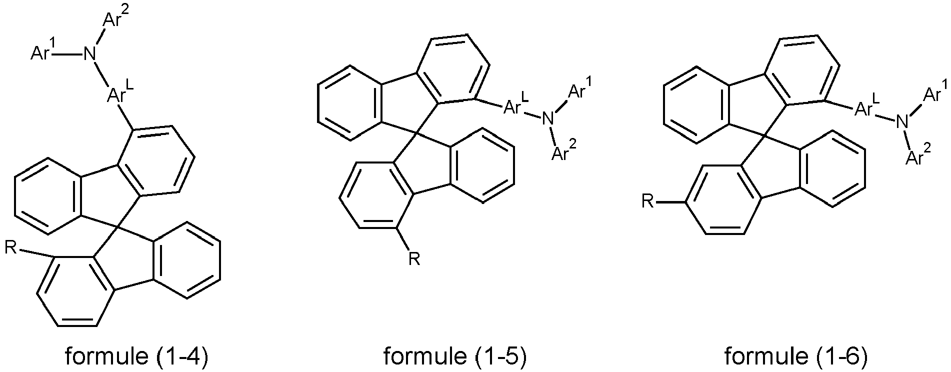 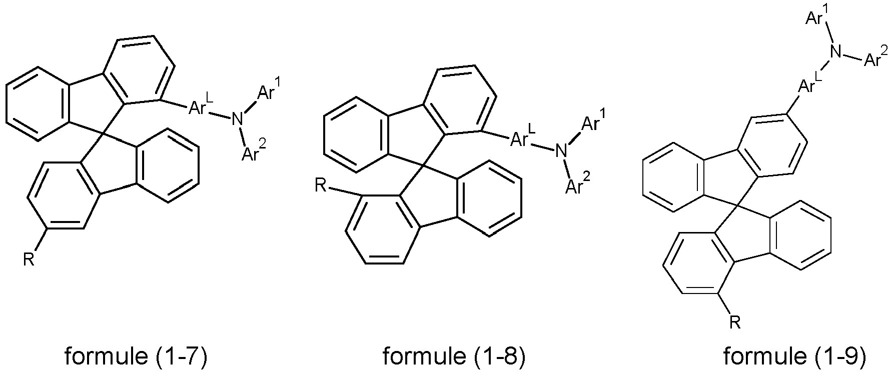 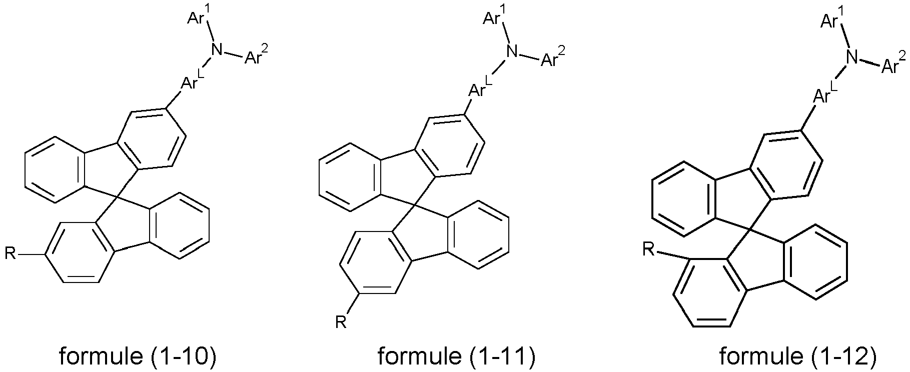 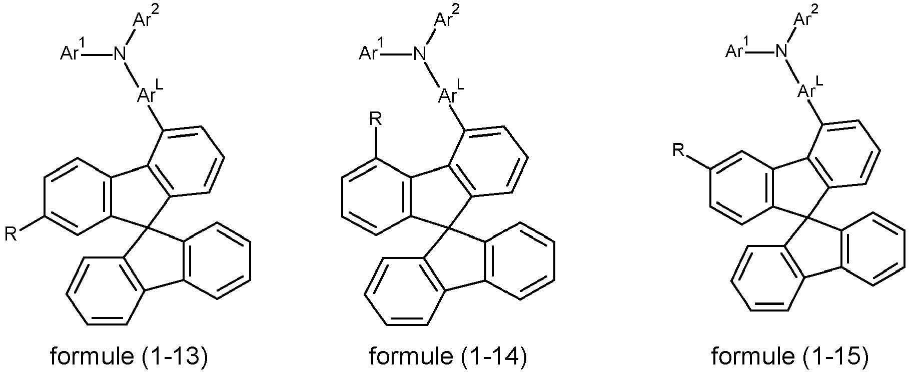 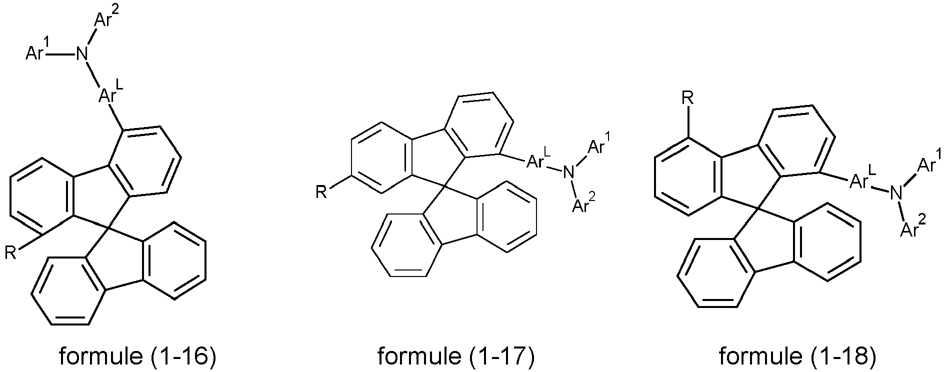 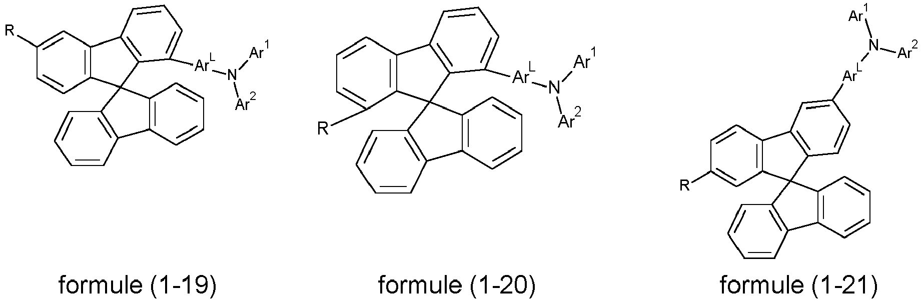 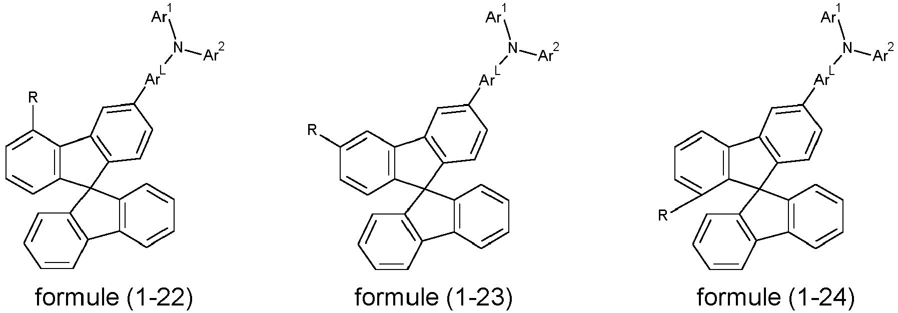 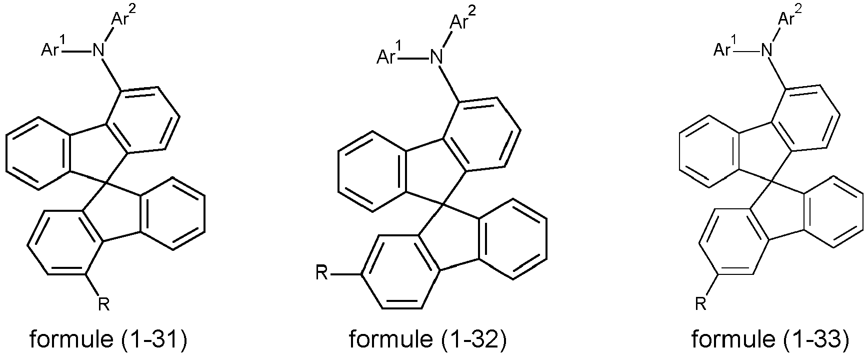 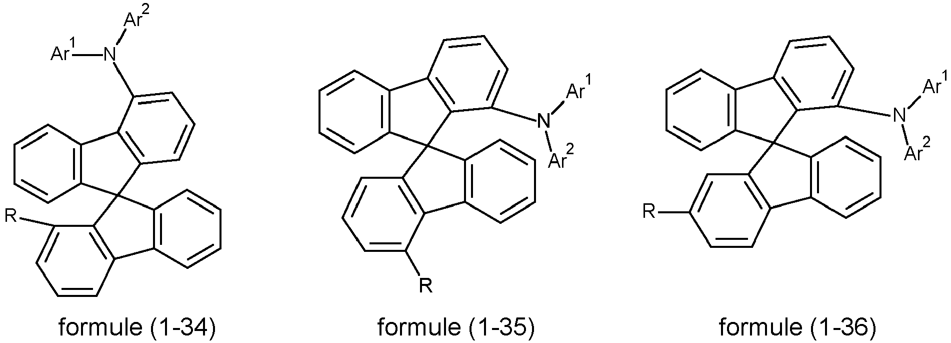 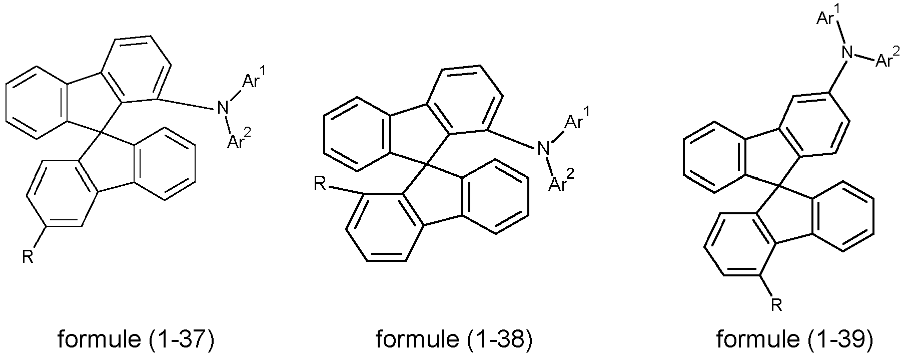 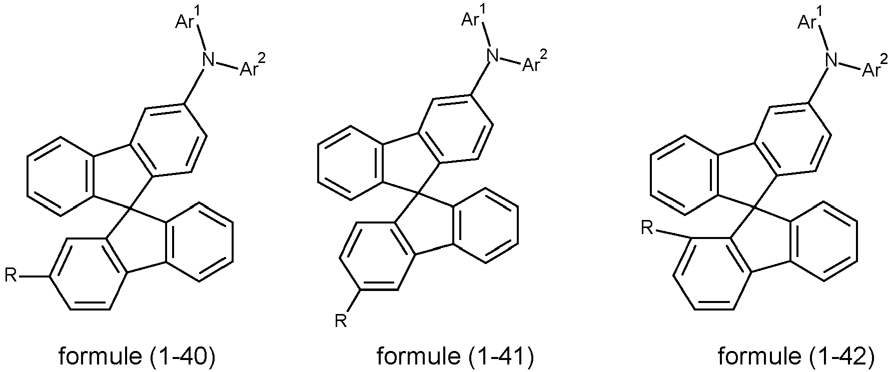 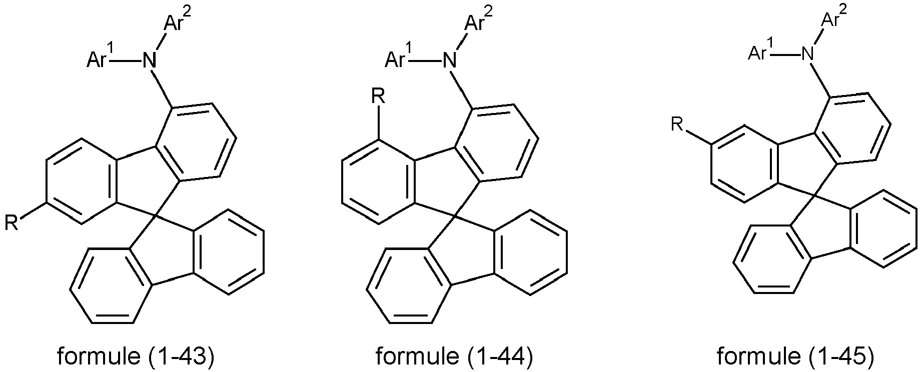 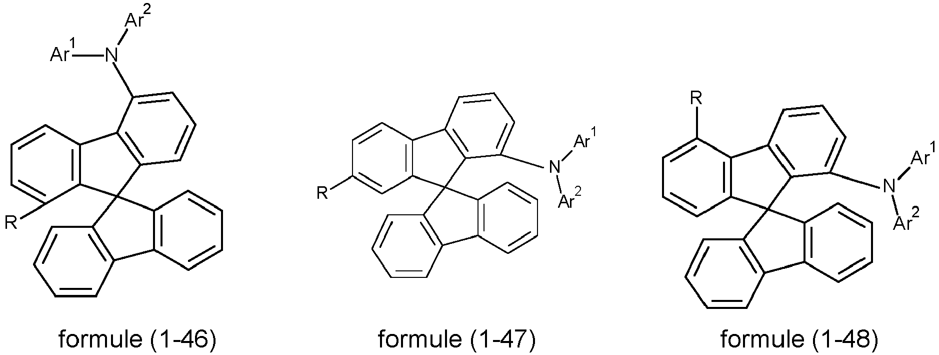 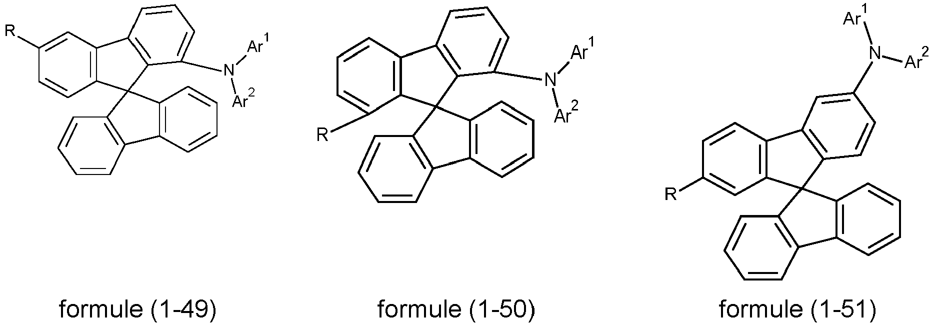 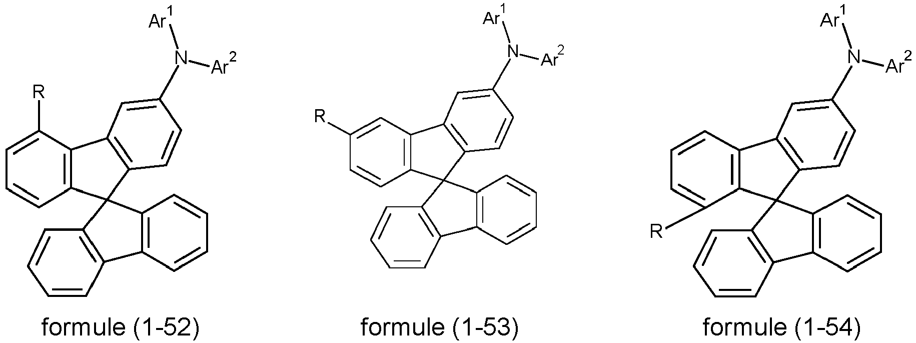 dans lesquelles les symboles et les indices qui sont utilisés présentent les significations comme il a été donné selon la revendication 1.

7. Procédé pour la préparation d'un composé selon une ou plusieurs des revendications 1 à 6, dans lequel indice i = 0, **caractérisé en ce qu'**un groupe diarylamino est introduit au moyen d'une réaction de couplage C-N entre un composé diarylamine et un spirobifluorène halogéné qui est substitué par un groupe R comme il a été défini selon la revendication 1.

8. Formulation comprenant au moins un composé, selon une ou plusieurs des revendications 1 à 6, et au moins un solvant.

9. Utilisation d'un composé selon une ou plusieurs des revendications 1 à 6 dans un dispositif électronique, en particulier dans un dispositif électroluminescent organique.

10. Dispositif électronique comprenant au moins un composé selon une ou plusieurs des revendications 1 à 6, de préférence sélectionné parmi le groupe qui est constitué par les dispositifs électroluminescents organiques, les circuits intégrés organiques, les transistors à effet de champ organiques, les transistors à film mince organiques, les transistors à émission de lumière ou électroluminescents organiques, les cellules solaires organiques, les cellules solaires organiques sensibilisées par colorant, les détecteurs optiques organiques, les photorécepteurs organiques, les dispositifs à extinction de champ organiques, les cellules électrochimiques à émission de lumière ou électroluminescentes, les diodes laser organiques et les dispositifs à émission de plasmons organiques.

11. Dispositif électronique selon la revendication 10, lequel est un dispositif électroluminescent organique, **caractérisé en ce que** le composé selon une ou plusieurs des revendications 1 à 6 est utilisé en tant que matériau de transport de trous dans une couche de transport de trous ou d'injection de trous ou de blocage d'excitons ou de blocage d'électrons ou en tant que matériau de matrice pour les émetteurs fluorescents ou phosphorescents.
